(19) Europäisches Patentamt
European Patent Office
Office européen des brevets

(11) EP 4 008 351 A1

(12) EUROPEAN PATENT APPLICATION
published in accordance with Art. 153(4) EPC

(43) Date of publication:
**08.06.2022 Bulletin 2022/23**

(21) Application number: **20850986.9**

(22) Date of filing: **31.07.2020**

(51) International Patent Classification (IPC):
***A61K 39/395*** $^{(2006.01)}$ ***C07K 16/18*** $^{(2006.01)}$
***C12N 5/20*** $^{(2006.01)}$ ***C12N 15/13*** $^{(2006.01)}$
***G01N 33/68*** $^{(2006.01)}$ ***G01N 33/577*** $^{(2006.01)}$
***G01N 33/574*** $^{(2006.01)}$ ***A61P 35/00*** $^{(2006.01)}$
***A61P 35/02*** $^{(2006.01)}$

(52) Cooperative Patent Classification (CPC):
**A61K 31/47; A61K 31/4709; A61K 39/395; A61K 45/06; A61K 49/00; A61K 51/10; A61P 35/00; A61P 35/02; C07K 16/18; C07K 16/28; G01N 33/574; G01N 33/577; G01N 33/68**

(86) International application number:
**PCT/CN2020/106219**

(87) International publication number:
**WO 2021/023108 (11.02.2021 Gazette 2021/06)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **02.08.2019 CN 201910711138**
**13.11.2019 CN 201911105711**
**13.11.2019 CN 201911105715**
**19.11.2019 CN 201911133858**

(71) Applicant: **CTTQ-Akeso (ShangHai) Biomed. Tech. Co., Ltd.**
**Shang 201908 (CN)**

(72) Inventors:
- **WANG, Zhongmin**
  **Zhongshan, Guangdong 528437 (CN)**
- **ZHANG, Peng**
  **Zhongshan, Guangdong 528437 (CN)**
- **LI, Baiyong**
  **Zhongshan, Guangdong 528437 (CN)**
- **XIA, Yu**
  **Zhongshan, Guangdong 528437 (CN)**

(74) Representative: **Cooley (UK) LLP**
**22 Bishopsgate**
**London EC2N 4BQ (GB)**

# (54) ANTI-PD-1 ANTIBODY AND MEDICAL USE THEREOF

(57) The present invention relates to the field of tumor treatment and molecular immunology, and particularly, to an anti-PD-1 antibody and pharmaceutical use thereof. Specifically, the present invention relates to a monoclonal antibody, wherein a heavy chain variable region of the monoclonal antibody comprises CDRs with amino acid sequences of SEQ ID NOs: 19-21, and/or a light chain variable region of the monoclonal antibody comprises CDRs with amino acid sequences of SEQ ID NOs: 22-24; wherein according to the EU numbering system, a heavy chain constant region of the antibody comprises mutations at any 2 or 3 of positions 234, 235 and 237, and an affinity constant of the antibody to FcγRIIIa and/or Clq is reduced after the mutation as compared to that before the mutation. The monoclonal antibody of the present invention can be well and specifically bind to PD-1, specifically relieve immunosuppression of PD-1 in an organism and activate T lymphocytes.



Processed by Luminess, 75001 PARIS (FR)

(Cont. next page)

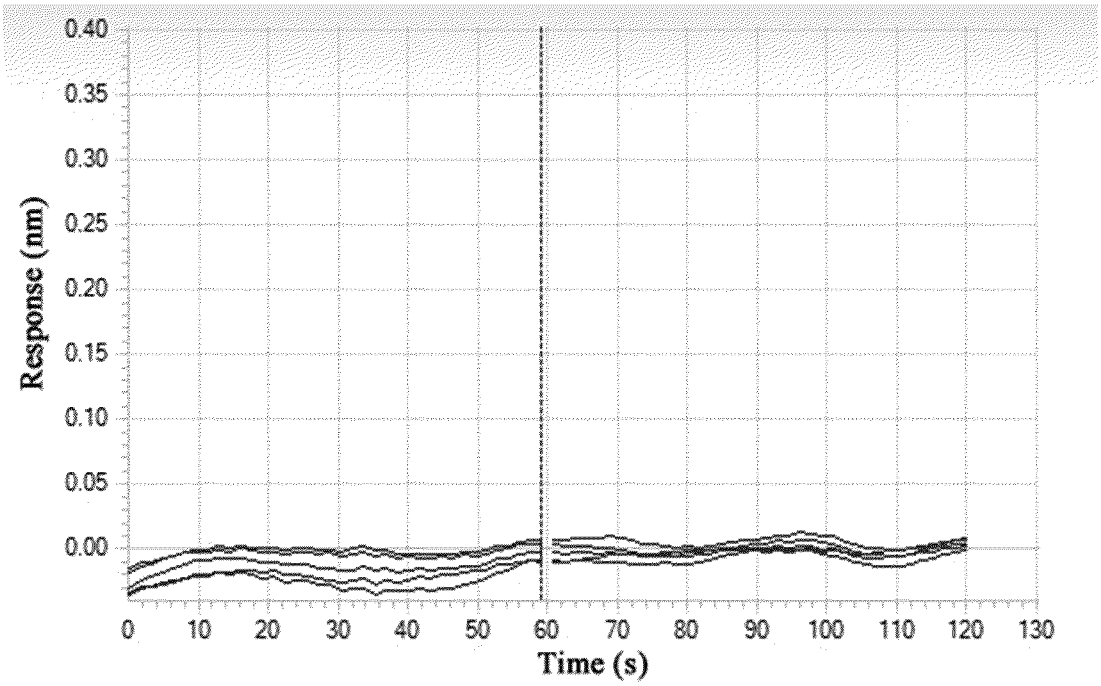
0.40
0.35
0.30
0.25
0.20
0.15
0.10
0.05
0.00
Response (nm)
0
10
20
30
40
50
60
70
80
90
100
110
120
130
Time (s)

FIG. 34

SEQUENCE LISTING

<110> CTTQ-Akeso (Shanghai) Biomed. Tech. Co., Ltd

<120> ANTI-PD-1 ANTIBODY AND PHARMACEUTICAL USE THEREOF

<130>

<150> CN201910711138.5
<151> 2019-08-02

<150> CN201911133858.4
<151> 2019-11-19

<150> CN201911105715.2
<151> 2019-11-13

<150> CN201911105711.4
<151> 2019-11-13

<160> 24

<170> PatentIn version 3.5

<210> 1
<211> 354
<212> DNA
<213> Artificial

<220>
<223> Nucleic acid sequence of 14C12 heavy chain variable region

<400> 1
gaggtcaaac tggtggagag cggcggcggg ctggtgaagc ccggcgggtc actgaaactg 60

agctgcgccg cttccggctt cgcctttagc tcctacgaca tgtcatgggt gaggcagacc 120

cctgagaagc gcctggaatg ggtcgctact atcagcggag gcgggcgata cacctactat 180

cctgactctg tcaaagggag attcacaatt agtcgggata acgccagaaa tactctgtat 240

ctgcagatgt ctagtctgcg gtccgaggat acagctctgt actattgtgc aaaccggtac 300

ggcgaagcat ggtttgccta ttggggacag ggcaccctgg tgacagtctc tgcc 354

<210> 2
<211> 118
<212> PRT
<213> Artificial

<220>
<223> The amino acid sequence of 14C12 heavy chain variable region

<400> 2

Glu Val Lys Leu Val Glu Ser Gly Gly Gly Leu Val Lys Pro Gly Gly
1 5 10 15

Ser Leu Lys Leu Ser Cys Ala Ala Ser Gly Phe Ala Phe Ser Ser Tyr
20 25 30

```
Asp Met Ser Trp Val Arg Gln Thr Pro Glu Lys Arg Leu Glu Trp Val
        35                  40                  45

Ala Thr Ile Ser Gly Gly Gly Arg Tyr Thr Tyr Tyr Pro Asp Ser Val
    50                  55                  60

Lys Gly Arg Phe Thr Ile Ser Arg Asp Asn Ala Arg Asn Thr Leu Tyr
65                  70                  75                  80

Leu Gln Met Ser Ser Leu Arg Ser Glu Asp Thr Ala Leu Tyr Tyr Cys
                85                  90                  95

Ala Asn Arg Tyr Gly Glu Ala Trp Phe Ala Tyr Trp Gly Gln Gly Thr
            100                 105                 110

Leu Val Thr Val Ser Ala
        115


<210>  3
<211>  321
<212>  DNA
<213>  Artificial

<220>
<223>  Nucleic acid sequence of 14C12 light chain variable region

<400>  3
gacattaaga tgacacagtc cccttcctca atgtacgcta gcctgggcga gcgagtgacc        60

ttcacatgca aagcatccca ggacatcaac acatacctgt cttggtttca gcagaagcca       120

ggcaaaagcc ccaagaccct gatctaccgg gccaatagac tggtggacgg ggtccccagc       180

agattctccg gatctggcag tgggcaggat tactccctga ccatcagctc cctggagtat       240

gaagacatgg gcatctacta ttgcctgcag tatgatgagt tccctctgac ctttggagca       300

ggcacaaaac tggaactgaa g                                                 321


<210>  4
<211>  107
<212>  PRT
<213>  Artificial

<220>
<223>  The amino acid sequence of 14C12 light chain variable region

<400>  4

Asp Ile Lys Met Thr Gln Ser Pro Ser Ser Met Tyr Ala Ser Leu Gly
1               5                   10                  15

Glu Arg Val Thr Phe Thr Cys Lys Ala Ser Gln Asp Ile Asn Thr Tyr
            20                  25                  30
```

Leu Ser Trp Phe Gln Gln Lys Pro Gly Lys Ser Pro Lys Thr Leu Ile
35 40 45

Tyr Arg Ala Asn Arg Leu Val Asp Gly Val Pro Ser Arg Phe Ser Gly
50 55 60

Ser Gly Ser Gly Gln Asp Tyr Ser Leu Thr Ile Ser Ser Leu Glu Tyr
65 70 75 80

Glu Asp Met Gly Ile Tyr Tyr Cys Leu Gln Tyr Asp Glu Phe Pro Leu
85 90 95

Thr Phe Gly Ala Gly Thr Lys Leu Glu Leu Lys
100 105

<210> 5
<211> 354
<212> DNA
<213> Artificial

<220>
<223> Nucleic acid sequence of 14C12H1L1(hG1WT) heavy chain variable region

<400> 5
gaagtgcagc tggtcgagtc tgggggaggg ctggtgcagc ccggcgggtc actgcgactg 60

agctgcgcag cttccggatt cgcctttagc tcctacgaca tgtcctgggt gcgacaggca 120

ccaggaaagg gactggattg ggtcgctact atctcaggag gcgggagata cacctactat 180

cctgacagcg tcaagggccg gttcacaatc tctagagata acagtaagaa caatctgtat 240

ctgcagatga acagcctgag ggctgaggac accgcactgt actattgtgc caaccgctac 300

ggggaagcat ggtttgccta ttgggggcag ggaaccctgg tgacagtctc tagt 354

<210> 6
<211> 118
<212> PRT
<213> Artificial

<220>
<223> The amino acid sequence of 14C12H1L1(hG1WT) heavy chain variable region

<400> 6

Glu Val Gln Leu Val Glu Ser Gly Gly Gly Leu Val Gln Pro Gly Gly
1 5 10 15

Ser Leu Arg Leu Ser Cys Ala Ala Ser Gly Phe Ala Phe Ser Ser Tyr
20 25 30

Asp Met Ser Trp Val Arg Gln Ala Pro Gly Lys Gly Leu Asp Trp Val
35 40 45

Ala Thr Ile Ser Gly Gly Gly Arg Tyr Thr Tyr Tyr Pro Asp Ser Val
50 55 60

Lys Gly Arg Phe Thr Ile Ser Arg Asp Asn Ser Lys Asn Asn Leu Tyr
65 70 75 80

Leu Gln Met Asn Ser Leu Arg Ala Glu Asp Thr Ala Leu Tyr Tyr Cys
85 90 95

Ala Asn Arg Tyr Gly Glu Ala Trp Phe Ala Tyr Trp Gly Gln Gly Thr
100 105 110

Leu Val Thr Val Ser Ser
115

<210> 7
<211> 321
<212> DNA
<213> Artificial

<220>
<223> Nucleic acid sequence of 14C12H1L1(hG1WT) light chain variable region

<400> 7
gacattcaga tgactcagag cccctcctcc atgtccgcct ctgtgggcga cagggtcacc 60

ttcacatgcc gcgctagtca ggatatcaac acctacctga gctggtttca gcagaagcca 120

gggaaaagcc ccaagacact gatctaccgg gctaatagac tggtgtctgg agtcccaagt 180

cggttcagtg gctcagggag cggacaggac tacactctga ccatcagctc cctgcagcct 240

gaggacatgg caacctacta ttgcctgcag tatgatgagt tcccactgac ctttggcgcc 300

gggacaaaac tggagctgaa g 321

<210> 8
<211> 107
<212> PRT
<213> Artificial

<220>
<223> The amino acid sequence of 14C12H1L1(hG1WT) light chain variable region

<400> 8

Asp Ile Gln Met Thr Gln Ser Pro Ser Ser Met Ser Ala Ser Val Gly
1 5 10 15

Asp Arg Val Thr Phe Thr Cys Arg Ala Ser Gln Asp Ile Asn Thr Tyr
20 25 30

Leu Ser Trp Phe Gln Gln Lys Pro Gly Lys Ser Pro Lys Thr Leu Ile
35 40 45

Tyr Arg Ala Asn Arg Leu Val Ser Gly Val Pro Ser Arg Phe Ser Gly
50 55 60

Ser Gly Ser Gly Gln Asp Tyr Thr Leu Thr Ile Ser Ser Leu Gln Pro
65 70 75 80

Glu Asp Met Ala Thr Tyr Tyr Cys Leu Gln Tyr Asp Glu Phe Pro Leu
85 90 95

Thr Phe Gly Ala Gly Thr Lys Leu Glu Leu Lys
100 105

<210> 9
<211> 1344
<212> DNA
<213> Artificial

<220>
<223> Nucleic acid sequence of 14C12H1L1 (hG1WT) heavy chain

<400> 9
gaagtgcagc tggtcgagtc tgggggaggg ctggtgcagc ccggcgggtc actgcgactg 60
agctgcgcag cttccggatt cgcctttagc tcctacgaca tgtcctgggt gcgacaggca 120
ccaggaaagg gactggattg ggtcgctact atctcaggag gcgggagata cacctactat 180
cctgacagcg tcaagggccg gttcacaatc tctagagata acagtaagaa caatctgtat 240
ctgcagatga acagcctgag ggctgaggac accgcactgt actattgtgc caaccgctac 300
ggggaagcat ggtttgccta ttgggggcag ggaaccctgg tgacagtctc tagtgccagc 360
accaaaggac ctagcgtgtt tcctctcgcc ccctcctcca aaagcaccag cggaggaacc 420
gctgctctcg gatgtctggt gaaggactac ttccctgaac ccgtcaccgt gagctggaat 480
agcggcgctc tgacaagcgg agtccataca ttccctgctg tgctgcaaag cagcggactc 540
tattccctgt ccagcgtcgt cacagtgccc agcagcagcc tgggcaccca gacctacatc 600
tgtaacgtca accacaagcc ctccaacacc aaggtggaca agaaagtgga gcccaaatcc 660
tgcgacaaga cacacacctg tccccccctgt cctgctcccg aactcctcgg aggccctagc 720
gtcttcctct ttcctcccaa acccaaggac accctcatga tcagcagaac ccctgaagtc 780
acctgtgtcg tcgtggatgt cagccatgag gacccccgagg tgaaattcaa ctggtatgtc 840
gatggcgtcg aggtgcacaa cgccaaaacc aagcccaggg aggaacagta caactccacc 900
tacagggtgg tgtccgtgct gacagtcctc caccaggact ggctgaacgg caaggagtac 960

```
aagtgcaagg tgtccaacaa ggctctccct gcccccattg agaagaccat cagcaaggcc     1020

aaaggccaac ccagggagcc ccaggtctat acactgcctc cctccaggga cgaactcacc     1080

aagaaccagg tgtccctgac ctgcctggtc aagggctttt atcccagcga catcgccgtc     1140

gagtgggagt ccaacggaca gcccgagaat aactacaaga ccacccctcc tgtcctcgac     1200

tccgacggct ccttcttcct gtacagcaag ctgaccgtgg acaaaagcag gtggcagcag     1260

ggaaacgtgt tctcctgcag cgtgatgcac gaagccctcc acaaccacta cacccagaaa     1320

agcctgtccc tgagccccgg caaa                                            1344
```

```
<210>  10
<211>  448
<212>  PRT
<213>  Artificial

<220>
<223>  Amino acid sequence of 14C12H1L1 (hG1WT) heavy chain

<400>  10

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Ala Phe Ser Ser Tyr
            20                  25                  30

Asp Met Ser Trp Val Arg Gln Ala Pro Gly Lys Gly Leu Asp Trp Val
        35                  40                  45

Ala Thr Ile Ser Gly Gly Gly Arg Tyr Thr Tyr Tyr Pro Asp Ser Val
    50                  55                  60

Lys Gly Arg Phe Thr Ile Ser Arg Asp Asn Ser Lys Asn Asn Leu Tyr
65                  70                  75                  80

Leu Gln Met Asn Ser Leu Arg Ala Glu Asp Thr Ala Leu Tyr Tyr Cys
                85                  90                  95

Ala Asn Arg Tyr Gly Glu Ala Trp Phe Ala Tyr Trp Gly Gln Gly Thr
            100                 105                 110

Leu Val Thr Val Ser Ser Ala Ser Thr Lys Gly Pro Ser Val Phe Pro
        115                 120                 125

Leu Ala Pro Ser Ser Lys Ser Thr Ser Gly Gly Thr Ala Ala Leu Gly
    130                 135                 140

Cys Leu Val Lys Asp Tyr Phe Pro Glu Pro Val Thr Val Ser Trp Asn
145                 150                 155                 160
```

```
Ser Gly Ala Leu Thr Ser Gly Val His Thr Phe Pro Ala Val Leu Gln
                165                 170                 175

Ser Ser Gly Leu Tyr Ser Leu Ser Ser Val Val Thr Val Pro Ser Ser
            180                 185                 190

Ser Leu Gly Thr Gln Thr Tyr Ile Cys Asn Val Asn His Lys Pro Ser
        195                 200                 205

Asn Thr Lys Val Asp Lys Lys Val Glu Pro Lys Ser Cys Asp Lys Thr
    210                 215                 220

His Thr Cys Pro Pro Cys Pro Ala Pro Glu Leu Leu Gly Gly Pro Ser
225                 230                 235                 240

Val Phe Leu Phe Pro Pro Lys Pro Lys Asp Thr Leu Met Ile Ser Arg
                245                 250                 255

Thr Pro Glu Val Thr Cys Val Val Val Asp Val Ser His Glu Asp Pro
            260                 265                 270

Glu Val Lys Phe Asn Trp Tyr Val Asp Gly Val Glu Val His Asn Ala
        275                 280                 285

Lys Thr Lys Pro Arg Glu Glu Gln Tyr Asn Ser Thr Tyr Arg Val Val
    290                 295                 300

Ser Val Leu Thr Val Leu His Gln Asp Trp Leu Asn Gly Lys Glu Tyr
305                 310                 315                 320

Lys Cys Lys Val Ser Asn Lys Ala Leu Pro Ala Pro Ile Glu Lys Thr
                325                 330                 335

Ile Ser Lys Ala Lys Gly Gln Pro Arg Glu Pro Gln Val Tyr Thr Leu
            340                 345                 350

Pro Pro Ser Arg Asp Glu Leu Thr Lys Asn Gln Val Ser Leu Thr Cys
        355                 360                 365

Leu Val Lys Gly Phe Tyr Pro Ser Asp Ile Ala Val Glu Trp Glu Ser
    370                 375                 380

Asn Gly Gln Pro Glu Asn Asn Tyr Lys Thr Thr Pro Pro Val Leu Asp
385                 390                 395                 400

Ser Asp Gly Ser Phe Phe Leu Tyr Ser Lys Leu Thr Val Asp Lys Ser
```

405 410 415

Arg Trp Gln Gln Gly Asn Val Phe Ser Cys Ser Val Met His Glu Ala
420 425 430

Leu His Asn His Tyr Thr Gln Lys Ser Leu Ser Leu Ser Pro Gly Lys
435 440 445

<210> 11
<211> 642
<212> DNA
<213> Artificial

<220>
<223> Nucleic acid sequence of 14C12H1L1 (hG1WT) light chain

<400> 11
gacattcaga tgactcagag cccctcctcc atgtccgcct ctgtgggcga cagggtcacc 60

ttcacatgcc gcgctagtca ggatatcaac acctacctga gctggtttca gcagaagcca 120

gggaaaagcc ccaagacact gatctaccgg gctaatagac tggtgtctgg agtcccaagt 180

cggttcagtg gctcagggag cggacaggac tacactctga ccatcagctc cctgcagcct 240

gaggacatgg caacctacta ttgcctgcag tatgatgagt tcccactgac ctttggcgcc 300

gggacaaaac tggagctgaa gcgaactgtg gccgctccct ccgtcttcat ttttcccccct 360

tctgacgaac agctgaaatc aggcacagcc agcgtggtct gtctgctgaa caatttctac 420

cctagagagg caaaagtgca gtggaaggtc gataacgccc tgcagtccgg caacagccag 480

gagagtgtga ctgaacagga ctcaaaagat agcacctatt ccctgtctag tacactgact 540

ctgtccaagg ctgattacga gaagcacaaa gtgtatgcat gcgaagtgac acatcaggga 600

ctgtcaagcc ccgtgactaa gtcttttaac cggggcgaat gt 642

<210> 12
<211> 214
<212> PRT
<213> Artificial

<220>
<223> Amino acid sequence of the light chain of 14C12H1L1 (hG1WT)

<400> 12

Asp Ile Gln Met Thr Gln Ser Pro Ser Ser Met Ser Ala Ser Val Gly
1 5 10 15

Asp Arg Val Thr Phe Thr Cys Arg Ala Ser Gln Asp Ile Asn Thr Tyr
20 25 30

Leu Ser Trp Phe Gln Gln Lys Pro Gly Lys Ser Pro Lys Thr Leu Ile
35 40 45

Tyr Arg Ala Asn Arg Leu Val Ser Gly Val Pro Ser Arg Phe Ser Gly
50 55 60

Ser Gly Ser Gly Gln Asp Tyr Thr Leu Thr Ile Ser Ser Leu Gln Pro
65 70 75 80

Glu Asp Met Ala Thr Tyr Tyr Cys Leu Gln Tyr Asp Glu Phe Pro Leu
85 90 95

Thr Phe Gly Ala Gly Thr Lys Leu Glu Leu Lys Arg Thr Val Ala Ala
100 105 110

Pro Ser Val Phe Ile Phe Pro Pro Ser Asp Glu Gln Leu Lys Ser Gly
115 120 125

Thr Ala Ser Val Val Cys Leu Leu Asn Asn Phe Tyr Pro Arg Glu Ala
130 135 140

Lys Val Gln Trp Lys Val Asp Asn Ala Leu Gln Ser Gly Asn Ser Gln
145 150 155 160

Glu Ser Val Thr Glu Gln Asp Ser Lys Asp Ser Thr Tyr Ser Leu Ser
165 170 175

Ser Thr Leu Thr Leu Ser Lys Ala Asp Tyr Glu Lys His Lys Val Tyr
180 185 190

Ala Cys Glu Val Thr His Gln Gly Leu Ser Ser Pro Val Thr Lys Ser
195 200 205

Phe Asn Arg Gly Glu Cys
210

<210> 13
<211> 1335
<212> DNA
<213> Artificial

<220>
<223> Nucleic acid sequence of 14C12H1L1(hG4) heavy chain

<400> 13
gaagtgcagc tggtcgagtc tggggaggg ctggtgcagc ccggcgggtc actgcgactg 60

agctgcgcag cttccggatt cgcctttagc tcctacgaca tgtcctgggt gcgacaggca 120

ccaggaaagg gactggattg ggtcgctact atctcaggag gcgggagata cacctactat 180

cctgacagcg tcaagggccg gttcacaatc tctagagata acagtaagaa caatctgtat 240

ctgcagatga acagcctgag ggctgaggac accgcactgt actattgtgc caaccgctac 300

ggggaagcat ggtttgccta ttgggggcag ggaaccctgg tgacagtctc tagtgccagc 360

accaaagggc cctcggtctt ccccctggcg ccctgctcca ggagcacctc cgagagcaca 420

gccgccctgg gctgcctggt caaggactac ttccccgaac cggtgacggt gtcgtggaac 480

tcaggcgccc tgaccagcgg cgtgcacacc ttcccggctg tcctacagtc ctcaggactc 540

tactccctca gcagcgtggt gaccgtgccc tccagcagct tgggcacgaa gacctacacc 600

tgcaacgtag atcacaagcc cagcaacacc aaggtggaca agagagttga gtccaaatat 660

ggtcccccat gcccaccatg cccagcacct gagttcctgg ggggaccatc agtcttcctg 720

ttccccccaa aacccaagga cactctcatg atctcccgga cccctgaggt cacgtgcgtg 780

gtggtggacg tgagccagga agaccccgag gtccagttca actggtacgt ggatggcgtg 840

gaggtgcata atgccaagac aaagccgcgg gaggagcagt tcaacagcac gtaccgtgtg 900

gtcagcgtcc tcaccgtcct gcaccaggac tggctgaacg gcaaggagta caagtgcaag 960

gtctccaaca aaggcctccc gtcctccatc gagaaaacca tctccaaagc caaagggcag 1020

ccccgagagc cacaggtgta caccctgccc ccatcccagg aggagatgac caagaaccag 1080

gtcagcctga cctgcctggt caaaggcttc taccccagcg acatcgccgt ggagtgggag 1140

agcaatgggc agccggagaa caactacaag accacgcctc ccgtgctgga ctccgacggc 1200

tccttcttcc tctacagcag gctaaccgtg gacaagagca ggtggcagga ggggaatgtc 1260

ttctcatgct ccgtgatgca tgaggctctg cacaaccact acacacagaa gagcctctcc 1320

ctgtctctgg gtaaa 1335

<210> 14
<211> 445
<212> PRT
<213> Artificial

<220>
<223> Amino acid sequence of 14C12H1L1(hG4) heavy chain

<400> 14

```
Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Ala Phe Ser Ser Tyr
            20                  25                  30

Asp Met Ser Trp Val Arg Gln Ala Pro Gly Lys Gly Leu Asp Trp Val
        35                  40                  45

Ala Thr Ile Ser Gly Gly Gly Arg Tyr Thr Tyr Tyr Pro Asp Ser Val
    50                  55                  60
```

```
Lys Gly Arg Phe Thr Ile Ser Arg Asp Asn Ser Lys Asn Asn Leu Tyr
65                  70                  75                  80

Leu Gln Met Asn Ser Leu Arg Ala Glu Asp Thr Ala Leu Tyr Tyr Cys
                85                  90                  95

Ala Asn Arg Tyr Gly Glu Ala Trp Phe Ala Tyr Trp Gly Gln Gly Thr
            100                 105                 110

Leu Val Thr Val Ser Ser Ala Ser Thr Lys Gly Pro Ser Val Phe Pro
        115                 120                 125

Leu Ala Pro Cys Ser Arg Ser Thr Ser Glu Ser Thr Ala Ala Leu Gly
    130                 135                 140

Cys Leu Val Lys Asp Tyr Phe Pro Glu Pro Val Thr Val Ser Trp Asn
145                 150                 155                 160

Ser Gly Ala Leu Thr Ser Gly Val His Thr Phe Pro Ala Val Leu Gln
                165                 170                 175

Ser Ser Gly Leu Tyr Ser Leu Ser Ser Val Val Thr Val Pro Ser Ser
            180                 185                 190

Ser Leu Gly Thr Lys Thr Tyr Thr Cys Asn Val Asp His Lys Pro Ser
        195                 200                 205

Asn Thr Lys Val Asp Lys Arg Val Glu Ser Lys Tyr Gly Pro Pro Cys
    210                 215                 220

Pro Pro Cys Pro Ala Pro Glu Phe Leu Gly Gly Pro Ser Val Phe Leu
225                 230                 235                 240

Phe Pro Pro Lys Pro Lys Asp Thr Leu Met Ile Ser Arg Thr Pro Glu
                245                 250                 255

Val Thr Cys Val Val Val Asp Val Ser Gln Glu Asp Pro Glu Val Gln
            260                 265                 270

Phe Asn Trp Tyr Val Asp Gly Val Glu Val His Asn Ala Lys Thr Lys
        275                 280                 285

Pro Arg Glu Glu Gln Phe Asn Ser Thr Tyr Arg Val Val Ser Val Leu
    290                 295                 300

Thr Val Leu His Gln Asp Trp Leu Asn Gly Lys Glu Tyr Lys Cys Lys
```

305 310 315 320

Val Ser Asn Lys Gly Leu Pro Ser Ser Ile Glu Lys Thr Ile Ser Lys
325 330 335

Ala Lys Gly Gln Pro Arg Glu Pro Gln Val Tyr Thr Leu Pro Pro Ser
340 345 350

Gln Glu Glu Met Thr Lys Asn Gln Val Ser Leu Thr Cys Leu Val Lys
355 360 365

Gly Phe Tyr Pro Ser Asp Ile Ala Val Glu Trp Glu Ser Asn Gly Gln
370 375 380

Pro Glu Asn Asn Tyr Lys Thr Thr Pro Pro Val Leu Asp Ser Asp Gly
385 390 395 400

Ser Phe Phe Leu Tyr Ser Arg Leu Thr Val Asp Lys Ser Arg Trp Gln
405 410 415

Glu Gly Asn Val Phe Ser Cys Ser Val Met His Glu Ala Leu His Asn
420 425 430

His Tyr Thr Gln Lys Ser Leu Ser Leu Ser Leu Gly Lys
435 440 445

<210> 15
<211> 1344
<212> DNA
<213> Artificial

<220>
<223> Nucleic acid sequence of 14C12H1L1(hG1TM) heavy chain

<400> 15
gaagtgcagc tggtcgagtc tgggggaggg ctggtgcagc ccggcgggtc actgcgactg 60

agctgcgcag cttccggatt cgcctttagc tcctacgaca tgtcctgggt gcgacaggca 120

ccaggaaagg gactggattg ggtcgctact atctcaggag gcgggagata cacctactat 180

cctgacagcg tcaagggccg gttcacaatc tctagagata acagtaagaa caatctgtat 240

ctgcagatga acagcctgag ggctgaggac accgcactgt actattgtgc caaccgctac 300

ggggaagcat ggtttgccta ttgggggcag ggaaccctgg tgacagtctc tagtgccagc 360

accaaagggc ccagcgtgtt tcctctcgcc ccctcctcca aaagcaccag cggaggaacc 420

gctgctctcg gatgtctggt gaaggactac ttccctgaac ccgtcaccgt gagctggaat 480

agcggcgctc tgacaagcgg agtccataca ttccctgctg tgctgcaaag cagcggactc 540

tattccctgt ccagcgtcgt cacagtgccc agcagcagcc tgggcaccca gacctacatc 600

tgtaacgtca accacaagcc ctccaacacc aaggtggaca agaaagtgga gcccaaatcc 660

tgcgacaaga cacacacctg tcccccctgt cctgctcccg aagctgctgg agcccctagc 720

gtcttcctct ttcctcccaa acccaaggac accctcatga tcagcagaac ccctgaagtc 780

acctgtgtcg tcgtggatgt cagccatgag gaccccgagg tgaaattcaa ctggtatgtc 840

gatggcgtcg aggtgcacaa cgccaaaacc aagcccaggg aggaacagta caactccacc 900

tacagggtgg tgtccgtgct gacagtcctc caccaggact ggctgaacgg caaggagtac 960

aagtgcaagg tgtccaacaa ggctctccct gcccccattg agaagaccat cagcaaggcc 1020

aaaggccaac ccagggagcc ccaggtctat acactgcctc cctccaggga cgaactcacc 1080

aagaaccagg tgtccctgac ctgcctggtc aagggctttt atcccagcga catcgccgtc 1140

gagtgggagt ccaacggaca gcccgagaat aactacaaga ccacccctcc tgtcctcgac 1200

tccgacggct ccttcttcct gtacagcaag ctgaccgtgg acaaaagcag gtggcagcag 1260

ggaaacgtgt tctcctgcag cgtgatgcac gaagccctcc acaaccacta cacccagaaa 1320

agcctgtccc tgagccccgg caaa 1344

<210> 16
<211> 448
<212> PRT
<213> Artificial

<220>
<223> Amino acid sequence of 14C12H1L1(hG1TM) heavy chain

<400> 16

Glu Val Gln Leu Val Glu Ser Gly Gly Gly Leu Val Gln Pro Gly Gly
1 5 10 15

Ser Leu Arg Leu Ser Cys Ala Ala Ser Gly Phe Ala Phe Ser Ser Tyr
20 25 30

Asp Met Ser Trp Val Arg Gln Ala Pro Gly Lys Gly Leu Asp Trp Val
35 40 45

Ala Thr Ile Ser Gly Gly Gly Arg Tyr Thr Tyr Tyr Pro Asp Ser Val
50 55 60

Lys Gly Arg Phe Thr Ile Ser Arg Asp Asn Ser Lys Asn Asn Leu Tyr
65 70 75 80

Leu Gln Met Asn Ser Leu Arg Ala Glu Asp Thr Ala Leu Tyr Tyr Cys
85 90 95

Ala Asn Arg Tyr Gly Glu Ala Trp Phe Ala Tyr Trp Gly Gln Gly Thr

100 105 110

Leu Val Thr Val Ser Ser Ala Ser Thr Lys Gly Pro Ser Val Phe Pro
115 120 125

Leu Ala Pro Ser Ser Lys Ser Thr Ser Gly Gly Thr Ala Ala Leu Gly
130 135 140

Cys Leu Val Lys Asp Tyr Phe Pro Glu Pro Val Thr Val Ser Trp Asn
145 150 155 160

Ser Gly Ala Leu Thr Ser Gly Val His Thr Phe Pro Ala Val Leu Gln
165 170 175

Ser Ser Gly Leu Tyr Ser Leu Ser Ser Val Val Thr Val Pro Ser Ser
180 185 190

Ser Leu Gly Thr Gln Thr Tyr Ile Cys Asn Val Asn His Lys Pro Ser
195 200 205

Asn Thr Lys Val Asp Lys Lys Val Glu Pro Lys Ser Cys Asp Lys Thr
210 215 220

His Thr Cys Pro Pro Cys Pro Ala Pro Glu Ala Ala Gly Ala Pro Ser
225 230 235 240

Val Phe Leu Phe Pro Pro Lys Pro Lys Asp Thr Leu Met Ile Ser Arg
245 250 255

Thr Pro Glu Val Thr Cys Val Val Val Asp Val Ser His Glu Asp Pro
260 265 270

Glu Val Lys Phe Asn Trp Tyr Val Asp Gly Val Glu Val His Asn Ala
275 280 285

Lys Thr Lys Pro Arg Glu Glu Gln Tyr Asn Ser Thr Tyr Arg Val Val
290 295 300

Ser Val Leu Thr Val Leu His Gln Asp Trp Leu Asn Gly Lys Glu Tyr
305 310 315 320

Lys Cys Lys Val Ser Asn Lys Ala Leu Pro Ala Pro Ile Glu Lys Thr
325 330 335

Ile Ser Lys Ala Lys Gly Gln Pro Arg Glu Pro Gln Val Tyr Thr Leu
340 345 350

Pro Pro Ser Arg Asp Glu Leu Thr Lys Asn Gln Val Ser Leu Thr Cys
        355                 360                 365

Leu Val Lys Gly Phe Tyr Pro Ser Asp Ile Ala Val Glu Trp Glu Ser
    370                 375                 380

Asn Gly Gln Pro Glu Asn Asn Tyr Lys Thr Thr Pro Pro Val Leu Asp
385                 390                 395                 400

Ser Asp Gly Ser Phe Phe Leu Tyr Ser Lys Leu Thr Val Asp Lys Ser
                405                 410                 415

Arg Trp Gln Gln Gly Asn Val Phe Ser Cys Ser Val Met His Glu Ala
            420                 425                 430

Leu His Asn His Tyr Thr Gln Lys Ser Leu Ser Leu Ser Pro Gly Lys
        435                 440                 445

<210> 17
<211> 1344
<212> DNA
<213> Artificial

<220>
<223> Nucleic acid sequence of 14C12H1L1(hG1DM) heavy chain

<400> 17
gaagtgcagc tggtcgagtc tgggggaggg ctggtgcagc ccggcgggtc actgcgactg 60

agctgcgcag cttccggatt cgcctttagc tcctacgaca tgtcctgggt gcgacaggca 120

ccaggaaagg gactggattg ggtcgctact atctcaggag gcgggagata cacctactat 180

cctgacagcg tcaagggccg gttcacaatc tctagagata acagtaagaa caatctgtat 240

ctgcagatga acagcctgag ggctgaggac accgcactgt actattgtgc caaccgctac 300

ggggaagcat ggtttgccta ttgggggcag ggaaccctgg tgacagtctc tagtgctagc 360

accaaagggc ccagcgtgtt tcctctcgcc ccctcctcca aaagcaccag cggaggaacc 420

gctgctctcg gatgtctggt gaaggactac ttccctgaac ccgtcaccgt gagctggaat 480

agcggcgctc tgacaagcgg agtccataca ttccctgctg tgctgcaaag cagcggactc 540

tattccctgt ccagcgtcgt cacagtgccc agcagcagcc tgggcaccca gacctacatc 600

tgtaacgtca accacaagcc ctccaacacc aaggtggaca agaaagtgga gcccaaatcc 660

tgcgacaaga cacacacctg tcccccctgt cctgctcccg aagctgctgg aggccctagc 720

gtcttcctct ttcctcccaa acccaaggac accctcatga tcagcagaac ccctgaagtc 780

acctgtgtcg tcgtggatgt cagccatgag gaccccgagg tgaaattcaa ctggtatgtc 840

gatggcgtcg aggtgcacaa cgccaaaacc aagcccaggg aggaacagta caactccacc 900

tacagggtgg tgtccgtgct gacagtcctc caccaggact ggctgaacgg caaggagtac 960

aagtgcaagg tgtccaacaa ggctctccct gcccccattg agaagaccat cagcaaggcc 1020

aaaggccaac ccagggagcc ccaggtctat acactgcctc cctccaggga cgaactcacc 1080

aagaaccagg tgtccctgac ctgcctggtc aagggctttt atcccagcga catcgccgtc 1140

gagtgggagt ccaacggaca gcccgagaat aactacaaga ccacccctcc tgtcctcgac 1200

tccgacggct ccttcttcct gtacagcaag ctgaccgtgg acaaaagcag gtggcagcag 1260

ggaaacgtgt tctcctgcag cgtgatgcac gaagccctcc acaaccacta cacccagaaa 1320

agcctgtccc tgagccccgg caaa 1344

<210> 18
<211> 448
<212> PRT
<213> Artificial

<220>
<223> Amino acid sequence of 14C12H1L1(hG1DM) heavy chain

<400> 18

Glu Val Gln Leu Val Glu Ser Gly Gly Gly Leu Val Gln Pro Gly Gly
1 5 10 15

Ser Leu Arg Leu Ser Cys Ala Ala Ser Gly Phe Ala Phe Ser Ser Tyr
20 25 30

Asp Met Ser Trp Val Arg Gln Ala Pro Gly Lys Gly Leu Asp Trp Val
35 40 45

Ala Thr Ile Ser Gly Gly Gly Arg Tyr Thr Tyr Tyr Pro Asp Ser Val
50 55 60

Lys Gly Arg Phe Thr Ile Ser Arg Asp Asn Ser Lys Asn Asn Leu Tyr
65 70 75 80

Leu Gln Met Asn Ser Leu Arg Ala Glu Asp Thr Ala Leu Tyr Tyr Cys
85 90 95

Ala Asn Arg Tyr Gly Glu Ala Trp Phe Ala Tyr Trp Gly Gln Gly Thr
100 105 110

Leu Val Thr Val Ser Ser Ala Ser Thr Lys Gly Pro Ser Val Phe Pro
115 120 125

Leu Ala Pro Ser Ser Lys Ser Thr Ser Gly Gly Thr Ala Ala Leu Gly
130 135 140

```
Cys Leu Val Lys Asp Tyr Phe Pro Glu Pro Val Thr Val Ser Trp Asn
145                 150                 155                 160

Ser Gly Ala Leu Thr Ser Gly Val His Thr Phe Pro Ala Val Leu Gln
                165                 170                 175

Ser Ser Gly Leu Tyr Ser Leu Ser Ser Val Val Thr Val Pro Ser Ser
            180                 185                 190

Ser Leu Gly Thr Gln Thr Tyr Ile Cys Asn Val Asn His Lys Pro Ser
        195                 200                 205

Asn Thr Lys Val Asp Lys Lys Val Glu Pro Lys Ser Cys Asp Lys Thr
    210                 215                 220

His Thr Cys Pro Pro Cys Pro Ala Pro Glu Ala Ala Gly Gly Pro Ser
225                 230                 235                 240

Val Phe Leu Phe Pro Pro Lys Pro Lys Asp Thr Leu Met Ile Ser Arg
                245                 250                 255

Thr Pro Glu Val Thr Cys Val Val Val Asp Val Ser His Glu Asp Pro
            260                 265                 270

Glu Val Lys Phe Asn Trp Tyr Val Asp Gly Val Glu Val His Asn Ala
        275                 280                 285

Lys Thr Lys Pro Arg Glu Glu Gln Tyr Asn Ser Thr Tyr Arg Val Val
    290                 295                 300

Ser Val Leu Thr Val Leu His Gln Asp Trp Leu Asn Gly Lys Glu Tyr
305                 310                 315                 320

Lys Cys Lys Val Ser Asn Lys Ala Leu Pro Ala Pro Ile Glu Lys Thr
                325                 330                 335

Ile Ser Lys Ala Lys Gly Gln Pro Arg Glu Pro Gln Val Tyr Thr Leu
            340                 345                 350

Pro Pro Ser Arg Asp Glu Leu Thr Lys Asn Gln Val Ser Leu Thr Cys
        355                 360                 365

Leu Val Lys Gly Phe Tyr Pro Ser Asp Ile Ala Val Glu Trp Glu Ser
    370                 375                 380

Asn Gly Gln Pro Glu Asn Asn Tyr Lys Thr Thr Pro Pro Val Leu Asp
385                 390                 395                 400
```

Ser Asp Gly Ser Phe Phe Leu Tyr Ser Lys Leu Thr Val Asp Lys Ser
405 410 415

Arg Trp Gln Gln Gly Asn Val Phe Ser Cys Ser Val Met His Glu Ala
420 425 430

Leu His Asn His Tyr Thr Gln Lys Ser Leu Ser Leu Ser Pro Gly Lys
435 440 445

<210> 19
<211> 8
<212> PRT
<213> Artificial

<220>
<223> HCDR1

<400> 19

Gly Phe Ala Phe Ser Ser Tyr Asp
1 5

<210> 20
<211> 8
<212> PRT
<213> Artificial

<220>
<223> HCDR2

<400> 20

Ile Ser Gly Gly Gly Arg Tyr Thr
1 5

<210> 21
<211> 11
<212> PRT
<213> Artificial

<220>
<223> HCDR3

<400> 21

Ala Asn Arg Tyr Gly Glu Ala Trp Phe Ala Tyr
1 5 10

<210> 22
<211> 6
<212> PRT
<213> Artificial

<220>
<223> LCDR1

<400> 22

Gln Asp Ile Asn Thr Tyr
1 5

<210> 23
<211> 3
<212> PRT
<213> Artificial

<220>
<223> LCDR2

<400> 23

Arg Ala Asn
1

<210> 24
<211> 9
<212> PRT
<213> Artificial

<220>
<223> LCDR3

<400> 24

Leu Gln Tyr Asp Glu Phe Pro Leu Thr
1 5

## Description

TECHNICAL FIELD

The present invention relates to the field of tumor treatment and molecular immunology, and particularly, to an anti-PD-1 antibody and pharmaceutical use thereof. More particularly, the present invention relates to mutant anti-PD-1 antibodies.

BACKGROUND

The transmembrane receptor PD-1 (programmed cell death protein 1) is a member of the CD28 family, and is expressed in activated T cells, B cells and myeloid cells. Both ligands of PD-1, PDL1 (programmed cell death 1 ligand 1, or PDL-1) and PDL2 (programmed cell death 1 ligand 2, or PDL-2), are members of the B7 superfamily. PDL1 is expressed in a variety of cells including T cells, B cells, endothelial cells and epithelial cells, and PDL2 is expressed only in antigen presenting cells such as dendritic cells and macrophages.

The PD-1/PDL1 signaling pathway plays an important role in regulating immune tolerance, microbial infection and tumor immune escape. PD-1 is mainly expressed in immune cells such as T cells, and the ligand PDL1 of PD-1 is highly expressed in a plurality of human tumor tissues. Blocking the PD-1/PDL1 signaling pathway may activate inhibited T cells, which thus attack cancer cells. Blocking the PD-1/PDL1 signaling can promote the proliferation of tumor antigen-specific T cells, activate tumor cell killing process and further inhibit local tumor growth (Julie R et al., 2012, N Engl J Med., 366:2455-2465). PD-1/PD-L1 is an important specific immune checkpoint. The formation of a PD-1/PD-L1 complex transmits inhibitory signals and negatively regulates the immune response of T cells. It inhibits TCR-mediated T cell activation, cytokine production and T cell proliferation (Fife et al., (2011) Nature Immunology 10:1185-1193), induces the depletion or anergy in homologous antigen-specific T cells (Hofmeyer et al., (2011) Journal of Biomedicine and Biotechnology, 2011:1-9), promotes the differentiation of Th1 cells into Foxp3+ regulatory T cells (Armanath et al., (2011) Science Trans. Med., 3:1-13; Francisco et al., (2009) J. Exp. Med., 206:3015-3029), and induces the apoptosis of effector T cells. The disruption of PD-L1 genes results in an upregulated T cell response and the production of autoreactive T cells (Latchman et al., (2004) PNAS, 101:10691-10696). The blockade of PD-1 or PD-L1 by antibody leads to elevated anti-tumor immunity (Iwai et al., (2002) PNAS, 99:12293-12297). In the past nearly 20 years, researchers have made great efforts to develop a specific immune checkpoint inhibitor, expecting to provide new immunotherapeutic regimens for treating cancer. Among these, the innate T-lymphocyte immune system can respond to a variety of tumor antigens owning to its high anti-cancer capacity and broad and precise specificity. This emerging cancer immunotherapy enhances the anti-tumor immune response by the adoptive transfer of activated effector cells, the immunization against relevant antigens, or the provision of non-specific immunostimulants. Thus, PD-1/PD-L1-specific immune checkpoint inhibitors have potential for treating related cancers.

The mechanism of action of anti-PD-1 antibodies is to block the binding of PD-1 proteins on the surfaces of immune cells to ligands PDL1 or PDL2 thereof, and to activate the immune cells to kill a tumor. At present, there is still a need for developing a novel anti-PD-1 antibody to reduce or eliminate the damage caused by antibody-mediated ADCC, ADCP and/or CDC activity on immune cells to which the anti-PD-1 antibody binds, and to improve the efficacy of the antibody therapy. ADCC (antibody-dependent cell-mediated cytotoxicity) refers to killing of a target cell by a killer cell (NK cell, macrophage, etc.) that is mediated by binding of the Fab fragment of an antibody to an epitope of a virus-infected cell or a tumor cell and binding of the Fc fragment of the antibody to an Fc receptor (FcR) on the surface of the killer cell.

CDC (Complement-dependent cytotoxicity) refers to a lytic effect on target cells by a membrane-attacking complex that is formed by serial bindings of an antibody to corresponding antigens on the surfaces of the cell membranes and the complement C1q and activation of C2-C9.

Fc receptors belong to an immunoglobulin family that are expressed on the surface of specific immune cells to recognize antibody Fc regions and mediate immune responses. After the Fab region recognizes an antigen, the Fc region of the antibody binds to the Fc receptor on the immune cell (e.g., a killer cell) to initiate the response function of the immune cell, such as phagocytosis and ADCC.

According to the type of antibody recognized by the Fc receptor and the type of expression cells, Fc receptors are mainly classified into three types, FcγR, FcαR and FcεR. FcγR can be further classified into four subtypes, FcγRI (CD64), FcγRII (CD32), FcγRIII (CD16) and FcRn (neonatal Fc receptor). Among these, FcγRI, FcγRII and FcγRIII are closely associated with ADCC effect. FcγRIII is the most predominant molecule mediating ADCC, with two highly homologous subtypes, FcγRIIIa and FcγRIIIb, in different cell types. In FcγRIIIa populations, two subtypes distinguished by sites of single-nucleotide polymorphism (SNP), FcγRIIIa_V158 with high affinity and FcγRIIIa_F158 with low affinity, are present. FcγRI has higher affinity for the Fc region of IgG and participates in ADCC process; FcγRII comprises three subtypes, FcγRIIa, FcγRIIb and FcγRIIc (also referred to as CD32a, CD32b and CD32c, respectively), among which

FcγRIIa has ADCC activity; for FcγRIIa, two subtypes, FcγRIIa_H131 and FcγRIIa_R131, are present in humans due to single nucleotide mutation; FcγRIIb is an inhibitory receptor, and is a typical inhibitory FcγR that inhibits nearby ITAM pathways. For example, after the binding of the immune complex to BCR, the Fc fragment binds to FcγRIIb on the same cell, negatively regulating B cell activation and decreasing secretion of antibodies and cytokines (Hogarth PM, Pietersz GA., 2012, NATURE REVIEWS DRUG DISCOVERY, 11(4):311-331).

The IgG family comprises four members, IgG1, IgG2, IgG3 and IgG4, which differ in amino acids in the fragment crystallizable (Fc) region of the heavy chain constant region, resulting in their varying affinities for FcγRs. IgG1 is the most abundant subtype in humans and is also the most common subtype used in monoclonal antibody medication. IgG1 is capable of binding various FcγRs and is able to induce ADCC and CDC effects. IgG2 has the lowest affinity for FcγRs, but is still able to induce monocyte-mediated ADCC by binding to FcγRIIa. IgG3 features the highest binding capacity to FcγRs, and can induce ADCC and a greater CDC effect than IgG1. IgG4 molecules demonstrate a weak binding to FcγRs other than FcγRI, having a lower probability of causing CDC and NK cell-mediated ADCC. However, antibodies of the IgG4 subtype can mediate ADCP effects through binding to FcγRI, and the ADCP effects, present in antibody therapies targeting immune cells, may cause damage to immune cells, posing pharmacological adverse effects.

Zhang et al. (Zhang T et al, Cancer Immunol Immunother., 2018; 67(7):1079-1090.) and Dahan et al. (Dahan R et al., Cancer cell, 2015, 28(3):285-95.) reported that the binding of Fc fragments of antibodies targeting immune checkpoints such as PD-1 and CTLA-4 to Fc receptors negatively affects antibody-mediated anti-cancer activity, possibly due to Fc-dependent effector function-induced immune cell damage including antibody-dependent cell-mediated cytotoxicity, where antibody-dependent cellular phagocytosis (ADCP) is an important mechanism leading to immune cell damage.

Non-squamous non-small cell lung cancer (NSCLC) and squamous non-small cell lung cancer (sNSCLC) are both lung tissue malignancies. Current therapeutic strategies include early stage surgery. However, most diagnosed lung cancer patients are at the advanced stage, showing poor response to surgery and radiotherapy. Thus chemotherapy has become an important treatment. Currently, combined chemotherapy of platinum and other chemotherapeutics is still the first-line chemotherapy for lung cancer including advanced sNSCLC and NSCLC (Pfister DG. et al., J. Clin. Oncol., 2003, 22:330; De Ruysscher et al., (2006) Annals of Oncology, 17:543-552).

Chemotherapies are currently mainly classified into the following nine classes (He Jie, et al., Clinical Oncology, Beijing, People's Medical Publishing House, 2016:230-237). The first class are drugs that directly bind to DNA and prevent DNA replication, including various alkylating agents, mitomycin, bleomycin, dacarbazine, platinum-based drugs (e.g., cisplatin and carboplatin), camptothecins, and derivatives thereof. The second class are drugs for preventing nucleic acid biosynthesis, which mainly affect the enzyme system of tumor cells and block the synthesis of precursors of DNA and RNA, thereby inhibiting the formation of DNA or RNA, including methotrexate, fluorouracil, 6-mercaptopurine, hydroxyurea and cytarabine; such drugs mainly act on cells in S phase, and are antimetabolite chemotherapeutic drugs and cell cycle-specific anticancer drugs. The third class are chemotherapeutic drugs which affect transcription through the pharmacological mechanism that the drugs are inserted into the DNA double helix to form non-covalent binding with the DNA double helix, interfering with the transcription of genetic information on DNA to the DNA-dependent mRNA and causing compromised template function and hindered transcription. The fourth class are those affecting tubulin and mitosis, including vinca alkaloids, podophyllotoxins and taxanes. The fifth class are drugs affecting the function of ribosomes and blocking protein synthesis; representatives of such drugs are harringtonines, which inhibit the initiation of protein synthesis, decompose the ribosome and release new peptide chain, but do not block the binding of mRNA and tRNA to ribosomes; such drugs cause the reduction of nuclear DNA and cytoplasmic RNA and depolymerization of polysomes, and inhibit mitosis. The sixth class are drugs that affect the tumor cell membrane such as concanavalin (Con-A) and phytohemagglutinin (PHA); they can bind to glycoprotein receptors on the cell membrane, thereby affecting DNA synthesis in tumor cells and preventing tumor cell from dividing. The seventh class are drugs that induce apoptosis, such as arsenic trioxide. The eighth class are hormones that treat tumors by regulating the endocrine system, including estrogens, antiestrogens, progestogens, androgens, antiandrogens, corticosteroids, and anticorticosteroids (including dichlorodiphenyldichloroethane and aminoglutethimide). The ninth class are anticancer targeted therapies, including monoclonal antibodies, epidermal growth factor signaling inhibitors (e.g., targeted drugs against receptor tyrosine kinase pathway), ubiquitin-proteasome inhibitors, and angiogenesis inhibitors. However, in addition to killing tumor cells, chemotherapeutics also damage normal human cells, so conventional chemotherapy regimens for cancer patients often cause serious toxic and side effects. More importantly, in addition to obvious toxicity, chemotherapeutics only demonstrate a short-term control over the diseases and a low 5-year survival rate in patients receiving chemotherapeutics. Therefore, developing a medication or combination therapy with lower toxicity and higher efficacy is of great meaning.

Anlotinib is a quinoline derivative tyrosine kinase inhibitor. As a multi-target tyrosine kinase inhibitor (TKI), it affects tumor angiogenesis and proliferation signal transduction. The major targets include: receptor tyrosine kinases vascular endothelial growth factor receptors (VEGFRs) 1 to 3, epidermal growth factor receptor (EGFR), fibroblast growth factor receptors (FGFRs) 1 to 4, platelet-derived growth factor receptors (PDGFRs) α and β, and stem cell factor receptors (SCFRs) 7, 8 and 9. A phase 2 trial showed that anlotinib improved progression-free survival with the potential benefit

for overall survival (Han B, et al., Br J Cancer, 2018; 118(5):654-661). A multicenter, double-blind, randomized phase 3 clinical trial showed that anlotinib resulted in extended overall and progression-free survivals in Chinese patients. The finding suggested that anlotinib is well tolerated and is a potential third-line or further treatment for patients with advanced NSCLC (Han B, et al., JAMA Oncol., 2018 Nov.; 4(11):1569-1575).

Example 24 of Patent No. WO2008112407 discloses a quinoline-derived tyrosine kinase inhibitor 1-[[[4-(4-fluoro-2-methyl-1*H*-indol-5-yl)oxy-6-methoxyquinolin-7-yl]oxy]methyl] cyclopropylamine and a method for preparing the same. The structural formula of the quinoline-derived tyrosine kinase inhibitor is shown in formula I. Anlotinib hydrochloride is the hydrochloride salt of the compound of formula I.

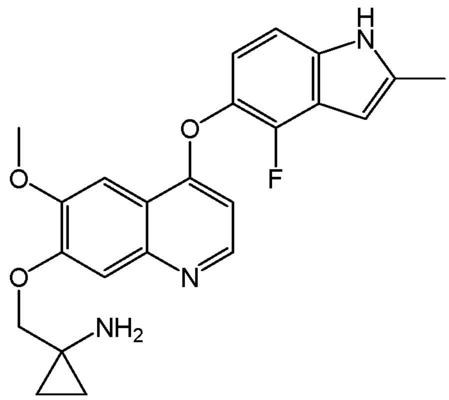

**formula I**

Lenvatinib, an oral multiple tyrosine kinase inhibitor developed by Eisai (Japan), is a multi-target receptor tyrosine kinase inhibitor that inhibits the kinase activity of VEGFR1 (FLT1), VEGFR2 (KDR) and VEGFR3 (FLT4). In addition to normal cellular function, lenvatinib also inhibits other receptor tyrosine kinases involved in pathogenic angiogenesis, tumor growth and cancer progression, including fibroblast growth factor (FGF) receptors FGFR1, FGFR2, FGFR3 and FGFR4, "rearranged during transfection" (RET) receptor, KIT and platelet-derived growth factor receptor α (PDGFRα). Lenvatinib also exhibits antiproliferative activity in hepatocellular carcinoma cell lines, which is dependent on activated FGFR signaling and simultaneous inhibition of phosphorylation of FGF receptor substrate 2α (FRS2α).

The structure of lenvatinib, 4-(3-chloro-4(cyclopropylaminocarbonyl)aminophenoxy)-7-methoxy-6-quinolinecarboxamide, is disclosed in Example 368 of U.S. Patent No. 7,612,208. U.S. Patent No. 7,253,286 discloses the mesylate salt form of lenvatinib (i.e., lenvatinib mesylate), named 4-[3-chloro-4-(cyclopropylureido)phenoxy]-7-methoxyquinoline-6-carboxamide mesylate, the chemical structure of which is provided below (formula II):

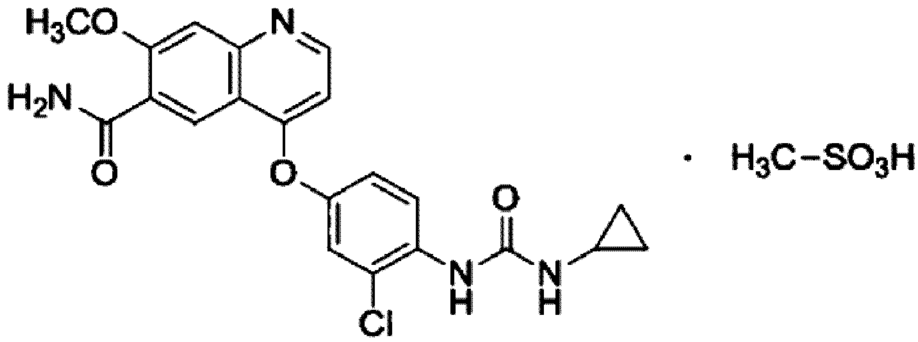

**formula II**

However, for a variety of tumors, the disease is still uncontrollable for a long term after chemotherapy, and the 5-year survival rate is still very low. Therefore, developing a medication or combination therapy with lower toxicity and higher efficacy is of great meaning.

SUMMARY

By intensive research and creative efforts, the inventor correspondingly modified the Fc fragment of the anti-PD-1 antibody structure to reduce the binding capacity of the Fc region to Fc receptors, thereby reducing ADCC, ADCP and/or CDC effects on T cells and increasing the efficacy of the anti-PD-1 antibody. The present invention is detailed below.

One aspect of the present invention relates to an antibody, wherein

a heavy chain variable region of the antibody comprises HCDR1-HCDR3 with amino acid sequences set forth in SEQ ID NOs: 19-21, respectively, and a light chain variable region of the antibody comprises LCDR1-LCDR3 with amino acid sequences set forth in SEQ ID NOs: 22-24, respectively;
the antibody is of human IgG1 subtype;
wherein, according to the EU numbering system, a heavy chain constant region of the antibody comprises mutations at any 2 or 3 of positions 234, 235 and 237, and an affinity constant of the antibody to FcγRIIIa and/or C1q is reduced after the mutation as compared to that before the mutation; preferably, the affinity constant is measured by a Fortebio Octet system.

In one embodiment of the present invention, the antibody is a monoclonal antibody.

In one embodiment of the present invention, the antibody is an anti-PD-1 antibody, preferably an anti-PD-1 monoclonal antibody.

In some embodiments of the present invention, for the antibody, according to the EU numbering system, the heavy chain constant region of the antibody comprises the following mutations at positions 234, 235 and/or 237:

L234A and L235A;
L234A and G237A;
L235A and G237A;
or
L234A, L235A and G237A.

In the present invention, letters before the position number represent amino acids before mutation, and letters after the position number represent amino acids after mutation, unless otherwise specified.

The present invention also relates to an antibody, wherein

a heavy chain variable region of the antibody comprises HCDR1-HCDR3 with amino acid sequences set forth in SEQ ID NOs: 19-21, respectively, and a light chain variable region of the antibody comprises LCDR1-LCDR3 with amino acid sequences set forth in SEQ ID NOs: 22-24, respectively;
the antibody is of human IgG1 subtype;
wherein according to the EU numbering system, a heavy chain constant region of the antibody comprises the following mutations at positions 234, 235 and/or 237:

L234A and L235A;
L234A and G237A;
L235A and G237A;
or
L234A, L235A and G237A.

In some embodiments of the present invention, according to the EU numbering system, the heavy chain constant region of the antibody further comprises one or more mutations selected from:
N297A, D265A, D270A, P238D, L328E, E233D, H268D, P271G, A330R, C226S, C229S, E233P, P331S, S267E, L328F, A330L, M252Y, S254T, T256E, N297Q, P238S, P238A, A327Q, A327G, P329A, K322A, T394D, G236R, G236A, L328R, A330S, P331S, H268A, E318A and K320A.

In some embodiments of the present invention, for the antibody,

the heavy chain variable region of the antibody comprises an amino acid sequence selected from SEQ ID NO: 2 and SEQ ID NO: 6; and
the light chain variable region of the antibody comprises an amino acid sequence selected from SEQ ID NO: 4 and SEQ ID NO: 8.

In some embodiments of the present invention, for the antibody,

the heavy chain variable region of the antibody comprises an amino acid sequence set forth in SEQ ID NO: 2, and the light chain variable region of the antibody comprises an amino acid sequence set forth in SEQ ID NO: 4;

the heavy chain variable region of the antibody comprises an amino acid sequence set forth in SEQ ID NO: 2, and the light chain variable region of the antibody comprises an amino acid sequence set forth in SEQ ID NO: 8;
the heavy chain variable region of the antibody comprises an amino acid sequence set forth in SEQ ID NO: 6, and the light chain variable region of the antibody comprises an amino acid sequence set forth in SEQ ID NO: 4; or
the heavy chain variable region of the antibody comprises an amino acid sequence set forth in SEQ ID NO: 6, and the light chain variable region of the antibody comprises an amino acid sequence set forth in SEQ ID NO: 8.

In one embodiment of the present invention, for the antibody,

the heavy chain is set forth in SEQ ID NO: 16, and the light chain is set forth in SEQ ID NO: 12;
or
the heavy chain is set forth in SEQ ID NO: 18, and the light chain is set forth in SEQ ID NO: 12.

The variable regions of the light chain and the heavy chain determine the binding of the antigen; the variable region of each chain comprises three hypervariable regions, i.e., complementarity determining regions (CDRs) (the CDRs of the heavy chain (H) include HCDR1, HCDR2, HCDR3, and the CDRs of the light chain (L) include LCDR1, LCDR2, LCDR3; defined by Kabat et al., see Sequences of Proteins of Immunological Interest, Fifth Edition (1991), Volumes 1-3, NIH Publication 91-3242, Bethesda MD).
The amino acid sequences of the CDR regions of the monoclonal antibody in (1) to (3) above are analyzed by technical means well known to those skilled in the art, for example, by a VBASE2 database:
The antibodies 14C12, 14C12H1L1(hG1WT), 14C12H1L1(hG1DM) and 14C12H1L1(hG1TM) involved in the present invention have the same CDRs.
The amino acid sequences of the 3 CDR regions of the heavy chain variable region are as follows:

HCDR1: GFAFSSYD (SEQ ID NO: 19),
HCDR2: ISGGGRYT (SEQ ID NO: 20), and
HCDR3: ANRYGEAWFAY (SEQ ID NO: 21).

The amino acid sequences of the 3 CDR regions of the light chain variable region are as follows:

LCDR1: QDINTY (SEQ ID NO: 22),
LCDR2: RAN (SEQ ID NO: 23), and
LCDR3: LQYDEFPLT (SEQ ID NO: 24).

In some embodiments of the present invention, the antibody binds to FcγRIIIa_F158, FcγRI, FcγRIIa_H131, FcγRIIIa_V158 and/or FcyRIIb with an affinity constant greater than about $10^{-7}$ M, for example, greater than about $10^{-6}$ M, $10^{-5}$ M, $10^{-4}$ M, or $10^{-3}$ M or greater; preferably, the affinity constant is measured by a Fortebio Octet system; preferably, the antibody has no binding signal or a binding signal of less than 0.1 nm to FcγRIIIa_F158, FcγRI, FcγRIIa_H131, FcγRIIIa_V158 and/or FcγRIIb; preferably, the binding signal refers to a response measured by a Fortebio Octet system.
In some embodiments of the present invention, the antibody binds to C1q with an affinity constant greater than about $10^{-9}$ M, for example, greater than about $10^{-8}$ M, $10^{-7}$ M, $10^{-6}$ M, or $10^{-5}$ M or greater; preferably, the affinity constant is measured by a Fortebio Octet system;

preferably, the antibody has no binding signal or a binding signal of less than 0.1 nm to C1q;
preferably, the binding signal refers to a response measured by a Fortebio Octet system.

In some embodiments of the present invention, the antibody is a monoclonal antibody.
In some embodiments of the present invention, the antibody is a humanized antibody.
Another aspect of the present invention relates to an isolated nucleic acid molecule encoding the antibody according to any embodiment of the present invention.
Yet another aspect of the present invention relates to a vector comprising the isolated nucleic acid molecule disclosed herein.
Yet another aspect of the present invention relates to a host cell comprising the isolated nucleic acid molecule or the vector disclosed herein.
Yet another aspect of the present invention relates to a conjugate, comprising an antibody and a conjugated moiety, wherein the antibody is the antibody according to any embodiment of the present invention, and the conjugated moiety is a detectable label; preferably, the conjugated moiety is a radioisotope, a fluorescent substance, a luminescent

substance, a colored substance, or an enzyme.

Yet another aspect of the present invention relates to a kit comprising the antibody according to any embodiment of the present invention or comprising the conjugate disclosed herein; preferably, the kit further comprises a second antibody specifically recognizing the antibody; optionally, the second antibody further comprises a detectable label, for example, a radioisotope, a fluorescent substance, a luminescent substance, a colored substance, or an enzyme.

Yet another aspect of the present invention relates to use of the antibody or the conjugate according to any embodiment of the present invention in preparing a kit for detecting the presence or level of PD-1 in a sample.

Yet another aspect of the present invention relates to a pharmaceutical composition comprising the antibody or the conjugate according to any embodiment of the present invention; optionally, the pharmaceutical composition further comprises a pharmaceutically acceptable carrier and/or excipient.

In one or more embodiments of the present invention, the pharmaceutical composition further comprises one or more anti-tumor chemotherapeutics;

preferably, the anti-tumor chemotherapeutic is a tyrosine kinase inhibitor; more preferably, the anti-tumor chemotherapeutic is anlotinib or a pharmaceutically acceptable salt thereof (e.g., hydrochloride salt), or lenvatinib or a pharmaceutically acceptable salt thereof (e.g., mesylate salt).

In one or more embodiments of the present invention, the unit dose of the pharmaceutical composition is 100-1000 mg, 200-800 mg, 200-500 mg, 300-600 mg, 400-500 mg, or 450 mg, based on the mass of the antibody.

Yet another aspect of the present invention relates to a therapeutic combination comprising: the antibody according to any embodiment of the present invention, and at least one (e.g., 1, 2 or 3) anti-tumor chemotherapeutic.

In one or more embodiments of the present invention, for the therapeutic combination, the anti-tumor chemotherapeutic is a tyrosine kinase inhibitor; preferably, the anti-tumor chemotherapeutic is anlotinib or a pharmaceutically acceptable salt thereof (e.g., hydrochloride salt), or lenvatinib or a pharmaceutically acceptable salt thereof (e.g., mesylate salt).

In one or more embodiments of the present invention, for the therapeutic combination, the unit dose of the antibody is 100-1000 mg, 200-800 mg, 200-500 mg, 300-600 mg, 400-500 mg, or 450 mg.

In one or more embodiments of the present invention, for the therapeutic combination, the unit dose of the anti-tumor chemotherapeutic is 0.1-100 mg, 0.5-50 mg, 0.5-10 mg, 1-10 mg, 2-8 mg, or 1-5 mg.

In one or more embodiments of the present invention, for the therapeutic combination, the unit dose of the anti-tumor chemotherapeutic is 1-20 mg, 2-15 mg, 4-12 mg, or 8-12 mg.

In one or more embodiments of the present invention, for the therapeutic combination, wherein
the therapeutic combination is a fixed combination, e.g., in the form of a solid pharmaceutical composition or a liquid pharmaceutical composition; or the therapeutic combination is a non-fixed combination, e.g., the anti-PD-1 antibody and the anti-tumor chemotherapeutic in the non-fixed combination are each in the form of a pharmaceutical composition.

Yet another aspect of the present invention relates to a kit product comprising the pharmaceutical composition according to any embodiment of the present invention or the therapeutic combination according to any embodiment of the present invention, and a package insert.

Yet another aspect of the present invention relates to use of the antibody according to any embodiment of the present invention, the conjugate disclosed herein, the pharmaceutical composition according to any embodiment of the present invention or the therapeutic combination according to any embodiment of the present invention in preparing a medicament for treating and/or preventing a tumor or anemia, or in preparing a medicament for diagnosing a tumor or anemia, wherein preferably the tumor is selected from one or more of melanoma, renal cancer, prostate cancer, bladder cancer, colon cancer, rectal cancer, gastric cancer, liver cancer, lung cancer, ovarian cancer, leukemia, nasopharyngeal cancer and endometrial cancer;

> preferably, the lung cancer is selected from one or more of non-small cell lung cancer, small cell lung cancer and squamous cell lung cancer;
> preferably, the gastric cancer is gastric adenocarcinoma or gastroesophageal junction adenocarcinoma;
> preferably, the tumor is a solid tumor of MSI-H/dMMR phenotype; preferably, the tumor is selected from one or more of the following tumors of MSI-H/dMMR phenotype:
> colon cancer, rectal cancer, endometrial cancer, gastric cancer, mesothelioma, sarcoma, adrenocortical carcinoma, malignant melanoma and ovarian germ cell neoplasm.

In one or more embodiments of the present invention, for the use, the tumor is a recurrent, metastatic (e.g., lymphatic metastasis, brain metastasis, and/or bone metastasis) or refractory tumor.

MSI refers to microsatellite instability. Microsatellites are short tandem repeats throughout the human genome, including 10-50 repeats of one, two or more nucleotides. Microsatellites in certain abnormal cells, such as tumors, are altered in length by insertion or deletion of repeat units as compared to normal cells. Such alteration is referred to as MSI. Based on instability and extent, MSI can be classified as microsatellite instability-high (MSI-H), microsatellite

instability-low (MSI-L) and microsatellite stable (MSS). The major cause of MSI is DNA mismatch repair (MMR) deficiency. Human mismatch repair genes (MMR genes) can express corresponding mismatch repair proteins through transcription and translation. Absence of any MMR protein may lead to mismatch repair deficiency, and basepair mismatch will accumulate in the process of DNA replication due to such deficiency, ultimately resulting in MSI. About 15% of colorectal cancers are attributed to the MSI pathway. This was first reported in colorectal cancer, and may also occur in gastric cancer, endometrial cancer, adrenocortical carcinoma and the like (Baretti M et al., Pharmacol Ther., 2018; 189:45-62). MSI-H/dMMR characteristics were also found in mesothelioma, sarcoma, adrenocortical carcinoma, malignant melanoma and ovarian germ cell neoplasm in subsequent studies.

MSI-H and dMMR represent the results of two different assays and are biologically consistent, called MSI-H/dMMR or MSI-high/dMMR, while MSI-L and MSS are phenotypes of proficient MMR (pMMR). The detection of dMMR is to perform an immunohistochemical assay of protein expression for four mismatch genes of MSH2, MLH1, MSH6 and PMS2 based on tumor specimens (including surgical specimens and aspiration specimens). Absence of any of the four proteins confirms the dMMR; positive results of all the four proteins indicate pMMR, i.e., a complete mismatch repair function. The detection of MSI is to match the length of the repeated DNA sequences (microsatellite sequences) in tumor cells and somatic cells, and to compare the lengths. When 5 standard loci are detected using PCR based on the American NCI standard, inconsistencies in two or more loci indicate instability, defined as MSI-H, one inconsistent locus indicates MSI-L, and 5 consistent loci indicate MSS. High-throughput sequencing (also referred to as next-generation sequencing, or NGS) can also be used as a method for detecting microsatellite instability. When more microsatellite loci are selected, such as more than 5 loci or additional microsatellite loci, for PCR assay, inconsistency in ≥30% loci is defined as MSI-H, consistency in all loci is defined as MSS, and inconsistency between 0 and 30% is defined as MSI-L.

Yet another aspect of the present invention relates to use of the antibody according to any embodiment of the present invention, the conjugate described herein, the pharmaceutical composition according to any embodiment of the present invention or the therapeutic combination according to any embodiment of the present invention in preparing:

a medicament for blocking the binding of PD-1 to PD-L1,
a medicament for down-regulating the activity or level of PD-1,
a medicament for relieving the immunosuppression of PD-1 in an organism, or
a medicament for elevating IFN-γ and/or IL-2 expression in T lymphocytes.

Interferon γ (IFN-γ) is produced primarily and innately by natural killer cells (NK) and natural killer T cells (NKT) and is produced by effector T cells such as CD4 Th1 cells and CD8 cytotoxic T lymphocytes stimulated by specific antigens. As an important innate and acquired immune cytokine, IFN-γ plays an important role in fighting or inhibiting viral infection and certain bacterial and protozoal disease infections. Meanwhile, IFN-γ can activate macrophages, induce the expression of type II major histocompatibility complex, and activate the immune response to control tumor progression (Schoenborn JR, Wilson CB., Regulation of Interferon-γ During Innate and Adaptive Immune Responses, Advances in Immunology, 2007, 96:41-101). In the *in vitro* experiment of the present invention, the antibody disclosed herein can induce the IFN-γ secretion to activate the immune response. Interleukin 2 (IL-2) is produced by T cells. It is a growth factor that regulates T cell subgroups, and an important factor in regulating immune responses. It promotes the proliferation of activated B cells, and participates in antibody responses, hematopoiesis and tumor surveillance. Recombinant human IL-2 has been approved by the U.S. FDA for treating malignancies, including melanoma and renal tumor (Chavez, A.R., et al.,

Pharmacologic administration of interleukin-2, Ann. N.Y. Acad. Sci., 2009, 1182:p.14-27). *In-vitro* studies demonstrated that the antibody disclosed herein can specifically relieve the immunosuppression of PD-1, activate T cells, and induce IL-2 generation, and is promising in wide applications in therapies against diseases such as tumors and parasite infections.

Yet another aspect of the present invention relates to an *in vivo* or *in vitro* method comprising: administering to a subject in need an effective amount of the antibody according to any embodiment of the present invention, the conjugate described herein, the pharmaceutical composition according to any embodiment of the present invention or the therapeutic combination according to any embodiment of the present invention. The method is selected from:

a method for blocking the binding of PD-1 to PD-L1,
a method for down-regulating the activity or level of PD-1,
a method for relieving the immunosuppression of PD-1 in an organism, or
a method for elevating IFN-γ and/or IL-2 expression in T lymphocytes.

Also related are the antibody according to any embodiment of the present invention, the conjugate described herein, the pharmaceutical composition according to any embodiment of the present invention or the therapeutic combination according to any embodiment of the present invention for use in treating and/or preventing a tumor or anemia,

or in diagnosing a tumor or anemia, wherein preferably the tumor is selected from one or more of melanoma, renal cancer, prostate cancer, bladder cancer, colon cancer, rectal cancer, gastric cancer, liver cancer, lung cancer, ovarian cancer, leukemia, nasopharyngeal cancer and endometrial cancer;

preferably, the lung cancer is selected from one or more of non-small cell lung cancer, small cell lung cancer and squamous cell lung cancer;
preferably, the gastric cancer is gastric adenocarcinoma or gastroesophageal junction adenocarcinoma;
preferably, the tumor is a solid tumor of MSI-H/dMMR phenotype; preferably, the tumor is selected from one or more of the following tumors of MSI-H/dMMR phenotype:
colon cancer, rectal cancer, endometrial cancer, gastric cancer, mesothelioma, sarcoma, adrenocortical carcinoma, malignant melanoma and ovarian germ cell neoplasm.

In one or more embodiments of the present invention, for the antibody or the conjugate described herein, the tumor is a recurrent, metastatic (e.g., lymphatic metastasis, brain metastasis, and/or bone metastasis) or refractory tumor.
The antibody according to any embodiment of the present invention, the conjugate described herein, the pharmaceutical composition according to any embodiment of the present invention or the therapeutic combination according to any embodiment of the present invention are used for:

blocking the binding of PD-1 to PD-L1,
down-regulating the activity or level of PD-1,
relieving the immunosuppression of PD-1 in an organism, or
elevating IFN-$\gamma$ and/or IL-2 expression in T lymphocytes.

Yet another aspect of the present invention relates to a method of treating and/or preventing a tumor or anemia, or a method of diagnosing a tumor or anemia, comprising: administering to a subject in need an effective amount of the antibody according to any embodiment of the present invention, the conjugate described herein, the pharmaceutical composition according to any embodiment of the present invention or the therapeutic combination according to any embodiment of the present invention, wherein preferably the tumor is selected from one or more of melanoma, renal cancer, prostate cancer, bladder cancer, colon cancer, rectal cancer, gastric cancer, liver cancer, lung cancer, ovarian cancer, leukemia, nasopharyngeal cancer and endometrial cancer;

preferably, the lung cancer is selected from one or more of non-small cell lung cancer, small cell lung cancer and squamous cell lung cancer;
preferably, the gastric cancer is gastric adenocarcinoma or gastroesophageal junction adenocarcinoma;
preferably, the tumor is a solid tumor of MSI-H/dMMR phenotype; preferably, the tumor is selected from one or more of the following tumors of MSI-H/dMMR phenotype:
colon cancer, rectal cancer, endometrial cancer, gastric cancer, mesothelioma, sarcoma, adrenocortical carcinoma, malignant melanoma and ovarian germ cell neoplasm.

In one or more embodiments of the present invention, for the method, the tumor is a recurrent, metastatic (e.g., lymphatic metastasis, brain metastasis, and/or bone metastasis) or refractory tumor.
In one or more embodiments of the present invention, for the method, the administration is before or after a surgical treatment and/or before or after a radiotherapy.
In one or more embodiments of the present invention, the method, wherein

the unit dose of the anti-PD-1 antibody is 0.1-100 mg, preferably 1-10 mg (e.g., 1 mg, 2 mg, 3 mg, 4 mg, 5 mg, 6 mg, 7 mg, 8 mg, 9 mg or 10 mg) per kg body weight; alternatively, the unit dose of the anti-PD-1 antibody is 10-1000 mg (e.g., about 100 mg, about 150 mg, about 200 mg, about 250 mg, about 300 mg, about 350 mg, about 400 mg, about 450 mg, about 500 mg, about 600 mg, about 700 mg, about 800 mg, about 900 mg or about 1000 mg), preferably 50-500 mg, 100-400 mg, 150-300 mg, 150-250 mg or 200 mg in each subject;
preferably, the dose is given once every 3 days, 4 days, 5 days, 6 days, 10 days, 1 week, 2 weeks or 3 weeks;
preferably, the route of administration is intravenous drip infusion or intravenous injection. In some embodiments, the administration of the anti-PD-1 antibody is performed in cycles of 2 weeks (14 days) or 3 weeks (21 days), and preferably, the anti-PD-1 antibody is administered intravenously on the first day (D1) of each cycle. For example, the anti-PD-1 antibody is administered once every two weeks (q2w) or three weeks (q3w).

In the present invention, unless otherwise defined, the scientific and technical terms used herein have the meanings generally understood by those skilled in the art. In addition, the laboratory operations of cell culture, molecular

genetics, nucleic acid chemistry and immunology used herein are the routine procedures widely used in the corresponding fields. Meanwhile, in order to better understand the present invention, the definitions and explanations of the relevant terms are provided below.

As used herein, when referring to the amino acid sequence of PD-1 protein (programmed cell death protein 1, NCBI GenBank: NP_005009.2), it includes the full length of the PD-1 protein, or the extracellular fragment PD-1ECD of PD-1 or a fragment comprising PD-1ECD, and it also includes a fusion protein of PD-1ECD, such as a fragment fused to an Fc protein fragment of a mouse or human IgG (mFc or hFc). However, those skilled in the art will appreciate that in the amino acid sequence of the PD-1 protein, mutations or variations (including but not limited to, substitutions, deletions and/or additions) can be naturally produced or artificially introduced without affecting biological functions thereof. Therefore, in the present invention, the term "PD-1 protein" should include all such sequences and natural or artificial variants thereof. Moreover, when describing the sequence fragment of the PD-1 protein, it includes not only the sequence fragment but also a corresponding sequence fragment in natural or artificial variants thereof.

As used herein, when referring to the amino acid sequence of PDL1 protein (NCBI Genebank ID: NP_054862.1), it includes the full length of PDL1 protein, or the extracellular fragment PDL1ECD of PDL1 or a fragment comprising PDL1ECD; also included are fusion proteins of PDL1ECD, such as a fragment fused to an Fc protein fragment of a mouse or human IgG (mFc or hFc). However, those skilled in the art will appreciate that in the amino acid sequence of the PDL1 protein, mutations or variations (including but not limited to, substitutions, deletions and/or additions) can be naturally produced or artificially introduced without affecting biological functions thereof. Therefore, in the present invention, the term "PDL1 protein" shall include all such sequences and natural or artificial variants thereof. Moreover, when describing the sequence fragment of the PDL1 protein, it includes not only a PDL1 sequence fragment but also a corresponding sequence fragment in natural or artificial variants thereof.

As used herein, the term $EC_{50}$ refers to the half maximum effective concentration.

As used herein, the term "antibody" refers to an immunoglobulin molecule that generally consists of two pairs of polypeptide chains (each pair with one "light" (L) chain and one "heavy" (H) chain). Antibody light chains are classified as $\kappa$ and $\lambda$ light chains. Heavy chains are classified as $\mu$, $\delta$, $\gamma$, $\alpha$, or $\varepsilon$. Isotypes of antibodies are defined as IgM, IgD, IgG, IgA, and IgE. In light chains and heavy chains, the variable region and constant region are linked by a "J" region of about 12 or more amino acids, and the heavy chain also comprises a "D" region of about 3 or more amino acids. Each heavy chain consists of a heavy chain variable region ($V_H$) and a heavy chain constant region ($C_H$). The heavy chain constant region consists of 3 domains ($C_{H1}$, $C_{H2}$, and $C_{H3}$). Each light chain consists of a light chain variable region ($V_L$) and a light chain constant region ($C_L$). The light chain constant region consists of one domain $C_L$. The constant region of the antibody can mediate the binding of immunoglobulins to host tissues or factors, including the binding of various cells of the immune system (e.g., effector cells) to the first component (C1q) of classical complement system. The $V_H$ and $V_L$ regions can be further subdivided into highly variable regions (called complementarity determining regions (CDRs)), between which conservative regions called framework regions (FRs) are distributed. Each $V_H$ and $V_L$ consists of 3 CDRs and 4 FRs arranged from amino terminus to carboxyl terminus in the following order: FR1, CDR1, FR2, CDR2, FR3, CDR3, FR4. The variable regions ($V_H$ and $V_L$) of each heavy chain/light chain pair form an antibody binding site. The assignment of amino acids to each region or domain follows the definition of Kabat Sequences of Proteins of Immunological Interest (National Institutes of Health, Bethesda, MD. (1987 and 1991)), Chothia & Lesk, (1987) J. Mol. Biol., 196:901-917, or Chothia et al. (1989) Nature, 342:878-883. The term "antibody" is not limited by any specific method for producing antibody. For example, the antibody includes, in particular, a recombinant antibody, a monoclonal antibody, and a polyclonal antibody. The antibody can be antibodies of different isotypes, such as IgG (e.g., subtype IgG1, IgG2, IgG3 or IgG4), IgA1, IgA2, IgD, IgE or IgM.

As used herein, the terms "mAb" and "monoclonal antibody" refer to an antibody or a fragment thereof that is derived from a group of highly homologous antibodies, i.e., from a group of identical antibody molecules, except for natural mutations that may occur spontaneously. The monoclonal antibody is highly specific for a single epitope on an antigen. The Polyclonal antibody, relative to the monoclonal antibody, generally comprises at least two or more different antibodies which generally recognize different epitopes on an antigen. Monoclonal antibodies can generally be obtained by hybridoma technique first reported by Kohler et al. (Nature, 256:495, 1975), and can also be obtained by recombinant DNA technique (for example, see U.S. Patent No. 4,816,567).

As used herein, the term "humanized antibody" refers to an antibody or antibody fragment obtained when all or a part of CDR regions of a human immunoglobulin (receptor antibody) are replaced by the CDR regions of a non-human antibody (donor antibody), wherein the donor antibody may be a non-human (e.g., mouse, rat or rabbit) antibody having expected specificity, affinity or reactivity. In addition, some amino acid residues in the framework regions (FRs) of the receptor antibody can also be replaced by the amino acid residues of corresponding non-human antibodies or by the amino acid residues of other antibodies to further improve or optimize the performance of the antibody. For more details on humanized antibodies, see, for example, Jones et al., Nature, 321:522-525 (1986); Reichmann et al., Nature, 332:323-329 (1988); Presta, Curr. Op. Struct. Biol., 2:593-596 (1992); and Clark, Immunol. Today, 21:397-402 (2000).

As used herein, the term "isolated" refers to obtaining by artificial means from natural state. If a certain "isolated"

substance or component is present in nature, it may be the case that change occurs in its natural environment, or that it is isolated from the natural environment, or both. For example, if a certain non-isolated polynucleotide or polypeptide naturally exists in a certain living animal, such a polynucleotide or polypeptide with a higher purity isolated from such a natural state is called an isolated polynucleotide or polypeptide. The term "isolated" does not exclude the existence of artificial or synthetic substances or other impurities that do not affect the activity of the substance.

As used herein, the term "vector" refers to a nucleic acid vehicle into which a polynucleotide can be inserted. When a vector allows the expression of the protein encoded by the inserted polynucleotide, the vector is called an expression vector. The vector can be introduced into a host cell by transformation, transduction, or transfection so that the genetic substance elements carried by the vector can be expressed in the host cell. Vectors are well known to those skilled in the art, including but not limited to: plasmids; phagemids; cosmids; artificial chromosomes, such as yeast artificial chromosome (YAC), bacterial artificial chromosome (BAC), or P1-derived artificial chromosome (PAC); phages such as lambda phages or M13 phages; and animal viruses. Animal viruses that can be used as vectors include, but are not limited to retroviruses (including lentiviruses), adenoviruses, adeno-associated viruses, herpes viruses (such as herpes simplex virus), poxviruses, baculoviruses, papillomaviruses, and papovaviruses (such as SV40). A vector may comprise a variety of elements that control expression, including, but not limited to promoter sequences, transcription initiation sequences, enhancer sequences, selection elements, and reporter genes. In addition, the vector may further comprise a replication initiation site.

As used herein, the term "host cell" refers to cells to which vectors can be introduced, including, but not limited to, prokaryotic cells such as *E. coli* or *bacillus subtilis*, fungal cells such as yeast cells or *aspergillus*, insect cells such as S2 drosophila cells or Sf9, or animal cells such as fibroblasts, CHO cells, COS cells, NSO cells, HeLa cells, BHK cells, HEK 293 cells, or human cells.

As used herein, the term "specific binding" refers to a non-random binding reaction between two molecules, such as a reaction between an antibody and an antigen it targets. In some embodiments, an antibody specifically binding to an antigen (or an antibody specific to an antigen) means that the antibody binds to the antigen with an affinity ($K_D$) of less than about $10^{-5}$ M, e.g., less than about $10^{-6}$ M, $10^{-7}$ M, $10^{-8}$ M, $10^{-9}$ M or $10^{-10}$ M or less.

As used herein, the term "$K_D$" refers to a dissociation equilibrium constant for a specific antibody-antigen interaction, which is used to describe the binding affinity between the antibody and the antigen. A smaller equilibrium dissociation constant indicates a stronger antibody-antigen binding and a higher affinity between the antibody and the antigen. Generally, antibodies bind to antigens (e.g., PD-1 protein) with a dissociation equilibrium constant ($K_D$) of less than about $10^{-5}$ M, such as less than about $10^{-6}$ M, $10^{-7}$ M, $10^{-8}$ M, $10^{-9}$ M or $10^{-10}$ M or less. $K_D$ can be determined using methods known to those skilled in the art, e.g., using a Fortebio system.

As used herein, the terms "monoclonal antibody" and "mAb" have the same meaning and can be used interchangeably; the terms "polyclonal antibody" and "pAb" have the same meaning and can be used interchangeably; the terms "polypeptide" and "protein" have the same meaning and can be used interchangeably. Besides, amino acids are generally represented herein by single-letter and three-letter abbreviations known in the art. For example, alanine can be represented by A or Ala.

As used herein, the term "pharmaceutically acceptable carrier and/or excipient" refers to a carrier and/or excipient that is pharmacologically and/or physiologically compatible with the subject and the active ingredient. Such carriers and/or excipients are well known in the art (see, e.g., Remington’s Pharmaceutical Sciences, edited by Gennaro AR, 19th Ed., Pennsylvania, Mack Publishing Company, 1995), including but not limited to: pH regulators, surfactants, adjuvants, and ionic strength enhancers. For example, the pH regulators include, but are not limited to, phosphate buffer; the surfactants include, but are not limited to, cationic, anionic, or non-ionic surfactants, such as Tween-80; the ionic strength enhancers include, but are not limited to, sodium chloride.

As used herein, the term "adjuvant" refers to a non-specific immune enhancer, which can enhance the immune response of an organism to antigens or change the type of immune response when delivered into the organism together with the antigens or in advance. There are various adjuvants, including, but not limited to, aluminum adjuvant (e.g., aluminum hydroxide), Freund’s adjuvant (e.g., complete Freund’s adjuvant and incomplete Freund’s adjuvant), *Corynebacterium parvum*, lipopolysaccharide, cytokine, etc. The Freund’s adjuvant is the most commonly used adjuvant in animal experiments. The aluminum hydroxide adjuvant is used more frequently in clinical trials.

As used herein, the term "effective amount" refers to an amount sufficient to obtain or at least partially obtain desired effects. For example, a prophylactically effective amount against a disease (e.g., RA) refers to an amount sufficient to prevent, stop, or delay the onset of the disease (e.g., RA); a therapeutically effective amount refers to an amount sufficient to cure or at least partially stop a disease and complications thereof in patients suffering from the disease. It is undoubtedly within the ability of those skilled in the art to determine such an effective amount. For example, the amount effective for therapeutic purpose will depend on the severity of the disease to be treated, the overall state of the patient’s own immune system, the general condition of the patient such as age, body weight and gender, the route of administration, and other treatments given concurrently, etc.

As used herein, the term "completely eliminated" refers to the absence of binding signal or an extremely weak

binding signal as detected by existing instrumentation (e.g., a Fortebio Octet system). In one embodiment of the present invention, the absence of binding signal or the extremely weak binding signal refers to a binding signal (i.e., response) below 0.1 nm. A "recurrent" cancer is one that regenerates at the original site or a distant site after response to a previous treatment (e.g., surgery). A "locally recurrent" cancer is one that occurs at the same site as the previously treated cancer after treatment.

A "metastatic" cancer refers to one that spreads from one part of the body (e.g., the lungs) to another.

Beneficial Effects

The present invention achieves one or more of the following technical effects (1) to (9):

(1) The antibodies disclosed herein, in particular 14C12H1L1(hG1TM) and 14C12H1L1(hG1WT), can effectively block the immunosuppression of immune cells induced by PD-1/PDL1 binding, and induce secretion of IFN-γ and IL-2 in human peripheral blood mononuclear cells.
(2) The present invention completely eliminates the binding activity of the antibodies, in particular 14C12H1L1(hG1TM), to Fc receptors, i.e., FcγRI, FcγRIIa_H131, FcγRIIIa_V158 and/or FcγRIIIa_F158, thereby eliminating the ADCC activity or ADCP activity.
(3) The present invention completely eliminates the binding activity of the antibodies, in particular 14C12H1L1(hG1TM), to complement C1q, thereby eliminating the CDC activity.
(4) The present invention significantly reduces the binding activity of the antibodies, e.g., 14C12H1L1 (hG1DM), to Fc receptors, i.e., FcγRI, FcγRIIa_H131, FcγRIIa_R131 and/or FcγRIIIa_V158 and completely eliminates the binding to FcγRIIIa_F158 and/or FcγRIIb, thereby significantly reducing the ADCC activity.
(5) The present invention completely eliminates the binding activity of the antibodies, in particular 14C12H1L1(hG1DM), to complement C1q, thereby eliminating the CDC activity.
(6) The monoclonal antibodies of the present invention, in particular 14C12H1L1(hG1TM), 14C12H1L1(hG1DM) and 14C12H1L1(hG1WT), can be well and specifically bind to PD-1, and can effectively block the binding of PD-1 to PDL1, thereby specifically relieving the immunosuppression by PD-1 in an organism and activating T lymphocytes. Among these, the PD-1 antibody 14C12H1L1(hG1TM) has an significantly stronger induction effect than those of the control anti-PD-1 antibody nivolumab and the control anti-PDLI antibody 5C10H2L2-IgG1mt on IFN-γ and IL-2 secretion, showing potential for use in preparing a medicament for preventing and treating tumors.
(7) The antibodies disclosed herein have ability to effectively prevent and treat the tumors described above.
(8) The antibodies disclosed herein have lower toxic and side effects.
(9) The anti-PD-1 antibodies disclosed herein or the anti-PD-1 antibodies in the therapeutic combination disclosed herein have a synergistic effect with a chemotherapeutic.

BRIEF DESCRIPTION OF THE DRAWINGS

FIG. 1: Affinity constant assay of 14C12H1L1(hG1DM) to FcyRI. The antibody concentrations for the curve pairs from top to bottom are 50 nM, 25 nM, 12.5 nM, 6.25 nM and 3.12 nM, respectively.
FIG. 2: Affinity constant assay of 14C12H1L1(hG4) to FcyRI. The antibody concentrations for the curve pairs from top to bottom are 50 nM, 25 nM, 12.5 nM, 6.25 nM and 3.12 nM, respectively.
FIG. 3: Affinity constant assay of 14C12H1L1(hG1WT) to FcyRI. The antibody concentrations for the curve pairs from top to bottom are 50 nM, 25 nM, 12.5 nM, 6.25 nM and 3.12 nM, respectively.
FIG. 4: Affinity constant assay of 14C12H1L1(hG1TM) to FcyRI. The antibody concentrations for the curve pairs from top to bottom are 50 nM, 25 nM, 12.5 nM, 6.25 nM and 3.12 nM, respectively.
FIG. 5: Affinity constant assay of 5C10H2L2-IgG1mt to FcyRI. The antibody concentrations for the curve pairs from top to bottom are 50 nM, 25 nM, 12.5 nM, 6.25 nM and 3.12 nM, respectively.
FIG. 6: Affinity constant assay of 14C12H1L1(hG1DM) to FcγRIIIa_V158. The antibody concentrations for the curve pairs from top to bottom are 500 nM, 250 nM, 125 nM, 62.5 nM and 31.25 nM, respectively.
FIG. 7: Affinity constant assay of 14C12H1L1(hG4) to FcγRIIIa_V158. The antibody concentrations for the curve pairs from top to bottom are 500 nM, 250 nM, 125 nM, 62.5 nM and 31.25 nM, respectively.
FIG. 8: Affinity constant assay of 14C12H1L1(hG1WT) to FcγRIIIa_V158. The antibody concentrations for the curve pairs from top to bottom are 500 nM, 250 nM, 125 nM, 62.5 nM and 31.25 nM, respectively.
FIG. 9: Affinity constant assay of 14C12H1L1(hG1TM) to FcγRIIIa_V158. The antibody concentrations for the curve pairs from top to bottom are 500 nM, 250 nM, 125 nM, 62.5 nM and 31.25 nM, respectively.
FIG. 10: Affinity constant assay of 5C10H2L2-IgG1mt to FcγRIIIa_V158. The antibody concentrations for the curve

pairs from top to bottom are 500 nM, 250 nM, 125 nM, 62.5 nM and 31.25 nM, respectively.

FIG. 11: Affinity constant assay of 14C12H1L1(hG1DM) to FcγRIIIa_F158. The antigen concentrations for the curve pairs from top to bottom are 500 nM, 250 nM, 125 nM, 62.5 nM and 31.25 nM, respectively.

FIG. 12: Affinity constant assay of 14C12H1L1(hG4) to FcγRIIIa_F158. The antibody concentrations for the curve pairs from top to bottom are 500 nM, 250 nM, 125 nM, 62.5 nM and 31.25 nM, respectively.

FIG. 13: Affinity constant assay of 14C12H1L1(hG1WT) to FcγRIIIa_F158. The antibody concentrations for the curve pairs from top to bottom are 500 nM, 250 nM, 125 nM, 62.5 nM and 31.25 nM, respectively.

FIG. 14: Affinity constant assay of 14C12H1L1(hG1TM) to FcγRIIIa_F158. The antibody concentrations for the curve pairs from top to bottom are 500 nM, 250 nM, 125 nM, 62.5 nM and 31.25 nM, respectively.

FIG. 15: Affinity constant assay of 5C10H2L2-IgG1mt to FcγRIIIa_F158. The antibody concentrations for the curve pairs from top to bottom are 500 nM, 250 nM, 125 nM, 62.5 nM and 31.25 nM, respectively.

FIG. 16: Affinity constant assay of 14C12H1L1(hG1DM) to FcγRIIa_H131. The antibody concentrations for the curve pairs from top to bottom are 200 nM, 100 nM, 50 nM, 25 nM and 12.5 nM, respectively.

FIG. 17: Affinity constant assay of 14C12H1L1(hG4) to FcγRIIa_H131. The antibody concentrations for the curve pairs from top to bottom are 200 nM, 100 nM, 50 nM, 25 nM and 12.5 nM, respectively.

FIG. 18: Affinity constant assay of 14C12H1L1(hG1WT) to FcγRIIa_H131. The antibody concentrations for the curve pairs from top to bottom are 200 nM, 100 nM, 50 nM, 25 nM and 12.5 nM, respectively.

FIG. 19: Affinity constant assay of 14C12H1L1(hG1TM) to FcγRIIa_H131. The antibody concentrations for the curve pairs from top to bottom are 200 nM, 100 nM, 50 nM, 25 nM and 12.5 nM, respectively.

FIG. 20: Affinity constant assay of 5C10H2L2-IgG1mt to FcγRIIa_H131. The antibody concentrations for the curve pairs from top to bottom are 200 nM, 100 nM, 50 nM, 25 nM and 12.5 nM, respectively.

FIG. 21: Affinity constant assay of 14C12H1L1(hG1DM) to FcγRIIa_R131. The antibody concentrations for the curve pairs from top to bottom are 200 nM, 100 nM, 50 nM, 25 nM and 12.5 nM, respectively.

FIG. 22: Affinity constant assay of 14C12H1L1(hG4) to FcγRIIa_R131. The antibody concentrations for the curve pairs from top to bottom are 200 nM, 100 nM, 50 nM, 25 nM and 12.5 nM, respectively.

FIG. 23: Affinity constant assay of 14C12H1L1(hG1WT) to FcγRIIa_R131. The antibody concentrations for the curve pairs from top to bottom are 200 nM, 100 nM, 50 nM, 25 nM and 12.5 nM, respectively.

FIG. 24: Affinity constant assay of 14C12H1L1(hG1TM) to FcγRIIa_R131. The antibody concentrations for the curve pairs from top to bottom are 200 nM, 100 nM, 50 nM, 25 nM and 12.5 nM, respectively.

FIG. 25: Affinity constant assay of 5C10H2L2-IgG1mt to FcγRIIa_R131. The antibody concentrations for the curve pairs from top to bottom are 200 nM, 100 nM, 50 nM, 25 nM and 12.5 nM, respectively.

FIG. 26: Affinity constant assay of 14C12H1L1(hG1DM) to FcγRIIb. The antibody concentrations for the curve pairs from top to bottom are 200 nM, 100 nM, 50 nM, 25 nM and 12.5 nM, respectively.

FIG. 27: Affinity constant assay of 14C12H1L1(hG4) to FcγRIIb. The antibody concentrations for the curve pairs from top to bottom are 200 nM, 100 nM, 50 nM, 25 nM and 12.5 nM, respectively.

FIG. 28: Affinity constant assay of 14C12H1L1(hG1WT) to FcγRIIb. The antibody concentrations for the curve pairs from top to bottom are 200 nM, 100 nM, 50 nM, 25 nM and 12.5 nM, respectively.

FIG. 29: Affinity constant assay of 14C12H1L1(hG1TM) to FcγRIIb. The antibody concentrations for the curve pairs from top to bottom are 200 nM, 100 nM, 50 nM, 25 nM and 12.5 nM, respectively.

FIG. 30: Affinity constant assay of 5C10H2L2-IgG1mt to FcγRIIb. The antibody concentrations for the curve pairs from top to bottom are 200 nM, 100 nM, 50 nM, 25 nM and 12.5 nM, respectively.

FIG. 31: Affinity constant assay of 14C12H1L1(hG1DM) to C1q. The antibody concentrations for the curve pairs from top to bottom are 20 nM, 10 nM, 5 nM, 2.5 nM and 1.25 nM, respectively.

FIG. 32: Affinity constant assay of 14C12H1L1(hG4) to C1q. The antibody concentrations for the curve pairs from top to bottom are 20 nM, 10 nM, 5 nM, 2.5 nM and 1.25 nM, respectively.

FIG. 33: Affinity constant assay of 14C12H1L1(hG1WT) to C1q. The antibody concentrations for the curve pairs from top to bottom are 20 nM, 10 nM, 5 nM, 2.5 nM and 1.25 nM, respectively.

FIG. 34: Affinity constant assay of 14C12H1L1(hG1TM) to C1q. The antibody concentrations for the curve pairs from top to bottom are 20 nM, 10 nM, 5 nM, 2.5 nM and 1.25 nM, respectively.

FIG. 35: Affinity constant assay of 5C10H2L2-IgG1mt to C1q. The antigen concentrations for the curve pairs from top to bottom are 20 nM, 10 nM, 5 nM, 2.5 nM and 1.25 nM, respectively.

FIG. 36: IFN-γ secretion assay by adding 14C12H1L1 (hG1WT) and 14C12H1L1(hG1TM) to mixed lymphocyte reaction.

FIG. 37: IL-2 secretion assay by adding 14C12H1L1 (hG1WT) and 14C12H1L1(hG1TM) to mixed lymphocyte reaction.

FIG. 38: ADCP effect assay of 14C12H1L1(hG1WT), nivolumab, and 14C12H1L1(hG1TM).

FIG. 39: Killing effect assay of 14C12H1L1(hG1TM) + anlotinib on human non-small cell lung cancer cells.

FIG. 40: Inhibited proliferation of mouse colorectal cancer MC38 cells by 14C12H1L1(hG1TM).

FIG. 41: Effectively enhanced immune response of immune cells to human gastric cancer KATO III cells by 14C12H1L1(hG1TM).

FIG. 42: Effectively enhanced immune response of immune cells to nasopharyngeal cancer CNE-2Z cells by 14C12H1L1(hG1TM).

FIG. 43: Effectively enhanced immune response of immune cells to mesothelioma NCI-H2452 cells by 14C12H1L1(hG1TM).

FIG. 44: Effectively enhanced immune response of immune cells to human non-small cell lung cancer NCI-H446 cells by 14C12H1L1(hG1TM).

FIG. 45: Effectively enhanced immune response of immune cells to nasopharyngeal cancer CNE-2Z cells by 14C12H1L1(hG1TM) in combination with anlotinib hydrochloride.

FIG. 46: Significantly enhanced immune response of immune cells to MSI-H/dMMR tumor SW48 cells by 14C12H1L1(hG1TM) in combination with anlotinib.

FIG. 47: Significantly enhanced immune response of immune cells to human colorectal cancer SW837 cells of non-MSI-H/dMMR (i.e., MSS) phenotype by 14C12H1L1(hG1TM).

FIG. 48: Significantly enhanced immune response of immune cells to human colorectal cancer SW837 cells of non-MSI-H/dMMR (i.e., MSS) phenotype by 14C12H1L1(hG1TM) in combination with anlotinib.

## DETAILED DESCRIPTION

The embodiments of the present invention will be described in detail below with reference to the examples. Those skilled in the art will understand that the following examples are only for illustrating the present invention, and should not be construed as limitation on the scope of the present invention. In the cases where the techniques or conditions are not specified, the examples were implemented according to the techniques or conditions described in the literature in the art (e.g., see, Molecular Cloning: A Laboratory Manual, authored by J. Sambrook et al., and translated by Huang Peitang et al., Third Edition, Science Press) or according to the product manual. Reagents or instruments used are commercially available conventional products if the manufacturers thereof are not specified.

In the following experiments of the present invention:

BALB/c mice were purchased from Guangdong Medical Laboratory Animal Center.

The anti-PDLI antibody 5C10H2L2-IgG1mt was prepared by methods described in PCT Publication No. WO2017148424A1.

The anti-PD-1 antibody nivolumab (trade name: Opdivo) was purchased from the Bristol-Myers Squibb.

Human peripheral blood mononuclear cells were isolated and prepared in Akeso Biopharma, Inc., with informed consent of the donor.

Raji-PDLI is a cell expressing human PD-L1 constructed by Akeso Biopharma on the basis of human B cells Raji via transfection.

Ficoll-Paque™ PLUS (or Ficoll-Paque PLUS) was purchased from GE Healthcare. Human IL-2 ELISA kit was purchased from Dakewe Biotech Co., Ltd.

RPMI 1640 medium, DMEM medium, Trypsin-EDTA (0.25%) phenol red and Blastidin were all purchased from Gibco.

*Staphylococcus aureus* enterotoxin B (SEB) was purchased from Dianotech.

FBS was purchased from Excell bio.

Mitomycin C (MMC) was purchased from Stressmarq.

The sequence of the isotype control, human anti-hen egg lysozyme IgG (anti-HEL antibody, or human IgG, abbreviated as hIgG) is derived from the variable region sequence of the Fab F10.6.6 sequence in the study reported by Acierno et al., entitled "Affinity maturation increases the stability and plasticity of the Fv domain of anti-protein antibodies" (Acierno et al., J Mol Biol., 2007; 374(1):130-146).

Anlotinib used in the examples is hydrochloride salt of anlotinib under the brand name Fukewei® and generic name anlotinib hydrochloride, and was purchased from CTTQ Pharma.

### Preparation Example 1: Sequence Design of Anti-PD-1 Antibody 14C12 and Its Humanized Antibody 14C12H1L1(hG1WT)

The amino acid sequences and encoding nucleotide sequences of the heavy and light chains of anti-PD-1 antibody 14C12 and its humanized antibody 14C12H1L1(hG1WT) are identical to those of 14C12 and 14C12H1L1 in Chinese Patent Publication No. CN106967172A (or No. CN106977602A), respectively.

(1) Heavy and light chain variable region sequences of 14C12

Nucleotide sequence of the heavy chain variable region of 14C12: (354 bp)

**GAGGTCAAACTGGTGGAGAGCGGCGGCGGGCTGGTGAAGCCCGGCGGGTCACTGAAACTGAGCTGCGCCGCTTCCGGCTTCGCCTTTAGCTCCTACGACATGTCATGGGTGAGGCAGACCCCTGAGAAGCGCCTGGAATGGGTCGCTACTATCAGCGGAGGCGGGCGATACACCTACTATCCTGACTCTGTCAAAGGGAGATTCACAATTAGTCGGGATAACGCCAGAAATACTCTGTATCTGCAGATGTCTAGTCTGCGGTCCGAGGATACAGCTCTGTACTATTGTGCAAACCGGTACGGCGAAGCATGGTTTGCCTATTGGGGACAGGGCACCCTGGTGACAGTCTCTGCC (SEQ ID NO: 1)**

Amino acid sequence of the heavy chain variable region of 14C12: (118 aa)

**EVKLVESGGGLVKPGGSLKLSCAASGFAFSSYDMSWVRQTPEKRLEWVATISGGGRYTYYPDSVKGRFTISRDNARNTLYLQMSSLRSEDTALYYCANRYGEAWFAYWGQGTLVTVSA (SEQ ID NO: 2)**

Nucleotide sequence encoding the light chain variable region of 14C12: (321 bp)

**GACATTAAGATGACACAGTCCCCTTCCTCAATGTACGCTAGCCTGGGCGAGCGAGTGACCTTCACATGCAAAGCATCCCAGGACATCAACACATACCTGTCTTGGTTTCAGCAGAAGCCAGGCAAAAGCCCCAAGACCCTGATCTACCGGGCCAATAGACTGGTGGACGGGGTCCCCAGCAGATTCTCCGGATCTGGCAGTGGGCAGGATTACTCCCTGACCATCAGCTCCCTGGAGTATGAAGACATGGGCATCTACTATTGCCTGCAGTATGATGAGTTCCCTCTGACCTTTGGAGCAGGCACAAAACTGGAACTGAAG (SEQ ID NO: 3)**

Amino acid sequence of the light chain variable region of 14C12: (107 aa)

**DIKMTQSPSSMYASLGERVTFTCKASQDINTYLSWFQQKPGKSPKTLIYRANRLVDGVPSRFSGSGSGQDYSLTISSLEYEDMGIYYCLQYDEFPLTFGAGTKLELK (SEQ ID NO: 4)**

(2) Heavy and light chain variable region and heavy and light chain sequences of humanized monoclonal antibody 14C12H1L1(hG1WT)

Nucleotide sequence of the heavy chain variable region of 14C12H1L1(hG1WT): (354 bp)

**GAAGTGCAGCTGGTCGAGTCTGGGGGAGGGCTGGTGCAGCCCGGCGGGTCACTGCGACTGAGCTGCGCAGCTTCCGGATTCGCCTTTAGCTCCTACGACATGTCCTGGGTGCGACAGGCACCAGGAAAGGGACTGGATTGGGTCGCTACTATCTCAGGAGGCGGGAGATACACCTACTATCCTGACAGCGTCAAGGGCCGGTTCACAATCTCTAGAGATAACAGTAAGAACAATCTGTATCTGCAGATGAACAGCCTGAGGGCTGAGGACACCGCACTGTACTATTGTGCCAACCGCTACGGGGAAGCATGGTTTGCCTATTGGGGGCAGGGAACCCTGGTGACAGTCTCTAGT (SEQ ID NO: 5)**

Amino acid sequence of the heavy chain variable region of 14C12H1L1(hG1WT): (118 aa)

**EVQLVESGGGLVQPGGSLRLSCAASGFAFSSYDMSWVRQAPGKGLDWVATISGGGRYTYYPDSVKGRFTISRDNSKNNLYLQMNSLRAEDTALYYCANRYGEAWFAYWGQGTLVTVSS (SEQ ID NO: 6)**

Nucleotide sequence encoding the light chain variable region of 14C12H1L1(hG1WT): (321 bp)

**GACATTCAGATGACTCAGAGCCCCTCCTCCATGTCCGCCTCTGTGGGCGACAGGGTCACCTTCACATGCCGCGCTAGTCAGGATATCAACACCTACCTGAGCTGGTTTCAGCAGAAGCCAGGGAAAAGCCCCAAGACACTGATCTACCGGGCTAATAGACTGGTGTCTGGAGTCCCAAGTCGGTTCAGTGGCTCAGGGAGCGGACAGGACTACACTCTGACCATCAGCTCCCTGCAGCCTGAGGACATGGCAACCTACTATTGCCTGCAGTATGATGAGTTCCCACTGACCTTTGGCGCCGGGACAAAACTGGAGCTGAAG (SEQ ID NO: 7)**

Amino acid sequence of the light chain variable region of 14C12H1L1(hG1WT): (107 aa)

**DIQMTQSPSSMSASVGDRVTFTCRASQDINTYLSWFQQKPGKSPKTLIYRANRLVSGVPSRFSGSGSGQDYTLTISSLQPEDMATYYCLQYDEFPLTFGAGTKLELK (SEQ ID NO: 8)**

Nucleotide sequence of the heavy chain of 14C12H1L1(hG1WT): (1344 bp)

**GAAGTGCAGCTGGTCGAGTCTGGGGGAGGGCTGGTGCAGCCCGGCGGGTCAC TGCGACTGAGCTGCGCAGCTTCCGGATTCGCCTTTAGCTCCTACGACATGTCCT GGGTGCGACAGGCACCAGGAAAGGGACTGGATTGGGTCGCTACTATCTCAGGA GGCGGGAGATACACCTACTATCCTGACAGCGTCAAGGGCCGGTTCACAATCTC TAGAGATAACAGTAAGAACAATCTGTATCTGCAGATGAACAGCCTGAGGGCTG AGGACACCGCACTGTACTATTGTGCCAACCGCTACGGGGAAGCATGGTTTGCC TATTGGGGGCAGGGAACCCTGGTGACAGTCTCTAGTGCCAGCACCAAAGGACC TAGCGTGTTTCCTCTCGCCCCCTCCTCCAAAAGCACCAGCGGAGGAACCGCTG CTCTCGGATGTCTGGTGAAGGACTACTTCCCTGAACCCGTCACCGTGAGCTGG AATAGCGGCGCTCTGACAAGCGGAGTCCATACATTCCCTGCTGTGCTGCAAAG CAGCGGACTCTATTCCCTGTCCAGCGTCGTCACAGTGCCCAGCAGCAGCCTGG GCACCCAGACCTACATCTGTAACGTCAACCACAAGCCCTCCAACACCAAGGTG GACAAGAAAGTGGAGCCCAAATCCTGCGACAAGACACACACCTGTCCCCCCTG TCCTGCTCCCGAACTCCTCGGAGGCCCTAGCGTCTTCCTCTTTCCTCCCAAACC CAAGGACACCCTCATGATCAGCAGAACCCCTGAAGTCACCTGTGTCGTCGTGG ATGTCAGCCATGAGGACCCCGAGGTGAAATTCAACTGGTATGTCGATGGCGTC GAGGTGCACAACGCCAAAACCAAGCCCAGGGAGGAACAGTACAACTCCACCTA CAGGGTGGTGTCCGTGCTGACAGTCCTCCACCAGGACTGGCTGAACGGCAAGG AGTACAAGTGCAAGGTGTCCAACAAGGCTCTCCCTGCCCCCATTGAGAAGACC ATCAGCAAGGCCAAAGGCCAACCCAGGGAGCCCCAGGTCTATACACTGCCTCC CTCCAGGGACGAACTCACCAAGAACCAGGTGTCCCTGACCTGCCTGGTCAAGG GCTTTTATCCCAGCGACATCGCCGTCGAGTGGGAGTCCAACGGACAGCCCGAG AATAACTACAAGACCACCCCTCCTGTCCTCGACTCCGACGGCTCCTTCTTCCTG TACAGCAAGCTGACCGTGGACAAAAGCAGGTGGCAGCAGGGAAACGTGTTCTC CTGCAGCGTGATGCACGAAGCCCTCCACAACCACTACACCCAGAAAAGCCTGT CCCTGAGCCCCGGCAAA (SEQ ID NO: 9)**

**Amino acid sequence of the heavy chain of 14C12H1L1(hG1WT): (448 aa)**

**EVQLVESGGGLVQPGGSLRLSCAASGFAFSSYDMSWVRQAPGKGLDWVATISGGG RYTYYPDSVKGRFTISRDNSKNNLYLQMNSLRAEDTALYYCANRYGEAWFAYWG QGTLVTVSSASTKGPSVFPLAPSSKSTSGGTAALGCLVKDYFPEPVTVSWNSGALTS GVHTFPAVLQSSGLYSLSSVVTVPSSSLGTQTYICNVNHKPSNTKVDKKVEPKSCDK THTCPPCPAPELLGGPSVFLFPPKPKDTLMISRTPEVTCVVVDVSHEDPEVKFNWY VDGVEVHNAKTKPREEQYNSTYRVVSVLTVLHQDWLNGKEYKCKVSNKALPAPIE KTISKAKGQPREPQVYTLPPSRDELTKNQVSLTCLVKGFYPSDIAVEWESNGQPEN NYKTTPPVLDSDGSFFLYSKLTVDKSRWQQGNVFSCSVMHEALHNHYTQKSLSLSP GK (SEQ ID NO: 10)**

Nucleotide sequence of the light chain of 14C12H1L1(hG1WT): (642 bp)

**GACATTCAGATGACTCAGAGCCCCTCCTCCATGTCCGCCTCTGTGGGCGACAG GGTCACCTTCACATGCCGCGCTAGTCAGGATATCAACACCTACCTGAGCTGGTT TCAGCAGAAGCCAGGGAAAAGCCCCAAGACACTGATCTACCGGGCTAATAGAC TGGTGTCTGGAGTCCCAAGTCGGTTCAGTGGCTCAGGGAGCGGACAGGACTAC ACTCTGACCATCAGCTCCCTGCAGCCTGAGGACATGGCAACCTACTATTGCCTG CAGTATGATGAGTTCCCACTGACCTTTGGCGCCGGGACAAAACTGGAGCTGAA GCGAACTGTGGCCGCTCCCTCCGTCTTCATTTTTCCCCCTTCTGACGAACAGCT GAAATCAGGCACAGCCAGCGTGGTCTGTCTGCTGAACAATTTCTACCCTAGAG AGGCAAAAGTGCAGTGGAAGGTCGATAACGCCCTGCAGTCCGGCAACAGCCAG GAGAGTGTGACTGAACAGGACTCAAAAGATAGCACCTATTCCCTGTCTAGTAC ACTGACTCTGTCCAAGGCTGATTACGAGAAGCACAAAGTGTATGCATGCGAAG TGACACATCAGGGACTGTCAAGCCCCGTGACTAAGTCTTTTAACCGGGGCGAA TGT (SEQ ID NO: 11)**

Amino acid sequence of the light chain of 14C12H1L1(hG1WT): (214 aa)

**DIQMTQSPSSMSASVGDRVTFTCRASQDINTYLSWFQQKPGKSPKTLIYRANRLVS GVPSRFSGSGSGQDYTLTISSLQPEDMATYYCLQYDEFPLTFGAGTKLELKRTVAA PSVFIFPPSDEQLKSGTASVVCLLNNFYPREAKVQWKVDNALQSGNSQESVTEQDS KDSTYSLSSTLTLSKADYEKHKVYACEVTHQGLSSPVTKSFNRGEC (SEQ ID NO:12)**

Preparation Example 2: Sequence Design of Humanized Antibody 14C12H1L1(hGT4)

The heavy and light chain variable regions are identical to those of 14C12H1L1(hG1WT). Ig gamma-4 chain C region (ACCESSION: P01861.1) was used as the heavy chain constant region, and Ig kappa chain C region (ACCESSION: P01834) was used as the light chain constant region, thus giving the antibody 14C12H1L1(hG4). The sequence of 14C12H1L1(hG4) is as follows:

Nucleotide sequence of the heavy chain of 14C12H1L1(hG4): (1335 bp)

**GAAGTGCAGCTGGTCGAGTCTGGGGGAGGGCTGGTGCAGCCCGGCGGGTCAC
TGCGACTGAGCTGCGCAGCTTCCGGATTCGCCTTTAGCTCCTACGACATGTCCT
GGGTGCGACAGGCACCAGGAAAGGGACTGGATTGGGTCGCTACTATCTCAGGA
GGCGGGAGATACACCTACTATCCTGACAGCGTCAAGGGCCGGTTCACAATCTC
TAGAGATAACAGTAAGAACAATCTGTATCTGCAGATGAACAGCCTGAGGGCTG
AGGACACCGCACTGTACTATTGTGCCAACCGCTACGGGGAAGCATGGTTTGCC
TATTGGGGGCAGGGAACCCTGGTGACAGTCTCTAGTGCCAGCACCAAAGGGCC
CTCGGTCTTCCCCCTGGCGCCCTGCTCCAGGAGCACCTCCGAGAGCACAGCCG
CCCTGGGCTGCCTGGTCAAGGACTACTTCCCCGAACCGGTGACGGTGTCGTGG
AACTCAGGCGCCCTGACCAGCGGCGTGCACACCTTCCCGGCTGTCCTACAGTC
CTCAGGACTCTACTCCCTCAGCAGCGTGGTGACCGTGCCCTCCAGCAGCTTGG
GCACGAAGACCTACACCTGCAACGTAGATCACAAGCCCAGCAACACCAAGGTG
GACAAGAGAGTTGAGTCCAAATATGGTCCCCCATGCCCACCATGCCCAGCACC
TGAGTTCCTGGGGGGACCATCAGTCTTCCTGTTCCCCCCAAAACCCAAGGACA
CTCTCATGATCTCCCGGACCCCTGAGGTCACGTGCGTGGTGGTGGACGTGAGC
CAGGAAGACCCCGAGGTCCAGTTCAACTGGTACGTGGATGGCGTGGAGGTGCA
TAATGCCAAGACAAAGCCGCGGGAGGAGCAGTTCAACAGCACGTACCGTGTGG
TCAGCGTCCTCACCGTCCTGCACCAGGACTGGCTGAACGGCAAGGAGTACAAG
TGCAAGGTCTCCAACAAAGGCCTCCCGTCCTCCATCGAGAAAACCATCTCCAAA
GCCAAAGGGCAGCCCCGAGAGCCACAGGTGTACACCCTGCCCCCATCCCAGGA
GGAGATGACCAAGAACCAGGTCAGCCTGACCTGCCTGGTCAAAGGCTTCTACC
CCAGCGACATCGCCGTGGAGTGGGAGAGCAATGGGCAGCCGGAGAACAACTA
CAAGACCACGCCTCCCGTGCTGGACTCCGACGGCTCCTTCTTCCTCTACAGCA
GGCTAACCGTGGACAAGAGCAGGTGGCAGGAGGGGAATGTCTTCTCATGCTCC
GTGATGCATGAGGCTCTGCACAACCACTACACACAGAAGAGCCTCTCCCTGTC
TCTGGGTAAA (SEQ ID NO: 13)**

Amino acid sequence of the heavy chain of 14C12H1L1(hG4): (445 aa)

**EVQLVESGGGLVQPGGSLRLSCAASGFAFSSYDMSWVRQAPGKGLDWVATISGGG**
**RYTYYPDSVKGRFTISRDNSKNNLYLQMNSLRAEDTALYYCANRYGEAWFAYWG**
**QGTLVTVSSASTKGPSVFPLAPCSRSTSESTAALGCLVKDYFPEPVTVSWNSGALTS**
**GVHTFPAVLQSSGLYSLSSVVTVPSSSLGTKTYTCNVDHKPSNTKVDKRVESKYGPP**
**CPPCPAPEFLGGPSVFLFPPKPKDTLMISRTPEVTCVVVDVSQEDPEVQFNWYVDG**
**VEVHNAKTKPREEQFNSTYRVVSVLTVLHQDWLNGKEYKCKVSNKGLPSSIEKTIS**
**KAKGQPREPQVYTLPPSQEEMTKNQVSLTCLVKGFYPSDIAVEWESNGQPENNYK**
**TTPPVLDSDGSFFLYSRLTVDKSRWQEGNVFSCSVMHEALHNHYTQKSLSLSLGK**
**(SEQ ID NO: 14)**

The nucleotide sequence of the 14C12H1L1(hG4) light chain is identical to SEQ ID NO: 11.
The amino acid sequence of the 14C12H1L1(hG4) light chain is identical to SEQ ID NO: 12.

Preparation Example 3: Sequence Design of Humanized Antibody 14C12H1L1(hG1TM)

On the basis of 14C12H1L1(hG1WT) obtained in Preparation Example 1, a humanized mutant 14C12H1L1(hG1TM) was obtained by introducing a leucine-to-alanine point mutation at position 234 (L234A), a leucine-to-alanine point mutation at position 235 (L235A), and a glycine-to-alanine point mutation at position 237 (G237A) in the hinge region of the heavy chain according to the EU numbering system.

Nucleotide sequence of the heavy chain of 14C12H1L1(hG1TM): (1344 bp)

**GAAGTGCAGCTGGTCGAGTCTGGGGGAGGGCTGGTGCAGCCCGGCGGGTCAC**
**TGCGACTGAGCTGCGCAGCTTCCGGATTCGCCTTTAGCTCCTACGACATGTCCT**
**GGGTGCGACAGGCACCAGGAAAGGGACTGGATTGGGTCGCTACTATCTCAGGA**
**GGCGGGAGATACACCTACTATCCTGACAGCGTCAAGGGCCGGTTCACAATCTC**
**TAGAGATAACAGTAAGAACAATCTGTATCTGCAGATGAACAGCCTGAGGGCTG**
**AGGACACCGCACTGTACTATTGTGCCAACCGCTACGGGGAAGCATGGTTTGCC**
**TATTGGGGGCAGGGAACCCTGGTGACAGTCTCTAGTGCCAGCACCAAAGGGCC**

**CAGCGTGTTTCCTCTCGCCCCCTCCTCCAAAAGCACCAGCGGAGGAACCGCTG CTCTCGGATGTCTGGTGAAGGACTACTTCCCTGAACCCGTCACCGTGAGCTGG AATAGCGGCGCTCTGACAAGCGGAGTCCATACATTCCCTGCTGTGCTGCAAAG CAGCGGACTCTATTCCCTGTCCAGCGTCGTCACAGTGCCCAGCAGCAGCCTGG GCACCCAGACCTACATCTGTAACGTCAACCACAAGCCCTCCAACACCAAGGTG GACAAGAAAGTGGAGCCCAAATCCTGCGACAAGACACACACCTGTCCCCCCTG TCCTGCTCCCGAAGCTGCTGGAGCCCCTAGCGTCTTCCTCTTTCCTCCCAAACC CAAGGACACCCTCATGATCAGCAGAACCCCTGAAGTCACCTGTGTCGTCGTGG ATGTCAGCCATGAGGACCCCGAGGTGAAATTCAACTGGTATGTCGATGGCGTC GAGGTGCACAACGCCAAAACCAAGCCCAGGGAGGAACAGTACAACTCCACCTA CAGGGTGGTGTCCGTGCTGACAGTCCTCCACCAGGACTGGCTGAACGGCAAGG AGTACAAGTGCAAGGTGTCCAACAAGGCTCTCCCTGCCCCCATTGAGAAGACC ATCAGCAAGGCCAAAGGCCAACCCAGGGAGCCCCAGGTCTATACACTGCCTCC CTCCAGGGACGAACTCACCAAGAACCAGGTGTCCCTGACCTGCCTGGTCAAGG GCTTTTATCCCAGCGACATCGCCGTCGAGTGGGAGTCCAACGGACAGCCCGAG AATAACTACAAGACCACCCCTCCTGTCCTCGACTCCGACGGCTCCTTCTTCCTG TACAGCAAGCTGACCGTGGACAAAAGCAGGTGGCAGCAGGGAAACGTGTTCTC CTGCAGCGTGATGCACGAAGCCCTCCACAACCACTACACCCAGAAAAGCCTGT CCCTGAGCCCCGGCAAA (SEQ ID NO: 15)**

Amino acid sequence of the heavy chain of 14C12H1L1(hG1TM): (448 aa)

**EVQLVESGGGLVQPGGSLRLSCAASGFAFSSYDMSWVRQAPGKGLDWVATISGGG RYTYYPDSVKGRFTISRDNSKNNLYLQMNSLRAEDTALYYCANRYGEAWFAYWG QGTLVTVSSASTKGPSVFPLAPSSKSTSGGTAALGCLVKDYFPEPVTVSWNSGALTS GVHTFPAVLQSSGLYSLSSVVTVPSSSLGTQTYICNVNHKPSNTKVDKKVEPKSCDK THTCPPCPAPEAAGAPSVFLFPPKPKDTLMISRTPEVTCVVVDVSHEDPEVKFNWY VDGVEVHNAKTKPREEQYNSTYRVVSVLTVLHQDWLNGKEYKCKVSNKALPAPIE KTISKAKGQPREPQVYTLPPSRDELTKNQVSLTCLVKGFYPSDIAVEWESNGQPEN NYKTTPPVLDSDGSFFLYSKLTVDKSRWQQGNVFSCSVMHEALHNHYTQKSLSLSP GK (SEQ ID NO: 16)**

The nucleotide sequence of the 14C12H1L1(hG1TM) light chain is identical to SEQ ID NO: 11.
The amino acid sequence of the 14C12H1L1(hG1TM) light chain is identical to SEQ ID NO: 12.

Preparation Example 4: Sequence Design of Humanized Antibody 14C12H1L1(hG1DM)

On the basis of 14C12H1L1(hG1WT), a humanized mutant antibody 14C12H1L1(hG1DM) was obtained by introducing a leucine-to-alanine point mutation at position 234 (L234A) and a leucine-to-alanine point mutation at position 235 (L235A) in the hinge region of the heavy chain.

Nucleotide sequence of the heavy chain of 14C12H1L1(hG1DM): (1344 bp)

**GAAGTGCAGCTGGTCGAGTCTGGGGGAGGGCTGGTGCAGCCCGGCGGGTCAC TGCGACTGAGCTGCGCAGCTTCCGGATTCGCCTTTAGCTCCTACGACATGTCCT GGGTGCGACAGGCACCAGGAAAGGGACTGGATTGGGTCGCTACTATCTCAGGA GGCGGGAGATACACCTACTATCCTGACAGCGTCAAGGGCCGGTTCACAATCTC TAGAGATAACAGTAAGAACAATCTGTATCTGCAGATGAACAGCCTGAGGGCTG AGGACACCGCACTGTACTATTGTGCCAACCGCTACGGGGAAGCATGGTTTGCC TATTGGGGGCAGGGAACCCTGGTGACAGTCTCTAGTGCTAGCACCAAAGGGCC CAGCGTGTTTCCTCTCGCCCCCTCCTCCAAAAGCACCAGCGGAGGAACCGCTG CTCTCGGATGTCTGGTGAAGGACTACTTCCCTGAACCCGTCACCGTGAGCTGG AATAGCGGCGCTCTGACAAGCGGAGTCCATACATTCCCTGCTGTGCTGCAAAG CAGCGGACTCTATTCCCTGTCCAGCGTCGTCACAGTGCCCAGCAGCAGCCTGG GCACCCAGACCTACATCTGTAACGTCAACCACAAGCCCTCCAACACCAAGGTG GACAAGAAAGTGGAGCCCAAATCCTGCGACAAGACACACACCTGTCCCCCCTG TCCTGCTCCCGAAGCTGCTGGAGGCCCTAGCGTCTTCCTCTTTCCTCCCAAACC CAAGGACACCCTCATGATCAGCAGAACCCCTGAAGTCACCTGTGTCGTCGTGG ATGTCAGCCATGAGGACCCCGAGGTGAAATTCAACTGGTATGTCGATGGCGTC GAGGTGCACAACGCCAAAACCAAGCCCAGGGAGGAACAGTACAACTCCACCTA CAGGGTGGTGTCCGTGCTGACAGTCCTCCACCAGGACTGGCTGAACGGCAAGG AGTACAAGTGCAAGGTGTCCAACAAGGCTCTCCCTGCCCCCATTGAGAAGACC ATCAGCAAGGCCAAAGGCCAACCCAGGGAGCCCCAGGTCTATACACTGCCTCC CTCCAGGGACGAACTCACCAAGAACCAGGTGTCCCTGACCTGCCTGGTCAAGG GCTTTTATCCCAGCGACATCGCCGTCGAGTGGGAGTCCAACGGACAGCCCGAG AATAACTACAAGACCACCCCTCCTGTCCTCGACTCCGACGGCTCCTTCTTCCTG TACAGCAAGCTGACCGTGGACAAAAGCAGGTGGCAGCAGGGAAACGTGTTCTC CTGCAGCGTGATGCACGAAGCCCTCCACAACCACTACACCCAGAAAAGCCTGT CCCTGAGCCCCGGCAAA (SEQ ID NO: 17)**

Amino acid sequence of the heavy chain of 14C12H1L1(hG1DM): (448 aa)

**EVQLVESGGGLVQPGGSLRLSCAASGFAFSSYDMSWVRQAPGKGLDWVATISGGG RYTYYPDSVKGRFTISRDNSKNNLYLQMNSLRAEDTALYYCANRYGEAWFAYWG QGTLVTVSSASTKGPSVFPLAPSSKSTSGGTAALGCLVKDYFPEPVTVSWNSGALTS GVHTFPAVLQSSGLYSLSSVVTVPSSSLGTQTYICNVNHKPSNTKVDKKVEPKSCDK THTCPPCPAPEAAGGPSVFLFPPKPKDTLMISRTPEVTCVVVDVSHEDPEVKFNWY VDGVEVHNAKTKPREEQYNSTYRVVSVLTVLHQDWLNGKEYKCKVSNKALPAPIE KTISKAKGQPREPQVYTLPPSRDELTKNQVSLTCLVKGFYPSDIAVEWESNGQPEN NYKTTPPVLDSDGSFFLYSKLTVDKSRWQQGNVFSCSVMHEALHNHYTQKSLSLSP GK (SEQ ID NO: 18)**

The nucleotide sequence of the 14C12H1L1(hG1DM) light chain is identical to SEQ ID NO: 11.
The amino acid sequence of the 14C12H1L1(hG1DM) light chain is identical to SEQ ID NO: 12.

<u>Experimental Example 1: Affinity Assay of 14C12H1L1(hG1DM), 14C12H1L1(hG4), 14C12H1L1(hG1WT) and 14C12H1L1(hG1TM) to Fc Receptor FcγRI</u>

The Fc receptor FcγRI, also known as CD64, can bind to the Fc fragment of IgG antibodies and is involved in antibody-dependent cell-mediated cytotoxicity (ADCC). The binding capacity of a therapeutic monoclonal antibody to Fc receptors will influence the safety and efficacy of the antibody. The affinity constants of 14C12H1L1(hG1DM), 14C12H1L1(hG4), 14C12H1L1(hG1WT) and 14C12H1L1(hG1TM) to FcγRI were measured in this experiment using a Fortebio Octet system to evaluate the ADCC activity of the antibodies.

The method for determining the affinity constant of the antibodies to FcγRI by the Fortebio Octet system is briefly described as follows: the sample dilution buffer was a solution of 0.02% Tween-20 and 0.1% BSA in PBS, pH 7.4. A 1 μg/mL FcγRI solution (from Sinobio) was added to the HIS1K sensor to immobilize the FcγRI on the sensor surface for 50 s. The association and dissociation constants of the antibodies to FcγRI were both determined in the buffer with the antibody concentrations being 3.12-50 nM (serial two-fold dilution). The sensor with immobilized antigen was equilibrated in the buffer for 60 s, and then the binding of the immobilized FcγRI on the sensor to the antibodies was determined for 120 s; the dissociation of FcγRI from the antibodies was determined in 120 s. The temperature was 30 °C and the frequency was 0.3 Hz. The data were fitted and analyzed with a 1:1 model to obtain the affinity constants to FcγRI for the antibodies.

The results of affinity constant assay of 14C12H1L1(hG1DM), 14C12H1L1(hG4), 14C12H1L1(hG1WT) and 14C12H1L1(hG1TM) and the control antibody 5C10H2L2-IgG1mt to FcγRI are shown in Table 1 and FIGs. 1-5.

Table 1: Kinetics for binding of 14C12H1L1(hG1DM), 14C12H1L1(hG4), 14C12H1L1(hG1WT) and 14C12H1L1 (hG1TM) and the control antibody 5C10H2L2-IgG1mt to FcγRI

| Antibody | $K_D$ (M) | kon (1/Ms) | SE (kon) | kdis (1/s) | SE (kdis) | Rmax (nm) |
|---|---|---|---|---|---|---|
| 14C12H1L1 (hG1DM) | N/A | N/A | N/A | N/A | N/A | N/A |
| 14C12H1L1 (hG4) | 5.80E-09 | 6.37E+05 | 2.21E+04 | 3.69E-03 | 1.05E-04 | 0.45-0.54 |
| 14C12H1L1 (hG1WT) | 2.52E-09 | 6.94E+05 | 2.19E+04 | 1.75E-03 | 9.14E-05 | 0.50-0.55 |
| 14C12H1L1 (hG1TM) | N/A | N/A | N/A | N/A | N/A | N/A |
| 5C10H2L2-IgG1mt | N/A | N/A | N/A | N/A | N/A | N/A |

N/A indicates that the antibody had no binding or an extremely weak binding signal to the antigen, and thus the results were not analyzed and no corresponding data was obtained. The results showed that both 14C12H1L1(hG4) and 14C12H1L1(hG1WT) bound to FcγRI with affinity constants of 5.80E-09 M and 2.52E-09 M, respectively; 14C12H1L1(hG1TM) and 5C10H2L2-IgG1mt had no binding or an extremely weak binding signal to FcγRI, and thus the results were not analyzed and no corresponding data was obtained.

The results suggested that the binding activities of 14C12H1L1(hG1DM) and 14C12H1L1(hG1TM) to FcγRI are effectively eliminated as compared to 14C12H1L1(hG4) and 14C12H1L1(hG1WT).

Experimental Example 2: Affinity Assay of 14C12H1L1(hG1DM), 14C12H1L1(hG4), 14C12H1L1(hG1WT) and 14C12H1L1(hG1TM) to Fc Recentor FcγRIIIa and subtypes thereof

(1) Affinity constant assay of 14C12H1L1(hG1DM), 14C12H1L1(hG4), 14C12H1L1(hG1WT) and 14C12H1L1(hG1TM) to FcγRIIIa V158

The Fc receptor FcγRIIIa_V158 (also known as CD16a_V158), can bind to the Fc fragment of IgG antibodies and mediate ADCC effects. The affinity constants of 14C12H1L1(hG1DM), 14C12H1L1(hG4), 14C12H1L1(hG1WT) and 14C12H1L1(hG1TM) to FcγRIIIa_V158 were measured in this experiment using a Fortebio Octet system to evaluate the ADCC activity of the antibodies.

The method for determining the affinity constant of the antibodies and the control antibody 5C10H2L2-IgG1mt to FcγRIIIa_V158 by the Fortebio Octet system is briefly described as follows: the sample dilution buffer was a solution of 0.02% Tween-20 and 0.1% BSA in PBS, pH 7.4. 5 μg/mL FcγRIIIa_V158 was immobilized on the HIS1K sensor for 120 s. The sensor was equilibrated in a buffer for 60 s, and the binding of the immobilized FcγRIIIa_V158 on the sensor to the antibodies at concentrations of 31.25-500 nM (serial two-fold dilution) was determined for 60 s. The antibody was dissociated in the buffer for 60 s. The sensor was refreshed 4 times in 10 mM glycine pH 1.5, each for 5 s. The temperature was 30 °C and the frequency was 0.3 Hz. The data were analyzed by 1:1 model fitting to obtain affinity constants.

The results of affinity constant assay of 14C12H1L1(hG1DM), 14C12H1L1(hG4), 14C12H1L1(hG1WT) and 14C12H1L1(hG1TM) and the control antibody 5C10H2L2-IgG1mt to FcγRIIIa_V158 are shown in Table 2 and FIGs. 6-10.

Table 2: Kinetics for binding of 14C12H1L1(hG1DM), 14C12H1L1(hG4), 14C12H1L1(hG1WT) and 14C12H1L1 (hG1TM) and the control antibody 5C10H2L2-IgG1mt to FcγRIIIa_V158

| Antibody | $K_D$ (M) | kon (1/Ms) | SE (kon) | kdis (1/s) | SE (kdis) | Rmax (nm) |
|---|---|---|---|---|---|---|
| **14C12H1L1 (hG1DM)** | **6.12E-07** | **1.35E+05** | **3.74E+04** | **8.24E-02** | **8.34E-03** | **0.33-0.41** |
| **14C12H1L1 (hG4)** | **N/A** | **N/A** | **N/A** | **N/A** | **N/A** | **N/A** |
| **14C12H1L1 (hG1WT)** | **6.54E-08** | **2.61E+05** | **2.10E+04** | **1.71E-02** | **6.47E-04** | **0.80-1.17** |
| **14C12H1L1 (hG1TM)** | **N/A** | **N/A** | **N/A** | **N/A** | **N/A** | **N/A** |
| **5C10H2L2-IgG1mt** | **N/A** | **N/A** | **N/A** | **N/A** | **N/A** | **N/A** |

N/A indicates that the antibody had no binding or an extremely weak binding signal to the antigen, and thus the results were not analyzed and no corresponding data was obtained. The results showed that both 14C12H1L1(hG1DM) and 14C12H1L1(hG1WT) bound to FcγRIIIa_V158 with affinity constants of 6.21E-07M and 6.54E-08M, respectively; 14C12H1L1(hG4), 14C12H1L1(hG1TM) and 5C10H2L2-IgG1mt had no binding or an extremely weak binding signal to FcγRIIIa_V158, and thus the results were not analyzed. The results suggested that the binding activities of 14C12H1L1(hG4), 14C12H1L1(hG1TM) and the control antibody 5C10H2L2-IgG1mt to FcγR IIIa_V158 are effectively eliminated as compared to 14C12H1L1(hG1DM) and 14C12H1L1(hG1WT).

(2) Affinity constant assay of 14C12H1L1(hG1DM), 14C12H1L1(hG4), 14C12H1L1(hG1WT) and 14C12H1L1(hG1TM) to FcγRIIIa F158

The Fc receptor FcγRIIIa_F158 (also known as CD16a_F158), can bind to the Fc fragment of IgG antibodies

and mediate ADCC effects. The affinity constants of 14C12H1L1(hG1DM), 14C12H1L1(hG4), 14C12H1L1(hG1WT), 14C12H1L1(hG1TM) and the control antibody to FcγRIIIa_F158 were measured in this experiment using a Fortebio Octet system to evaluate the ADCC activity of the antibodies.

The method for determining the affinity constant of 14C12H1L1(hG1DM), 14C12H1L1(hG4), 14C12H1L1(hG1WT) and 14C12H1L1(hG1TM) to FcγRIIIa_F158 by the Fortebio Octet system is briefly described as follows: the sample dilution buffer was a solution of 0.02% Tween-20 and 0.1% BSA in PBS, pH 7.4. 5 μg/mL FcyRIIIa_F158 was immobilized on the HIS1K sensor for 120 s. The sensor was equilibrated in a buffer for 60 s, and the binding of the immobilized FcyRIIIa_F158 on the sensor to the antibodies at concentrations of 31.25-500 nM (serial two-fold dilution) was determined for 60 s. The antibody was dissociated in the buffer for 60 s. The sensor was refreshed 4 times in 10 mM glycine pH 1.5, each for 5 s. The temperature was 30 °C and the frequency was 0.3 Hz. The data were analyzed by 1:1 model fitting to obtain affinity constants.

The results of affinity constant assay of 14C12H1L1(hG1DM), 14C12H1L1(hG4), 14C12H1L1(hG1WT) and 14C12H1L1(hG1TM) and the control antibody 5C10H2L2-IgG1mt to FcγRIIIa_F158 are shown in Table 3 and FIGs. 11-15.

Table 3: Kinetics for binding of 14C12H1L1 antibodies and isotypes thereof to FcγRIIIa_F158

| Antibody | $K_D$ (M) | kon (1/Ms) | SE (kon) | kdis (1/s) | SE (kdis) | Rmax (nm) |
|---|---|---|---|---|---|---|
| 14C12H1L1(hG1DM) | N/A | N/A | N/A | N/A | N/A | N/A |
| 14C12H1L1(hG4) | N/A | N/A | N/A | N/A | N/A | N/A |
| 14C12H1L1(hG1WT) | 1.02E-07 | 2.52E+05 | 2.93E+04 | 2.56E-02 | 1.12E-03 | 0.34-0.57 |
| 14C12H1L1(hG1TM) | N/A | N/A | N/A | N/A | N/A | N/A |
| 5C10H2L2-IgG1mt | N/A | N/A | N/A | N/A | N/A | N/A |

N/A indicates that the antibody had no binding or an extremely weak binding signal to the antigen, and thus the results were not analyzed and no corresponding data was obtained. The results showed that 14C12H1L1(hG1WT) bound to FcγRIIIa_F158 with an affinity constant of 1.02E-07M; 14C12H1L1(hG1DM), 14C12H1L1(hG4), 14C12H1L1(hG1TM) and 5C10H2L2-IgG1mt had no binding or an extremely weak binding signal to FcγRIIIa_F158, and thus the results were not analyzed and no corresponding data was obtained.

The results suggested that the binding activities of 14C12H1L1(hG1DM), 14C12H1L1(hG4) and 14C12H1L1(hG1TM) to FcγRIIIa_F158 are effectively eliminated as compared to 14C12H1L1(hG1WT).

## Experimental Example 3: Affinity Assay of 14C12H1L1(hG1DM), 14C12H1L1(hG4), 14C12H1L1(hG1WT) and 14C12H1L1(hG1TM) to Fc Recentor FcγRIIa and subtypes thereof

### (1) Affinity constant assay of 14C12H1L1(hG1DM), 14C12H1L1(hG4), 14C12H1L1(hG1WT) and 14C12H1L1(hG1TM) to FcγRIIa H131

The Fc receptor FcγRIIa_H131, also known as CD32a_H131, can bind to the Fc fragment of IgG antibodies and is involved in antibody-dependent cell-mediated cytotoxicity (ADCC). The binding capacity of a therapeutic monoclonal antibody to Fc receptors will influence the safety and efficacy of the antibody. The affinity constants of 14C12H1L1(hG1DM), 14C12H1L1(hG4), 14C12H1L1(hG1WT) and 14C12H1L1(hG1TM) to FcγRIIa_H131 were measured in this experiment using a Fortebio Octet system to evaluate the binding capacity of the antibodies to Fc receptor.

The method for determining the affinity constant of 14C12H1L1(hG1DM), 14C12H1L1(hG4), 14C12H1L1(hG1WT) and 14C12H1L1(hG1TM) to FcγRIIa_H131 by the Fortebio Octet system is briefly described as follows: the immobilization dilution buffer was a solution of PBS, 0.02% Tween-20 and 0.1% BSA, pH 7.4, and the analyte dilution buffer was a solution of 0.02% Tween-20, 0.02% casein and 0.1% BSA in PBS, pH 7.4. 5 μg/mL FcyRIIa_H131 was immobilized on the NTA sensor at an immobilization height of about 1.0 nm. The sensor was equilibrated in a buffer of 0.02% Tween-20, 0.02% casein and 0.1% BSA in PBS, pH 7.4 for 300 s of blocking, and the binding of the immobilized FcyRIIa_H131 on the sensor to the antibodies at concentrations of 12.5-200 nM (serial two-fold dilution) was determined for 60 s. The antibody was dissociated in the buffer for 60 s. The sensor was refreshed in 10 mM glycine pH 1.7 and 10 nM nickel sulfate. The temperature was 30 °C and the frequency was 0.6 Hz. The data were analyzed by 1:1 model fitting to obtain affinity constants.

The results of affinity constant assay of 14C12H1L1(hG1DM), 14C12H1L1(hG4), 14C12H1L1(hG1WT) and 14C12H1L1(hG1TM) and the control antibody 5C10H2L2-IgG1mt to FcγRIIa_H131 are shown in Table 4 and FIGs. 16-20.

Table 4: Kinetics for binding of 14C12H1L1 antibodies and isotypes thereof to FcγRIIa_H131

| Sample ID | $K_D$ (M) | kon (1/Ms) | SE (kon) | kdis (1/s) | SE (kdis) | Rmax (nm) |
|---|---|---|---|---|---|---|
| 14C12H1L1(hG1DM) | N/A | N/A | N/A | N/A | N/A | N/A |
| 14C12H1L1(hG4) | 5.07E-08 | 2.57E+05 | 2.37E+04 | 1.30E-02 | 6.47E-04 | 0.18-0.35 |
| 14C12H1L1(hG1WT) | 5.74E-08 | 4.65E+05 | 5.99E+04 | 2.67E-02 | 1.24E-03 | 0.82-1.12 |
| 14C12H1L1(hG1TM) | N/A | N/A | N/A | N/A | N/A | N/A |
| 5C10H2L2-IgG1mt | N/A | N/A | N/A | N/A | N/A | N/A |

N/A indicates that the antibody had no binding or an extremely weak binding signal to the antigen, and thus the results were not analyzed and no corresponding data was obtained. The results showed that both 14C12H1L1(hG4) and 14C12H1L1(hG1WT) bound to FcyRIIa_H131 with affinity constants of 5.07E-08M and 5.74E-08M, respectively; 14C12H1L1(hG1DM), 14C12H1L1(hG1TM) and 5C10H2L2-IgG1mt had no binding or an extremely weak binding signal to FcγRIIa_H131, and thus the results were not analyzed and no corresponding data was obtained.

The results suggested that the binding activities of 14C12H1L1(hG1DM) and 14C12H1L1(hG1TM) to FcγRIIa_H131 are effectively eliminated as compared to 14C12H1L1(hG4) and 14C12H1L1(hG1WT).

(2) Affinity constant assay of 14C12H1L1(hG1DM), 14C12H1L1(hG4), 14C12H1L1(hG1WT) and 14C12H1L1(hG1TM) to FcγRIIa R131

The Fc receptor FcγRIIa_R131, also known as CD32a_R131, can bind to the Fc fragment of IgG antibodies and is involved in antibody-dependent cell-mediated cytotoxicity (ADCC). The binding capacity of a therapeutic monoclonal antibody to Fc receptors will influence the safety and efficacy of the antibody. The affinity constants of 14C12H1L1(hG1DM), 14C12H1L1(hG4), 14C12H1L1(hG1WT) and 14C12H1L1(hG1TM) to FcyRIIa_R131 were measured in this experiment using a Fortebio Octet system to evaluate the binding capacity of the antibodies to Fc receptor.

The method for determining the affinity constant of 14C12H1L1(hG1DM), 14C12H1L1(hG4), 14C12H1L1(hG1WT) and 14C12H1L1(hG1TM) to FcγRIIa_R131 by the Fortebio Octet system is briefly described as follows: the immobilization dilution buffer was a solution of PBS, 0.02% Tween-20 and 0.1% BSA, pH 7.4, and the analyte dilution buffer was a solution of 0.02% Tween-20, 0.02% casein and 0.1% BSA in PBS, pH 7.4. 5 μg/mL FcyRIIa_R131 was immobilized on the NTA sensor at an immobilization height of about 1.0 nm. The sensor was equilibrated in a buffer of 0.02% Tween-20, 0.02% casein and 0.1% BSA in PBS, pH 7.4 for 300 s of blocking, and the binding of the immobilized FcyRIIa_R131 on the sensor to the antibodies at concentrations of 12.5-200 nM (serial two-fold dilution) was determined for 60 s. The antibody was dissociated in the buffer for 60 s. The sensor was refreshed in 10 mM glycine pH 1.7 and 10 nM nickel sulfate. The temperature was 30 °C and the frequency was 0.6 Hz. The data were analyzed by 1:1 model fitting to obtain affinity constants.

The results of affinity constant assay of 14C12H1L1(hG1DM), 14C12H1L1(hG4), 14C12H1L1(hG1WT) and 14C12H1L1(hG1TM) and the control antibody 5C10H2L2-IgG1mt to FcγRIIa_R131 are shown in Table 5 and FIGs. 21-25.

Table 5: Kinetics for binding of 14C12H1L1 antibodies and isotypes thereof to FcγRIIa_R131

| Antibody | $K_D$ (M) | kon (1/Ms) | SE (kon) | kdis (1/s) | SE (kdis) | Rmax (nm) |
|---|---|---|---|---|---|---|
| 14C12H1L1(hG1DM) | N/A | N/A | N/A | N/A | N/A | N/A |
| 14C12H1L1(hG4) | 3.13E-08 | 3.50E+05 | 3.05E+04 | 1.10E-02 | 6.43E-04 | 0.21-0.46 |
| 14C12H1L1(hG1WT) | 3.46E-08 | 6.60E+05 | 9.46E+04 | 2.28E-02 | 1.33E-03 | 0.39-0.83 |
| 14C12H1L1(hG1TM) | 2.32E-07 | 4.04E+05 | 9.45E+04 | 9.38E-02 | 4.89E-03 | 0.19-0.35 |
| 5C10H2L2-IgG1mt | N/A | N/A | N/A | N/A | N/A | N/A |

N/A indicates that the antibody had no binding or an extremely weak binding signal to the antigen, and thus the results were not analyzed and no corresponding data was obtained. The results showed that 14C12H1L1(hG4), 14C12H1L1(hG1WT) and 14C12H1L1(hG1TM) bound to FcγRIIa_R131 with affinity constants of 3.13E-08M, 3.46E-08M and 2.32E-07M, respectively; 14C12H1L1(hG1DM) and 5C10H2L2-IgG1mt had no binding or an extremely weak binding signal to FcγRIIa_R131, and thus the results were not analyzed and no corresponding data was obtained.

The results suggest that among the antibodies with binding activities, 14C12H1L1(hG1TM) has the weakest binding capacity and the lowest binding activity to FcyRIIa_R131 as compared to 14C12H1L1(hG4) and 14C12H1L1(hG1WT).

Experimental Example 4: Affinity constant assay of 14C12H1L1(hG1DM), 14C12H1L1(hG4), 14C12H1L1(hG1WT) and 14C12H1L1(hG1TM) to FcγRIIb

The Fc receptor FcyRIIb (also known as CD32b), can bind to the Fc fragment of IgG antibodies, down-regulate functions of immune cells, inhibit the activation and proliferation of immune cells and inhibit the secretion of cytokines. The affinity constants of the antibodies to FcyRIIb were measured in this experiment using a Fortebio Octet system to evaluate the binding capacity of 14C12H1L1(hG1DM), 14C12H1L1(hG4), 14C12H1L1(hG1WT) and 14C12H1L1(hG1TM) to Fc receptor.

The method for determining the affinity constant of 14C12H1L1(hG1DM), 14C12H1L1(hG4), 14C12H1L1(hG1WT) and 14C12H1L1(hG1TM) to FcγRIIb by the Fortebio Octet system is briefly described as follows: the immobilization dilution buffer was a solution of PBS, 0.02% Tween-20 and 0.1% BSA, pH 7.4, and the analyte dilution buffer was a solution of 0.02% Tween-20, 0.02% casein and 0.1% BSA in PBS, pH 7.4. 5 μg/mL hFcγRIIb-his was immobilized on the NTA sensor at an immobilization height of about 1.0 nm. The sensor was equilibrated in a buffer of 0.02% Tween-20, 0.02% casein and 0.1% BSA in PBS, pH 7.4 for 300 s of blocking, and the binding of the immobilized hFcyRIIb-his on the sensor to the antibodies at concentrations of 12.5-200 nM (serial two-fold dilution) was determined for 60 s. The antibody was dissociated in the buffer for 60 s. The sensor was refreshed in 10 mM glycine pH 1.7 and 10 nM nickel sulfate. The temperature was 30 °C and the frequency was 0.6 Hz. The data were analyzed by 1:1 model fitting to obtain affinity constants.

The results of affinity constant assay of 14C12H1L1(hG1DM), 14C12H1L1(hG4), 14C12H1L1(hG1WT) and 14C12H1L1(hG1TM) and the control antibody 5C10H2L2-IgG1mt to FcγRIIb are shown in Table 6 and FIGs. 26-30.

Table 6: Kinetics for binding of 14C12H1L1 antibodies and isotypes thereof to FcγRIIb

| **Antibody** | **$K_D$ (M)** | **kon (1/Ms)** | **SE (kon)** | **kdis (1/s)** | **SE (kdis)** | **Rmax (nm)** |
|---|---|---|---|---|---|---|
| **14C12H1L1(hG1DM)** | **N/A** | **N/A** | **N/A** | **N/A** | **N/A** | **N/A** |
| **14C12H1L1(hG4)** | **5.62E-08** | **2.88E+05** | **2.94E+04** | **1.62E-02** | **7.63E-04** | **0.22-0.33** |
| **14C12H1L1(hG1WT)** | **6.13E-08** | **3.18E+05** | **3.48E+04** | **1.95E-02** | **8.96E-04** | **0.16-0.37** |
| **14C12H1L1(hG1TM)** | **N/A** | **N/A** | **N/A** | **N/A** | **N/A** | **N/A** |
| **5C10H2L2-IgG1mt** | **N/A** | **N/A** | **N/A** | **N/A** | **N/A** | **N/A** |

N/A indicates that the antibody had no binding or an extremely weak binding signal to the antigen, and thus the results were not analyzed and no corresponding data was obtained. The results showed that both 14C12H1L1(hG4) and 14C12H1L1(hG1WT) bound to FcγRIIb with affinity constants of 5.62E-08M and 6.13E-08M, respectively; 14C12H1L1(hG1DM), 14C12H1L1(hG1TM) and 5C10H2L2-IgG1mt had no binding or an extremely weak binding signal to FcγRIIb, and thus the results were not analyzed and no corresponding data was obtained.

The results suggested that the binding activities of 14C12H1L1(hG1DM) and 14C12H1L1(hG1TM) to FcγRIIb are effectively eliminated as compared to 14C12H1L1(hG4) and 14C12H1L1(hG1WT).

Experimental Example 5: Affinity assay of 14C12H1L1(hG1DM), 14C12H1L1(hG4), 14C12H1L1(hG1WT) and 14C12H1L1(hG1TM) to C1q

Serum complement C1q can bind to the Fc fragment of IgG antibodies and mediate CDC effects. The binding capacity of a therapeutic monoclonal antibody to C1q will influence the safety and efficacy of the antibody. The affinity constants of 14C12H1L1(hG1DM), 14C12H1L1(hG4), 14C12H1L1(hG1WT) and 14C12H1L1(hG1TM) to C1q were measured in this experiment using a Fortebio Octet system to evaluate the CDC activity of the antibodies.

The method for determining the affinity constants of the antibodies to C1q by the Fortebio Octet system is briefly described as follows: the sample dilution buffer was a solution of 0.02% Tween-20 and 0.1% BSA in PBS, pH 7.4. 50 μg/mL antibody was immobilized on the FAB2G sensor at an immobilization height of about 2.0 nm. The sensor was equilibrated in a buffer for 60 s for blocking, and the binding of the immobilized antibody on the sensor to the antigen C1q at concentrations of 1.25-20 nM (serial two-fold dilution) was determined for 60 s. The antigen and antibody were dissociated in the buffer for 60 s. The sensor was refreshed 4 times in 10 mM glycine pH 1.7, each for 5 s. The shaking

speed of the sample plate was 1000 rpm, the temperature was 30 °C and the frequency was 0.6 Hz. The data were analyzed by 1:1 model fitting to obtain affinity constants. The data acquisition software was Fortebio Data Acquisition 7.0, and the data analysis software was Fortebio Data Analysis 7.0.

The results of affinity constant assay of 14C12H1L1(hG1DM), 14C12H1L1(hG4), 14C12H1L1(hG1WT) and 14C12H1L1(hG1TM) and the control 5C10H2L2-IgG1mt to C1q are shown in Table 7 and FIGs. 31-35.

Table 7: Kinetics for binding of 14C12H1L1 antibodies and isotypes thereof to C1q

| Antibody | $K_D$ (M) | kon (1/Ms) | SE (kon) | kdis (1/s) | SE (kdis) | Rmax (nm) |
|---|---|---|---|---|---|---|
| 14C12H1L1(hG1DM) | N/A | N/A | N/A | N/A | N/A | N/A |
| 14C12H1L1(hG4) | N/A | N/A | N/A | N/A | N/A | N/A |
| 14C12H1L1(hG1WT) | 1.35E-09 | 4.83E+06 | 4.24E+05 | 6.54E-03 | 5.33E-04 | 0.32-0.53 |
| 14C12H1L1(hG1TM) | N/A | N/A | N/A | N/A | N/A | N/A |
| 5C10H2L2-IgG1mt | 4.43E-09 | 2.38E+06 | 4.21E+05 | 1.05E-02 | 1.10E-03 | 0.19-0.26 |

N/A indicates that the antibody had no binding or an extremely weak binding signal to the antigen, and thus the results were not analyzed and no corresponding data was obtained. The results showed that 14C12H1L1(hG1WT) bound to C1q with an affinity constant of 1.35E-09M; 14C12H1L1(hG1DM), 14C12H1L1(hG4) and 14C12H1L1(hG1TM) had no binding or an extremely weak binding signal to C1q, and thus the results were not analyzed and no corresponding data was obtained.

The results also showed that 5C10H2L2-IgG1mt bound to C1q with an affinity constant of 4.43E-09, indicating that it has binding activity to C1q and can cause CDC effects.

Experimental Example 6: Pharmacodynamic Activities of 14C12H1L1(hG1WT) and 14C12H1L1(hG1TM) in Co-culture System of Peripheral Blood Mononuclear Cells and Raji-PDL1 Cells

In this experiment, the pharmacodynamic activity of anti-PD-1 antibodies 14C12H1L1 (hG1WT) and 14C12H1L1 (hG1TM), and the control anti-PD-Ll antibodies 5C10H2L2-IgG1mt and nivolumab in relieving immunosuppression mediated by PD-1/PD-L1 were detected in a co-culture system of peripheral blood mononuclear cells and Raji-PDLl cells. In the mixed lymphocyte reaction, when isolated peripheral blood mononuclear cells (containing immune cells expressing immunocompetent PD-1) and the Raji-PDLl cells expressing PD-L1 are co-cultured, the interaction of PD-1 and PD-L1 can mediate the function inhibition of the immune cells, showing reduced secretion of cytokines IFN-$\gamma$ and IL-2. Anti-PD-1 or anti-PD-Ll antibodies can relieve such immunosuppression of immune cells, leading to increased cytokine secretion. Raji is a B cell line. As described above, B cells can be used as antigen presenting cells to mediate the immune response of immune cells to tumor cells. In the present invention, the Raji-PDLl and PBMCs co-culture system was used to evaluate the pharmacological activity of the anti-PD-1 antibodies, and a Raji-PDL1, PBMCs and tumor cell co-culture system was used to evaluate the pharmacological activity of the anti-PD-1 antibodies in different tumors.

Peripheral blood mononuclear cells were isolated by the Ficoll-Paque Plus (GE Healthcare, Cat No.: 171440-02), and then stimulated with SEB (0.5 $\mu$g/mL) for two days. The stimulated mature peripheral blood mononuclear cells ($1\times10^5$ cells/well) and Raji-PDLl cells ($1\times10^5$ cells/well) treated with MMC (Mito-mycin C with a treatment concentration of 2 $\mu$g/mL) for 1 h were added to a 96-well plate before 14C12H1L1(hG1WT), 14C12H1L1(hG1TM), the control antibody nivolumab or the control anti-PD-Ll antibody 5C10H2L2-IgG1mt was added. The mixture was well mixed and incubated. After 3 days, the culture supernatant was collected and detected for IFN-$\gamma$ secretion and IL-2 secretion by an ELISA kit (purchased from Dakewe Biotech Co., Ltd.).

The results of the IFN-$\gamma$ secretion in the mixed lymphocyte reaction are shown in FIG. 36. The results showed that in the co-culture system of PBMCs and Raji-PDL1, 14C12H1L1(hG1TM) induced a significantly higher IFN-$\gamma$ secretion than those induced by 14C12H1L1(hG1WT), nivolumab or 5C10H2L2-IgG1mt at the same dose level.

The results of the IL-2 secretion in mixed lymphocyte reaction are shown in FIG. 37. The results showed that in the co-culture system of PBMCs and Raji-PDL1, 14C12H1L1(hG1TM) induced a significantly higher IL-2 secretion than those induced by 14C12H1L1(hG1WT), nivolumab or 5C10H2L2-IgG1mt at the same dose level.

The results suggested that the pharmacodynamic activity of 14C12H1L1(hG1TM) in relieving the immunosuppression mediated by PD-1/PD-L1 is significantly superior to that of nivolumab, 14C12H1L1(hG1WT) or 5C10H2L2-IgG1mt.

Experimental Example 7: Antibody-Mediated Phagocytic Activities of Nivolumab, 14C12H1L1(hG1WT) and 14C12H1L1(hG1TM) on CHO-K1-PD1

To detect the antibody dependent cellular phagoxytosis (ADCP) activity, murine macrophages were used as effector cells and cell lines overexpressing PD1 were used as target cells. The femoral bone marrow of Blab/c mice (purchased from Guangdong Medical Laboratory Animal Center) was first aseptically collected and lysed by erythrocyte lysis buffer on ice for 5 min. The lysis was terminated with DMEM complete medium (containing 10% FBS), and the lysate was centrifuged at 1000 rpm and washed twice. The cell pellet was resuspended in 10 mL of DMEM complete medium and M-CSF were added at a working concentration of 100 ng/mL. The cells were cultured for 7 days at 37 °C and 5% $CO_2$ in a cell culture chamber for induction. Half of the medium was exchanged and M-CSF was added on Days 3 and 5. The induction of cells was completed on day 7. The cells were digested with 0.05% trypsin. Macrophages were collected, and centrifuged at 750x g for 5 min. The supernatant was discarded and the cells were suspended in DMEM complete medium (containing 10% FBS) and counted. The cell was adjusted to a proper density and filled into sterile EP tubes for further use.

CHO-K1-PD1 cells (a CHO-K1 cell line overexpressing PD1) were centrifuged at 170x g for 5 min, washed once with PBS, resuspended and counted. The viability was determined. Carboxyfluorescein diacetate succinimidyl ester (CFSE) was diluted to 2.5 μM with PBS to resuspend the cells (staining density: 10 million cells/mL). A proper amount of the cells were incubated in a cell incubator for 20 min. 6 mL of DMEM complete medium was added to stop the staining. The cells were centrifuged at 170x g for 5 min, and the supernatant was discarded. 1 mL of DMEM complete medium was added. The cells were incubated in an incubator for 10 min, and adjusted to the experiment density. The cells were coded as CHO-K-PD1-CFSE.

The test antibodies were diluted in DMEM complete medium to 20, 2 and 0.2 μg/mL (the working concentrations were 10, 1 and 0.1 μg/mL). An anti-HEL IgG1 antibody and a medium were used as the isotype control group and a blank control group. According to the study design, the diluted antibodies and CHO-K1-PD1-CFSE cells were added into 1.5-mL EP tubes containing macrophages (the final volume was 100 μL and the effector-to-target ratio was 50,000:150,000). The mixture was well mixed for resuspension and incubated in an incubator at 37 °C for 2 h. 800 μL of PBS containing 1% bovine serum albumin (BSA) was added at room temperature to each tube. The mixture was centrifuged at 500x g for 5 min, and the supernatant was discarded. The cells were washed once with 800 μL of 1% PBSA. APC anti-mouse/human CD11b antibody (Biolegend, Cat. No.: 101212) was diluted 400-fold with PBSA and added to the corresponding samples at 100 μL/sample. The mixture was mixed well, incubated on ice for 40 min, and washed with 800 μL of 1% PBSA and centrifuged at 1200× g for 5 min twice, and the supernatant was discarded. 200 μL of 1% PBSA was added to each tube to resuspend the cells. The cells were transferred to loading tube and analyzed by BD FACS Calibur flow cytometer. Macrophages in the system were $APC^+$ positive, and macrophages involved in phagocytosis were APC and CFSE double positive. The phagocytosis rate was determined as the ratio of the number of double positive cells to the number of APC positive cells, and the antibody-mediated ADCP activity was evaluated. The ADCP activity of each group, represented by P%, was calculated according to the following formulas:

$$\mathbf{P\%} = \frac{\textbf{Number of macrophages involved in phagocytosis}}{\textbf{Total number of macrophages}} \times \mathbf{100\%}$$

The results are shown in FIG. 38.

The results showed that at the same concentration, the phagocytic rates of 14C12H1L1(hG1WT) and nivolumab were 3.94 and 4.26 times that of the isotype control anti-HEL antibody, respectively, indicating that 14C12H1L1 (hG1WT) and nivolumab have ADCP effects; and at the same concentration, the phagocytic rate of 14C12H1L1(hG1TM) was comparable to that of the isotype control antibody, indicating that 14C12H1L1(hG1TM) has no ADCP effect.

The results suggest that the amino acid mutation introduced by 14C12H1L1(hG1TM) can effectively eliminate the ADCP effect, and a surprising technical effect is obtained.

Experimental Example 8: Pharmacodynamic Evaluation of 14C12H1L1(hG1TM) + anlotinib hydrochloride in Scid/beige Immunodeficient Mouse Model Bearing Human Non-Small Cell Lung Cancer HCC827 Subcutaneous Xenograft Tumor

Female Scid/beige immunodeficient mice (purchased from Vital River) were divided into groups of 8.0.2 μg/mL *Staphylococcus aureus* enterotoxin B (SEB) was added to 1 million/mL PBMC suspension. PBMC s were incubated for 3 days for activation to increase the expression of PD1 on PBMCs. Mice were grafted subcutaneously with a mixture of 800,000 SEB-activated PBMCs and 6,000,000 HCC827 human non-small cell lung cancer cells (purchased from GuangZhou Jennio Biotech Co., Ltd.) on day 0, and divided into 2 groups, the isotype control antibody group (i.e., the anti-HEL antibody, prepared by Zhongshan Akeso Biopharma as described above) and the 14C12H1L1(hG1TM) +

anlotinib hydrochloride group. The 14C12H1L1(hG1TM) was administered through the tail vein once weekly (the first dose was co-administered subcutaneously with the cells), and anlotinib was administered by oral gavage once daily for 30 days. The specific protocol is shown in Table 8. Tumors were measured continuously in the experiment, and the volume was calculated according to the formula: a (tumor length) × b (tumor width) × b (tumor width)/2.

Table 8: Experimental planning and grouping

| Group | n | Model | Dosage information |
|---|---|---|---|
| **Isotype control** | **7** | **6,000,000 HCC827 cells + 800,000 PBMCs (SEB-activated for 3 days) Subcutaneous xenograft (d0)** | **Anti-HEL antibody, 10 mg/kg, administered via tail vein once weekly for 9 doses (the first dose was co-administered subcutaneously with the cells)** |
| **14C12H1L1 (hG1TM) + anlotinib** | **8** | | **14C12H1L1(hG1TM), 10 mg/kg, administered via tail vein once weekly for 9 doses (the first dose was co-administered subcutaneously with the cells) Anlotinib hydrochloride, 3 mg/kg, administered by oral gavage daily for 30 days** |

The experimental results are shown in FIG. 39.

The results showed that 14C12H1L1(hG1TM) + anlotinib hydrochloride significantly inhibited the increase in tumor volume of human non-small cell lung cancer cells, indicating good tumor killing effects.

Experimental Example 9: Pharmacodynamic Evaluation of 14C12H1L1(hG1TM) in C57BL/6-hPD1/hPDL1/hCD73 Mouse Model Bearing Colon Cancer MC38-hPDL1/hCD73 Subcutaneous Graft Tumor

The mouse MC38 cell line is a mouse colorectal cancer cell line. It has been demonstrated that the MC38 cells line is a useful model for studying human MSI-H/dMMR tumors (Efremova M et al., Nat Commun., 2018; 9(1):32).

Female C57BL/6-hPD1/hPDL1/hCD73 mice (purchased from Nanjing GemPharmatech Co., Ltd.) were divided into groups of 8 and grafted subcutaneously on the right forelimb with colon cancer MC38-hPDL1/hCD73 cells (purchased from Nanjing GemPharmatech Co., Ltd.) ($2 \times 10^6$ cells/100 μL/mouse). The day of grafting was defined as D0. The dosing volume was adjusted according to the body weight: 10 μL/g mouse body weight (g). The anti-HEL antibody (the preparation and the source are the same as those of the Experimental Example 8) or 14C12H1L1(hG1TM) was administrated intraperitoneally twice weekly for 3 weeks, in a total of 6 doses. The specific protocol is shown in Table 9. Tumors were measured continuously in the experiment, and the volume was calculated as the following formula: tumor volume ($mm^3$) = (tumor length × $(\text{tumor width})^2$)/2.

Table 9: Protocol and grouping

| Group | N | Dose (mg/kg) | Frequency | Cycle |
|---|---|---|---|---|
| **Isotype Control** | **8** | **10** | **BIW* 3** | **6 doses in total** |
| **14C12H1L1 (hG1TM)** | **8** | **1** | **BIW*3** | **6 doses in total** |

The experimental results are shown in FIG. 40.

The results showed that as compared to the isotype control antibody, the tumor growth was inhibited, indicating that 14C12H1L1(hG1TM) can significantly inhibit the proliferation of MC38 cells, and can effectively treat solid tumors of the MSI-H/dMMR phenotype, such as colon and/or rectal cancers.

Experimental Example 10: Effectively Enhanced Immune Response of Immune Cells to Human Gastric Cancer KATO III Cells bv 14C12H1L1(hG1TM)

PBMCs were isolated from healthy human peripheral blood according to the Ficoll-Paque™ Plus reagent instruction, and the isolated PBMCs were counted and frozen. Raji-PDLl cells were cultured in RPMI 1640 + 10% FBS complete medium, and KATO III cells (purchased from Chinese Academy of Sciences Shanghai Cell Bank) were cultured in DMEM + 10% FBS complete medium. PBMCs were thawed and activated with 0.5 μg/mL SEB for two days. On the day of the experiment, Raji-PDLl cells were treated with 2 μg/mL MMC for 1 h. SEB-activated PBMCs and MMC-treated Raji-PDLl cells were collected, washed twice with PBS, resuspended in RPMI 1640 + 10% FBS complete medium and counted. Raji-PDLl and PBMC cells were seeded on 96-well plates at $1 \times 10^5$ cells/well. KATO III cells in logarithmic

growth phase were collected and seeded on the 96-well plate at 5 × $10^4$ cells/well. The diluted antibody was added according to the study design. The mixture was mixed evenly and incubated in a 5% $CO_2$ incubator at 37 °C for 3 days. After 3 days, the cell culture supernatant was collected and tested for IL-2 according to ELISA KIT instruction. The media in this experiment were all 10% FBS + RPMI 1640.

The experimental results are shown in FIG. 41.

The results showed that 14C12H1L1(hG1TM) co-cultured with human gastric cancer KATO III cells exhibited higher pharmacological activity as compared to 14C12H1L1(hG1WT) or nivolumab. 14C12H1L1(hG1TM) can stimulate PBMCs to secrete more IL-2 at the same concentration level, indicating potential for treating gastric cancer.

Experimental Example 11: Effectively Enhanced Immune Response of Immune Cells to Nasopharyngeal Cancer CNE-2Z Cells by 14C12H1L1(hG1TM)

Raji-PDL1, CNE-2Z cells (purchased from GuangZhou Jennio Biotech Co., Ltd.) and PBMCs were thawed, wherein the PBMCs were stimulated with SEB (0.5 μg/mL) for two days after 2 hours of thawing. On the day of the experiment, Raji-PDLl cells were treated with MMC (Mito-mycin C with a treatment concentration of 2 μg/mL and a cell treatment density of 200 × $10^4$ cells/mL) for 1 h. PBMCs were collected, and the treated Raji-PDLl cells were washed twice with PBS. The PBMCs and the Raji-PDLl cells were added to the cell plate at 10 × $10^4$ cells/well, and CNE-2Z cells were added at 3 × $10^4$ cells/well. The antibodies (with a final concentration of 300 nM and a final volume of 200 μL) were added according to the experimental design, and co-cultured with the cells for 3 days. The culture supernatant was collected and assayed for IL-2. The media in this experiment were all 10% FBS + RPMI 1640.

The results are shown in FIG. 42.

The results showed that 14C12H1L1(hG1TM) co-cultured with human nasopharyngeal cancer CNE-2Z cells exhibited higher pharmacological activity as compared to 14C12H1L1(hG1WT). 14C12H1L1(hG1TM) can stimulate PBMCs to secrete more IL-2 at the same concentration level, indicating potential for treating nasopharyngeal cancer.

Experimental Example 12: Effectively Enhanced Immune Response of Immune Cells to Mesothelioma NCI-H2452 Cells by 14C12H1L1(hG1TM)

Raji-PDL1, NCI-H2452 cells (purchased from Chinese Academy of Sciences, Shanghai Institutes for Biological Sciences) and PBMCs were thawed, wherein the PBMCs were stimulated with SEB (0.5 μg/mL) for two days after 2 hours of thawing. On the day of the experiment, Raji-PDLl cells were treated with MMC (Mito-mycin C with a treatment concentration of 2 μg/mL and a cell treatment density of 200 × $10^4$ cells/mL) for 1 h. PBMCs were collected, and the treated Raji-PDLl cells were washed twice with PBS. The PBMCs and the Raji-PDLl cells were added to the cell plate at 10 × $10^4$ cells/well, and NCI-H2452 cells were added at 3 × $10^4$ cells/well. The antibodies (with a final concentration of 300 nM and a final volume of 200 μL) were added according to the experimental design, and co-cultured with the cells for 3 days. The culture supernatant was collected and assayed for IL-2. The media in this experiment were all 10% FBS + RPMI 1640.

The results are shown in FIG. 43.

The results showed that 14C12H1L1(hG1TM) co-cultured with human mesothelioma NCI-H2452 cells exhibited higher pharmacological activity as compared to 14C12H1L1(hG1WT). 14C12H1L1(hG1TM) can stimulate PBMCs to secrete more IL-2 at the same concentration level, indicating potential for treating mesothelioma.

Experimental Example 12: Effectively Enhanced Immune Response of Immune Cells to Small Cell Lung Cancer NCI-H446 Cells by 14C12H1L1(hG1TM)

PBMCs were isolated from healthy human peripheral blood according to the Ficoll-Paque™ Plus reagent instruction, and the isolated PBMCs were counted and frozen. Raji-PDLl and NCI-H446 cells (purchased from Chinese Academy of Sciences, Shanghai Institutes for Biological Sciences) were cultured in RPMI 1640 + 10% FBS complete medium. PBMCs were thawed and activated with 0.5 μg/mL SEB for two days. On the day of the experiment, Raji-PDLl cells were treated with 2 μg/mL MMC for 1 h. SEB-activated PBMCs and MMC-treated Raji-PDLl cells were collected, washed twice with PBS, resuspended in RPMI 1640 + 10% FBS complete medium and counted. Raji-PDLl and PBMC cells were seeded on 96-well plates at 1 × $10^5$ cells/well. NCI-H446 cells in logarithmic growth phase were collected and seeded on the 96-well plate at 8 × $10^4$ cells/well. The diluted antibody was added according to the study design. The mixture was mixed evenly and incubated in a 5% $CO_2$ incubator at 37 °C for 3 days. After 3 days, the cell culture supernatant was collected and tested for IL-2 according to ELISA KIT instruction. The media in this experiment were all 10% FBS + RPMI 1640.

The results are shown in FIG. 44.

The results showed that 14C12H1L1(hG1TM) co-cultured with human small cell lung cancer NCI-H446 cells

exhibited equivalent or higher pharmacological activity as compared to 14C12H1L!I(hG1WT) and nivolumab on the basis of effectively eliminated ADCC, CDC and ADCP activities. 14C12H1L1(hG1TM) can stimulate PBMCs to secrete equivalent or more IL-2 at the same concentration level, indicating potential for treating small cell lung cancer.

Experimental Example 13: Effectively Enhanced Immune Response of Immune Cells to Human Nasopharyngeal Cancer CNE-2Z Cells bv 14C12H1L1(hG1TM) in combination with anlotinib hydrochloride

PBMCs were isolated from healthy human peripheral blood according to the Ficoll-Paque™ Plus reagent instruction, and the isolated PBMCs were counted and frozen. Raji-PDLl and CNE-2Z cells (purchased from GuangZhou Jennio Biotech Co., Ltd.) were cultured in RPMI 1640 + 10% FBS complete medium. PBMCs were thawed and activated with 0.5 μg/mL SEB for two days. On the day of the experiment, Raji-PDLl cells were treated with 2 μg/mL MMC for 1 h. SEB-activated PBMCs and MMC-treated Raji-PDLl cells were collected, washed twice with PBS, resuspended in RPMI 1640 + 10% FBS complete medium and counted. Raji-PDL1 and PBMC cells were seeded on 96-well plates at $1 \times 10^5$ cells/well. CNE-2Z cells in logarithmic growth phase were collected and seeded on the 96-well plate at $3 \times 10^4$ cells/well. The diluted antibodies and anlotinib were added according to the study design. The mixture was mixed evenly and incubated in a 5% $CO_2$ incubator at 37 °C for 3 days. After 3 days, the cell culture supernatant was collected and tested for IL-2 according to ELISA KIT instruction. The media in this experiment were all 10% FBS + RPMI 1640.

The results are shown in FIG. 45. As compared to the anti-HEL antibody and the anlotinib monotherapies, 14C12H1L1(hG1TM), 14C12H1L1(hG1WT) and nivolumab significantly enhanced the immune response of immune cells to human nasopharyngeal cancer CNE-2Z cells characterized by significantly increased IL-2 secretion level. 14C12H1L1(hG1TM) has superior pharmacological activity than those of 14C12H1L1(hG1WT) and nivolumab. Moreover, the pharmacological activity of 14C12H1L1(hG1TM) in combination with anlotinib in stimulating immune cell activation was superior to those of 14C12H1L1(hG1TM) monotherapy, 14C12H1L1(hG1WT) monotherapy, and nivolumab monotherapy and was also superior to those of 14C12H1L1(hG1WT) in combination with anlotinib and nivolumab in combination with anlotinib.

The above results indicated that 14C12H1L1(hG1TM) in combination with anlotinib has potential for treating human nasopharyngeal cancer.

Experimental Example 14: Significantly Enhanced Immune Resnonse of Immune Cells to MSI-H/dMMR Tumor SW48 Cells bv 14C12H1L1(hG1TM) in Combination with Anlotinib

SW48 is a human colorectal cancer cell line and is identified with MSI-H/dMMR phenotype (Branch P et al., (1995), Cancer Res, 55(11): 2304-2309.). It was used for detecting the enhanced immune cell response to tumor of MSI-H/dMMR phenotype by 14C12H1L1(hG1TM).

PBMCs were isolated from healthy human peripheral blood according to the Ficoll-Paque™ Plus reagent instruction, and the isolated PBMCs were counted and frozen. Raji-PDLl cells were cultured in RPMI 1640 + 10% FBS complete medium, and SW48 cells (purchased from GuangZhou Jennio Biotech Co., Ltd.) were cultured in DMEM + 10% FBS complete medium. PBMCs were thawed and activated with 0.5 μg/mL SEB for two days. On the day of the experiment, Raji-PDLl cells were treated with 2 μg/mL MMC for 1 h. SEB-activated PBMCs and MMC-treated Raji-PDLl cells were collected, washed twice with PBS, resuspended in RPMI 1640 + 10% FBS complete medium and counted. Raji-PDLl and PBMC cells were seeded on 96-well plates at $1 \times 10^5$ cells/well. SW48 cells in logarithmic growth phase were collected and seeded on the 96-well plate at $2 \times 10^5$ cells/well. The diluted antibody and anlotinib was added according to the study design. The mixture was mixed evenly and incubated in a 5% $CO_2$ incubator at 37 °C for 3 days. After 3 days, the cell culture supernatant was collected and tested for IL-2 according to ELISA KIT instruction. The media in this experiment were all 10% FBS + RPMI 1640.

The results are shown in FIG. 46.

The results showed that 14C12H1L1(hG1TM), 14C12H1L1(hG1WT) and nivolumab significantly enhanced the immune response of immune cells to human colorectal cancer cells SW48 cells of MSI-H/dMMR phenotype characterized by significantly increased secretion level of IL-2 as compared to anti-HEL antibody. 14C12H1L1(hG1TM) has superior pharmacological activity than that of 14C12H1L1(hG1WT).

Moreover, the pharmacological activity of 14C12H1L1(hG1TM) in combination with anlotinib in stimulating immune cell activation was superior to those of 14C12H1L1(hG1WT) monotherapy, 14C12H1L1(hG1TM) monotherapy, and nivolumab monotherapy and was also superior to those of 14C12H1L1(hG1WT) in combination with anlotinib and nivolumab in combination with anlotinib.

The above results showed that 14C12H1L1(hG1TM) in combination with anlotinib has potential for treating solid tumor of MSI-H/dMMR phenotype, particularly colon cancer and/or rectal cancer of MSI-H/dMMR phenotype.

Experimental Example 15: Significantly Enhanced Immune Response of Immune Cells to Human Colorectal Cancer SW837 Cells of MSI-H/dMMR Phenotype by 14C12H1L1(hG1TM)

SW837 is a human colorectal cancer cell line of non-MSI-H/dMMR (i.e., MSS) phenotype (Guo J et al., Cancer Res., 2011;71(8):2978-2987.), and was used for detecting the enhanced immune cell response to tumor of non-MSI-H/dMMR (i.e., MSS) phenotype by 14C12H1L1(hG1TM) in this example.

PBMCs were isolated from healthy human peripheral blood according to the Ficoll-Paque™ Plus reagent instruction, and the isolated PBMCs were counted and frozen. Raji-PDLl cells were cultured in RPMI 1640 + 10% FBS complete medium, and SW837 cells (purchased from Shanghai Honsun Biological Technology Co., Ltd) were cultured in 10% FBS + Leibovitz's L-15 complete medium (purchased from Gibco). PBMCs were thawed and activated with 0.5 μg/mL SEB for two days. On the day of the experiment, Raji-PDLl cells were treated with 2 μg/mL MMC for 1 h. SEB-activated PBMCs and MMC-treated Raji-PDL1 cells were collected, washed twice with PBS, resuspended in RPMI 1640 + 10% FBS complete medium and counted. Raji-PDLl and PBMC cells were seeded on 96-well plates at $1 \times 10^5$ cells/well. SW837 cells in logarithmic growth phase were collected and seeded on the 96-well plate at $5 \times 10^4$ cells/well. The diluted antibody was added according to the study design. The mixture was mixed evenly and incubated in a 5% $CO_2$ incubator at 37 °C for 3 days. After 3 days, the cell culture supernatant was collected and tested for IL-2 according to ELISA KIT instruction.

The results are shown in FIG. 47.

The results showed that 14C12H1L1(hG1TM), 14C12H1L1(hG1WT) and nivolumab significantly enhanced the immune response of immune cells to human colorectal cancer cells SW837 cells of non-MSI-H/dMMR phenotype. The pharmacological activity of 14C12H1L1(hG1TM) in the medium and high dose groups was superior to that of 14C12H1L1(hG1WT), characterized by significantly increased secretion level of IL-2.

The above results showed that 14C12H1L1(hG1TM) had better or equivalent pharmacological activity relative to 14C12H1L1(hG1WT) and nivolumab on the basis of effectively eliminating ADCC, CDC or ADCP effects, indicating the potential for treating solid tumor of non-MSI-H/dMMR (i.e., MSS) phenotype, particularly colon cancer and/or rectal cancer of non-MSI-H/dMMR phenotype.

Experimental Example 16: Significantly Enhanced Immune Response of Immune Cells to Human Colorectal Cancer SW837 Cells of MSI-H/dMMR Phenotype by 14C12H1L1(hG1TM) in Combination with Anlotinib

PBMCs were isolated from healthy human peripheral blood according to the Ficoll-Paque™ Plus reagent instruction, and the isolated PBMCs were counted and frozen. Raji-PDLl cells were cultured in RPMI 1640 + 10% FBS complete medium and SW837 cells were cultured in Leibovitz's L-15 + 10% FBS complete medium. PBMCs were thawed and activated with 0.5 μg/mL SEB for two days. On the day of the experiment, Raji-PDLl cells were treated with 2 μg/mL MMC for 1 h. SEB-activated PBMCs and MMC-treated Raji-PDLl cells were collected, washed twice with PBS, resuspended in RPMI 1640 + 10% FBS complete medium and counted. Raji-PDLl and PBMC cells were seeded on 96-well plates at $1 \times 10^5$ cells/well. SW837 cells in logarithmic growth phase were collected and seeded on the 96-well plate at $5 \times 10^4$ cells/well. The diluted antibody was added according to the study design. The mixture was mixed evenly and incubated in a 5% $CO_2$ incubator at 37 °C for 3 days. After 3 days, the cell culture supernatant was collected and tested for IL-2 according to ELISA KIT instruction.

The results are shown in FIG. 48.

The results show that as compared to 14C12H1L1(hG1WT) in combination with anlotinib and nivolumab in combination with anlotinib, 14C12H1L1(hG1TM) in combination with anlotinib significantly enhanced the immune response of immune cells to human colorectal cancer SW837 cells of the non-MSI-H/dMMR phenotype characterized by significantly increased IL-2 secretion level, indicating a superior therapeutic effect on solid tumors of non-MSI-H/dMMR phenotype, particularly colon cancer and/or rectal cancer of non-MSI-H/dMMR phenotype.

Although specific embodiments of the present invention have been described in detail, those skilled in the art will understand. Various modifications and substitutions can be made to those details according to all the teachings that have been disclosed, and these changes are all within the protection scope of the present invention. The full scope of the present invention is given by the appended claims and any equivalent thereof.

## Claims

1. An antibody, wherein

   a heavy chain variable region of the antibody comprises HCDR1-HCDR3 with amino acid sequences set forth in SEQ ID NOs: 19-21, respectively, and a light chain variable region of the antibody comprises LCDR1-LCDR3 with amino acid sequences set forth in SEQ ID NOs: 22-24, respectively;
   the antibody is of human IgG1 subtype;
   wherein, according to the EU numbering system, a heavy chain constant region of the antibody comprises mutations at any 2 or 3 of positions 234, 235 and 237, and an affinity constant of the antibody to FcγRIIIa and/or C1q is reduced after the mutation as compared to that before the mutation; preferably, the affinity constant is measured by a Fortebio Octet system.

2. The antibody according to claim 1, wherein according to the EU numbering system, the heavy chain constant region of the antibody comprises the following mutations:

   L234A and L235A;
   L234A and G237A;
   L235A and G237A;
   or
   L234A, L235A and G237A.

3. An antibody, wherein

   a heavy chain variable region of the antibody comprises HCDR1-HCDR3 with amino acid sequences set forth in SEQ ID NOs: 19-21, respectively, and a light chain variable region of the antibody comprises LCDR1-LCDR3

with amino acid sequences set forth in SEQ ID NOs: 22-24, respectively;
the antibody is of human IgG1 subtype;
wherein according to the EU numbering system, a heavy chain constant region of the antibody comprises the following mutations:

L234A and L235A;
L234A and G237A;
L235A and G237A;
or
L234A, L235A and G237A.

**4.** The antibody according to any of claims 1-3, wherein, according to the EU numbering system, the heavy chain constant region of the antibody further comprises one or more mutations selected from:
N297A, D265A, D270A, P238D, L328E, E233D, H268D, P271G, A330R, C226S, C229S, E233P, P331S, S267E, L328F, A330L, M252Y, S254T, T256E, N297Q, P238S, P238A, A327Q, A327G, P329A, K322A, T394D, G236R, G236A, L328R, A330S, P331S, H268A, E318A and K320A.

**5.** The antibody according to any of claims 1-4, wherein

the heavy chain variable region of the antibody comprises an amino acid sequence selected from SEQ ID NO: 2 and SEQ ID NO: 6; and
the light chain variable region of the antibody comprises an amino acid sequence selected from SEQ ID NO: 4 and SEQ ID NO: 8.

**6.** The antibody according to any of claims 1-4, wherein

the heavy chain variable region of the antibody comprises an amino acid sequence set forth in SEQ ID NO: 2, and the light chain variable region of the antibody comprises an amino acid sequence set forth in SEQ ID NO: 4;
the heavy chain variable region of the antibody comprises an amino acid sequence set forth in SEQ ID NO: 2, and the light chain variable region of the antibody comprises an amino acid sequence set forth in SEQ ID NO: 8;
the heavy chain variable region of the antibody comprises an amino acid sequence set forth in SEQ ID NO: 6, and the light chain variable region of the antibody comprises an amino acid sequence set forth in SEQ ID NO: 4;
or
the heavy chain variable region of the antibody comprises an amino acid sequence set forth in SEQ ID NO: 6, and the light chain variable region of the antibody comprises an amino acid sequence set forth in SEQ ID NO: 8.

**7.** The antibody according to any of claims 1-6, wherein

the heavy chain is set forth in SEQ ID NO: 16, and the light chain is set forth in SEQ ID NO: 12; or
the heavy chain is set forth in SEQ ID NO: 18, and the light chain is set forth in SEQ ID NO: 12.

**8.** The antibody according to any of claims 1-7, wherein the antibody binds to FcyRIIIa_F158, FcyRI, FcyRIIa_H131, FcγRIIIa_V158 and/or FcyRIIb with an affinity constant greater than about $10^{-7}$ M, for example, greater than about $10^{-6}$ M, $10^{-5}$ M, $10^{-4}$ M, or $10^{-3}$ M or greater; preferably, the affinity constant is measured by a Fortebio Octet system; preferably, the antibody has no binding signal or a binding signal of less than 0.1 nm to FcyRIIIa_F158, FcyRI, FcyRIIa_H131, FcγRIIIa_V158 and/or FcyRIIb; preferably, the binding signal refers to a response measured by a Fortebio Octet system.

**9.** The antibody according to any of claims 1-8, wherein the antibody binds to C1q with an affinity constant greater than about $10^{-9}$ M, for example, greater than about $10^{-8}$ M, $10^{-7}$ M, $10^{-6}$ M, or $10^{-5}$ M or greater; preferably, the affinity constant is measured by a Fortebio Octet system;
preferably, the antibody has no binding signal or a binding signal of less than 0.1 nm to C1q; preferably, the binding signal refers to a response measured by a Fortebio Octet system.

**10.** An isolated nucleic acid molecule, encoding the antibody according to any of claims 1-9.

**11.** A vector, comprising the isolated nucleic acid molecule according to claim 10.

**12.** A host cell, comprising the isolated nucleic acid molecule according to claim 10 or the vector according to claim 11.

**13.** A conjugate, comprising the antibody according to any of claims 1-9 and a conjugated moiety, wherein the conjugated moiety is a detectable label; preferably, the conjugated moiety is a radioisotope, a fluorescent substance, a luminescent substance, a colored substance, or an enzyme.

**14.** A kit, comprising the antibody according to any of claims 1-9 or the conjugate according to claim 13;
wherein preferably, the kit further comprises a second antibody specifically recognizing the antibody; optionally, the second antibody further comprises a detectable label, for example, a radioisotope, a fluorescent substance, a luminescent substance, a colored substance, or an enzyme.

**15.** Use of the antibody according to any of claims 1-9 or the conjugate according to claim 13 in preparing a kit for detecting the presence or level of PD-1 in a sample.

**16.** A pharmaceutical composition, comprising the antibody according to any of claims 1-9 or the conjugate according to claim 13, wherein, optionally, the pharmaceutical composition further comprises a pharmaceutically acceptable carrier and/or excipient.

**17.** The pharmaceutical composition according to claim 16, further comprising one or more anti-tumor chemotherapeutics;
preferably, the anti-tumor chemotherapeutic is a tyrosine kinase inhibitor; more preferably, the anti-tumor chemotherapeutic is anlotinib or a pharmaceutically acceptable salt thereof (e.g., hydrochloride salt), or lenvatinib or a pharmaceutically acceptable salt thereof (e.g., mesylate salt).

**18.** The pharmaceutical composition according to claim 16 or 17, wherein the unit dose of the pharmaceutical composition is 100-1000 mg, 200-800 mg, 200-500 mg, 300-600 mg, 400-500 mg, or 450 mg, based on the mass of the antibody.

**19.** A therapeutic combination, comprising the antibody according to any of claims 1-9, and at least one (e.g., 1, 2 or 3) anti-tumor chemotherapeutic.

**20.** The therapeutic combination according to claim 19, wherein the anti-tumor chemotherapeutic is a tyrosine kinase inhibitor; preferably, the anti-tumor chemotherapeutic is anlotinib or a pharmaceutically acceptable salt thereof (e.g., hydrochloride salt), or lenvatinib or a pharmaceutically acceptable salt thereof (e.g., mesylate salt).

**21.** The therapeutic combination according to claim 19 or 20, wherein the unit dose of the antibody is 100-1000 mg, 200-800 mg, 200-500 mg, 300-600 mg, 400-500 mg, or 450 mg.

**22.** The therapeutic combination according to claim 19 or 20, wherein the unit dose of the anti-tumor chemotherapeutic is 0.1-100 mg, 0.5-50 mg, 1-20 mg, 2-15 mg, 4-12 mg, or 8-12 mg.

**23.** The therapeutic combination according to any of claims 19-22, wherein

the therapeutic combination is a fixed combination, e.g., in the form of a solid pharmaceutical composition or a liquid pharmaceutical composition; or
the therapeutic combination is a non-fixed combination, e.g., the anti-PD-1 antibody and the anti-tumor chemotherapeutic in the non-fixed combination are each in the form of a pharmaceutical composition.

**24.** A kit product, comprising: the pharmaceutical composition according to any of claims 16-18 or the therapeutic combination according to any of claims 19-23, and a package insert.

**25.** Use of the antibody according to any of claims 1-9, the conjugate according to claim 13, the pharmaceutical composition according to any of claims 16-18 or the therapeutic combination according to any of claims 19-23 in preparing a medicament for treating and/or preventing a tumor or anemia, or in preparing a medicament for diagnosing a tumor or anemia, wherein preferably the tumor is selected from one or more of melanoma, renal cancer, prostate cancer, bladder cancer, colon cancer, rectal cancer, gastric cancer, liver cancer, lung cancer, ovarian cancer, leukemia, nasopharyngeal cancer and endometrial cancer;

preferably, the lung cancer is selected from one or more of non-small cell lung cancer, small cell lung cancer

and squamous cell lung cancer;
preferably, the gastric cancer is gastric adenocarcinoma or gastroesophageal junction adenocarcinoma;
preferably, the tumor is a solid tumor of MSI-H/dMMR phenotype; preferably, the tumor is selected from one or more of the following tumors of MSI-H/dMMR phenotype:
colon cancer, rectal cancer, endometrial cancer, gastric cancer, mesothelioma, sarcoma, adrenocortical carcinoma, malignant melanoma and ovarian germ cell neoplasm.

**26.** Use of the antibody according to any of claims 1-9, the conjugate according to claim 13, the pharmaceutical composition according to any of claims 16-18 or the therapeutic combination according to any of claims 19-23 in preparing:

a medicament for blocking the binding of PD-1 to PD-L1,
a medicament for down-regulating the activity or level of PD-1,
a medicament for relieving the immunosuppression of PD-1 in an organism, or
a medicament for elevating IFN-$\gamma$ and/or IL-2 expression in T lymphocytes.

**27.** The antibody according to any of claims 1-9, the conjugate according to claim 13, the pharmaceutical composition according to any of claims 16-18 or the therapeutic combination according to any of claims 19-23 for use in treating and/or preventing a tumor or anemia, or for use in diagnosing a tumor or anemia, wherein preferably the tumor is selected from one or more of melanoma, renal cancer, prostate cancer, bladder cancer, colon cancer, rectal cancer, gastric cancer, liver cancer, lung cancer, ovarian cancer, leukemia, nasopharyngeal cancer and endometrial cancer;

preferably, the lung cancer is selected from one or more of non-small cell lung cancer, small cell lung cancer and squamous cell lung cancer;
preferably, the gastric cancer is gastric adenocarcinoma or gastroesophageal junction adenocarcinoma;
preferably, the tumor is a solid tumor of MSI-H/dMMR phenotype; preferably, the tumor is selected from one or more of the following tumors of MSI-H/dMMR phenotype:
colon cancer, rectal cancer, endometrial cancer, gastric cancer, mesothelioma, sarcoma, adrenocortical carcinoma, malignant melanoma and ovarian germ cell neoplasm.

**28.** A method for treating and/or preventing a tumor or anemia, or a method of diagnosing a tumor or anemia, comprising: administering to a subject in need an effective amount of the antibody according to any of claims 1-9, the conjugate according to claim 13, the pharmaceutical composition according to any of claims 16-18 or the therapeutic combination according to any of claims 19-23, wherein preferably the tumor is selected from one or more of melanoma, renal cancer, prostate cancer, bladder cancer, colon cancer, rectal cancer, gastric cancer, liver cancer, lung cancer, ovarian cancer, leukemia, nasopharyngeal cancer and endometrial cancer;

preferably, the lung cancer is selected from one or more of non-small cell lung cancer, small cell lung cancer and squamous cell lung cancer;
preferably, the gastric cancer is gastric adenocarcinoma or gastroesophageal junction adenocarcinoma;
preferably, the tumor is a solid tumor of MSI-H/dMMR phenotype; preferably, the tumor is selected from one or more of the following tumors of MSI-H/dMMR phenotype:
colon cancer, rectal cancer, endometrial cancer, gastric cancer, mesothelioma, sarcoma, adrenocortical carcinoma, malignant melanoma and ovarian germ cell neoplasm.

**29.** The method according to claim 28, wherein the effective amount of the antibody is administered to the subject in need before or after surgical treatment and/or before or after radiotherapy.

**30.** The method according to claim 28 or 29, wherein the unit dose of the antibody is 0.1-100 mg per kg body weight, preferably 1-10 mg per kg body weight; alternatively, the unit dose of the antibody is 10-1000 mg, preferably 50-500 mg in each subject;

preferably, the dose is given once every 3 days, 4 days, 5 days, 6 days, 10 days, 1 week, 2 weeks or 3 weeks;
preferably, the route of administration is intravenous drip infusion or intravenous injection.

**31.** The method according to any of claims 28-30, wherein the administration of the antibody is performed in cycles of 2 or 3 weeks, and preferably, the antibody is administered intravenously on the first day of each cycle; preferably, the antibody is administered once every two or three weeks.

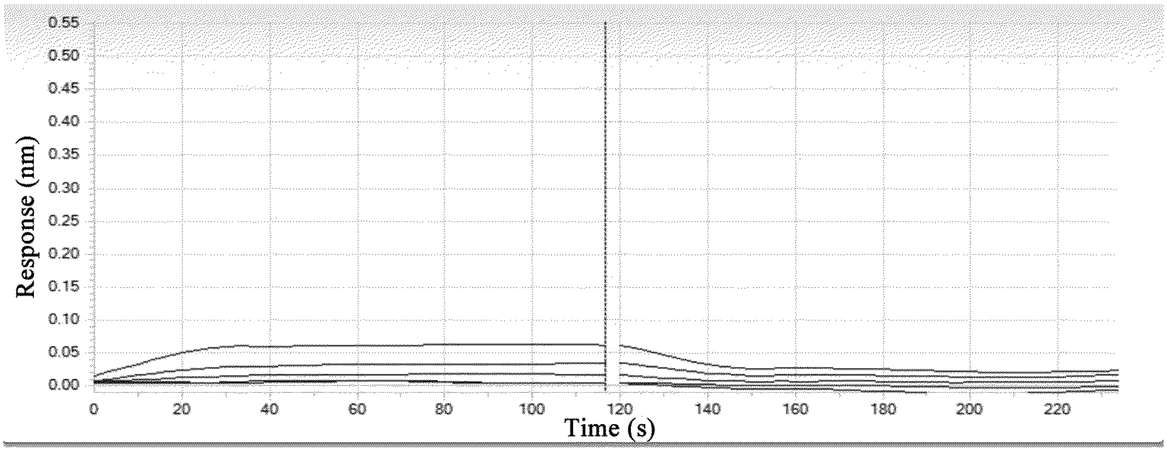
Response (nm)
0.55
0.50
0.45
0.40
0.35
0.30
0.25
0.20
0.15
0.10
0.05
0.00
0
20
40
60
80
100
120
140
160
180
200
220
Time (s)

FIG. 1

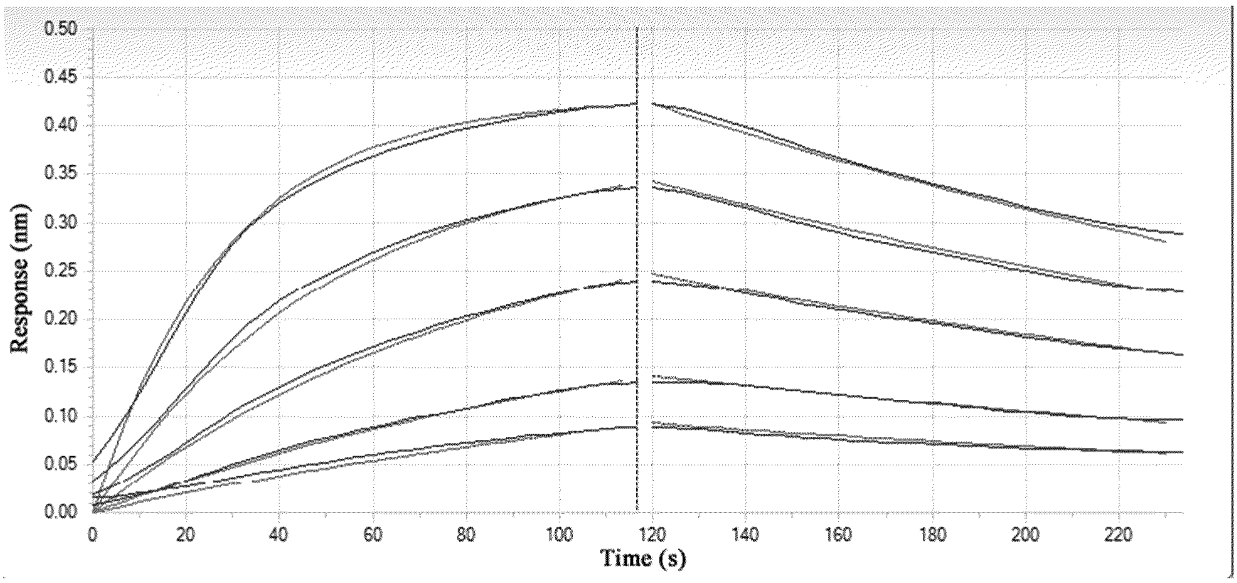
Response (nm)
0.50
0.45
0.40
0.35
0.30
0.25
0.20
0.15
0.10
0.05
0.00
0
20
40
60
80
100
120
140
160
180
200
220
Time (s)

FIG. 2

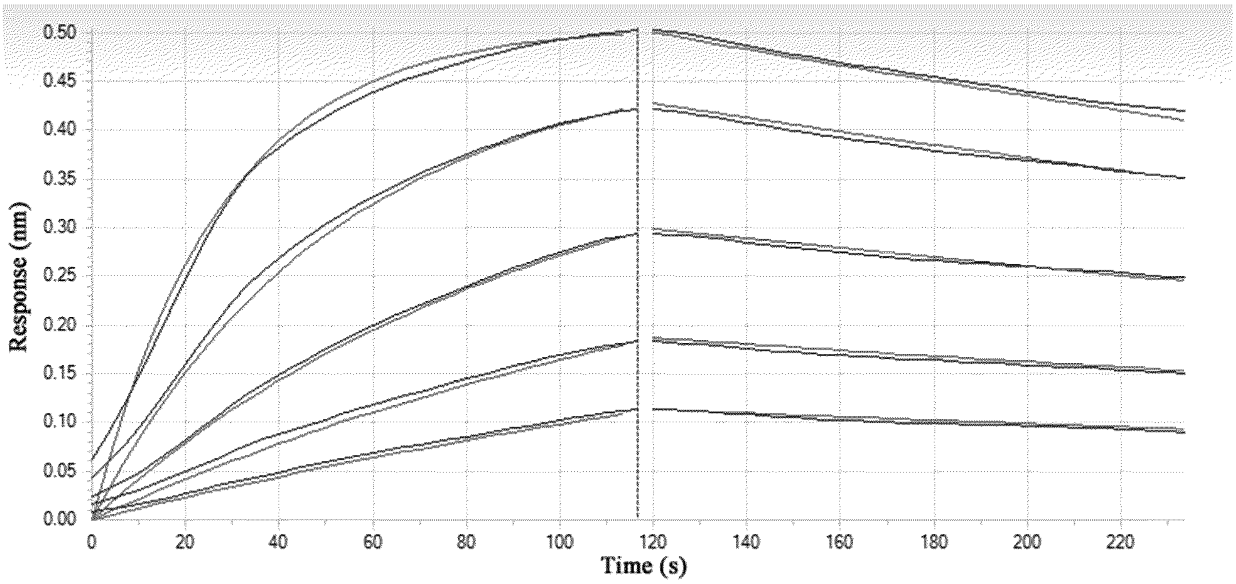
Response (nm)
0.50
0.45
0.40
0.35
0.30
0.25
0.20
0.15
0.10
0.05
0.00
0
20
40
60
80
100
120
140
160
180
200
220
Time (s)

FIG. 3

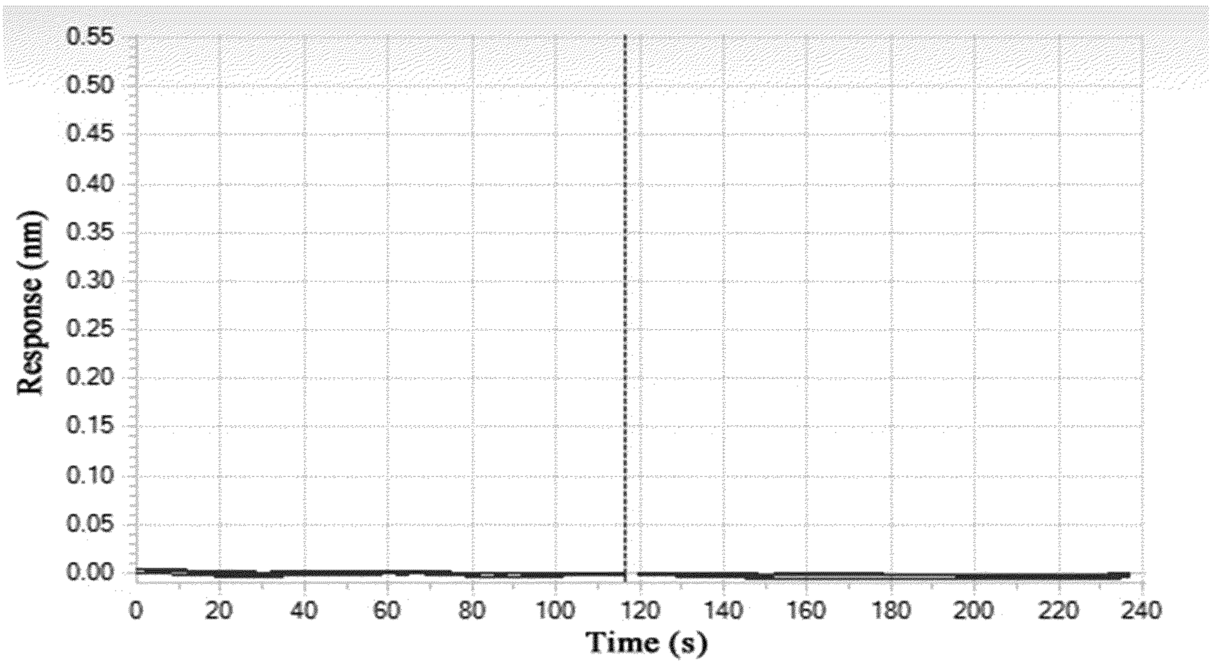
Response (nm)
0.55
0.50
0.45
0.40
0.35
0.30
0.25
0.20
0.15
0.10
0.05
0.00
0
20
40
60
80
100
120
140
160
180
200
220
240
Time (s)

FIG. 4

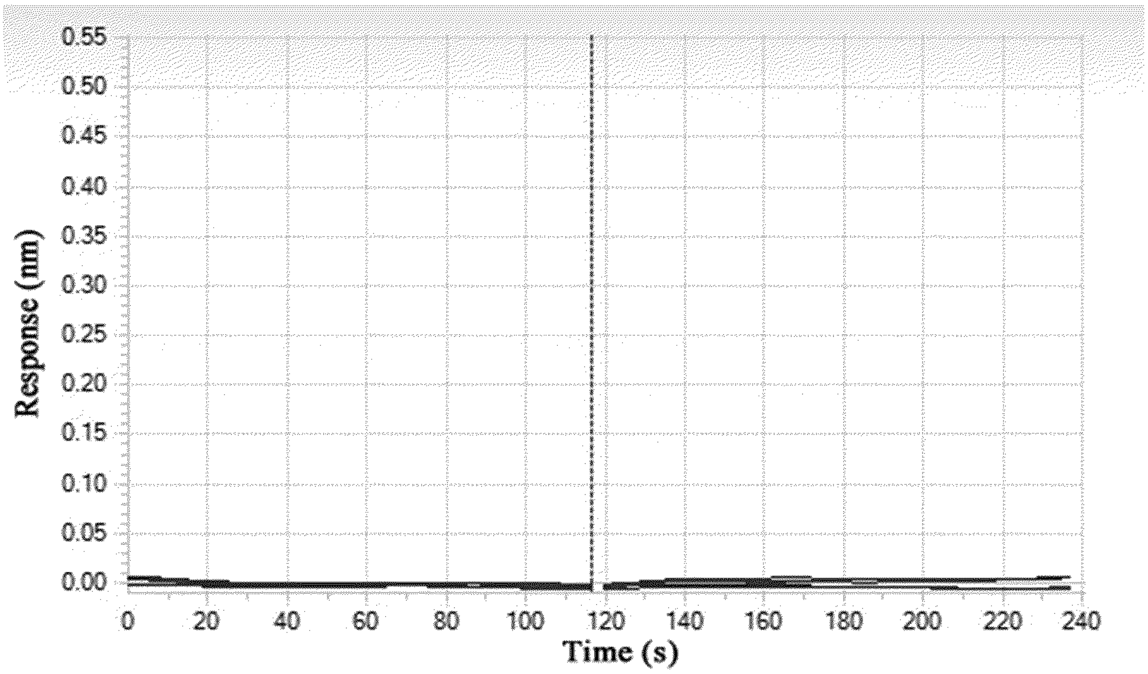
Response (nm)
0.55
0.50
0.45
0.40
0.35
0.30
0.25
0.20
0.15
0.10
0.05
0.00
0
20
40
60
80
100
120
140
160
180
200
220
240
Time (s)

FIG. 5

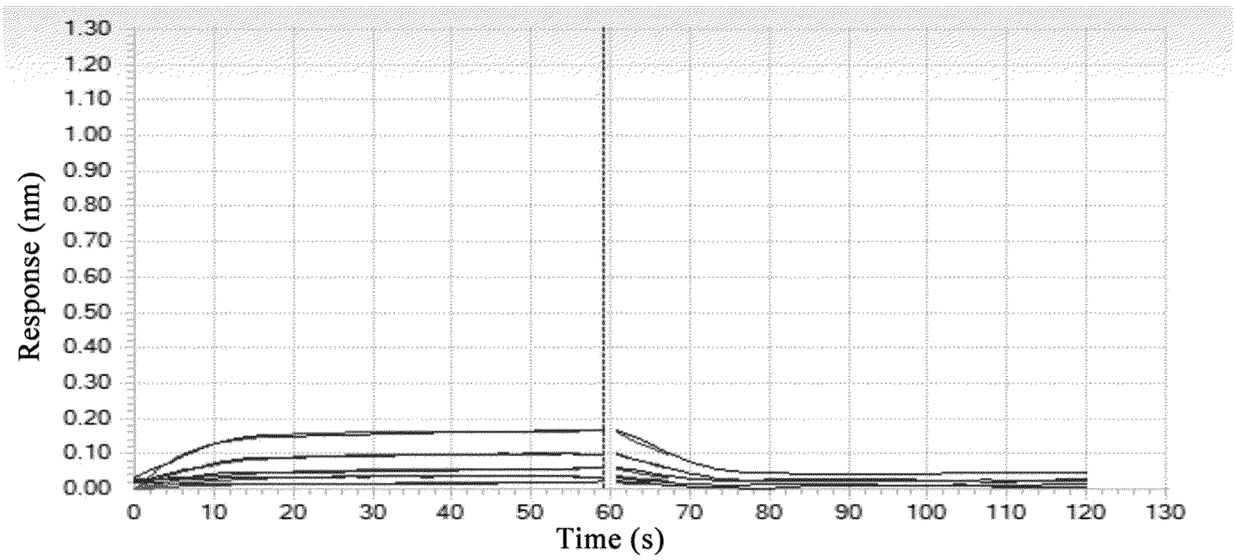
Response (nm)
1.30
1.20
1.10
1.00
0.90
0.80
0.70
0.60
0.50
0.40
0.30
0.20
0.10
0.00
0
10
20
30
40
50
60
70
80
90
100
110
120
130
Time (s)

FIG. 6

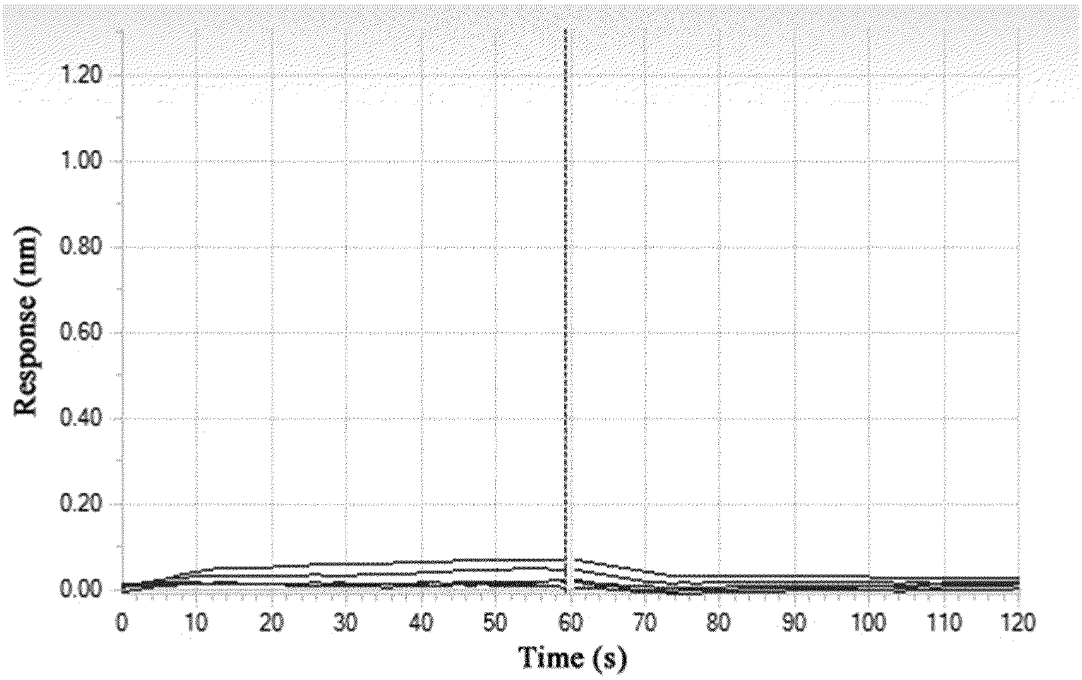

Response (nm)
1.20
1.00
0.80
0.60
0.40
0.20
0.00
0
10
20
30
40
50
60
70
80
90
100
110
120
Time (s)


FIG. 7

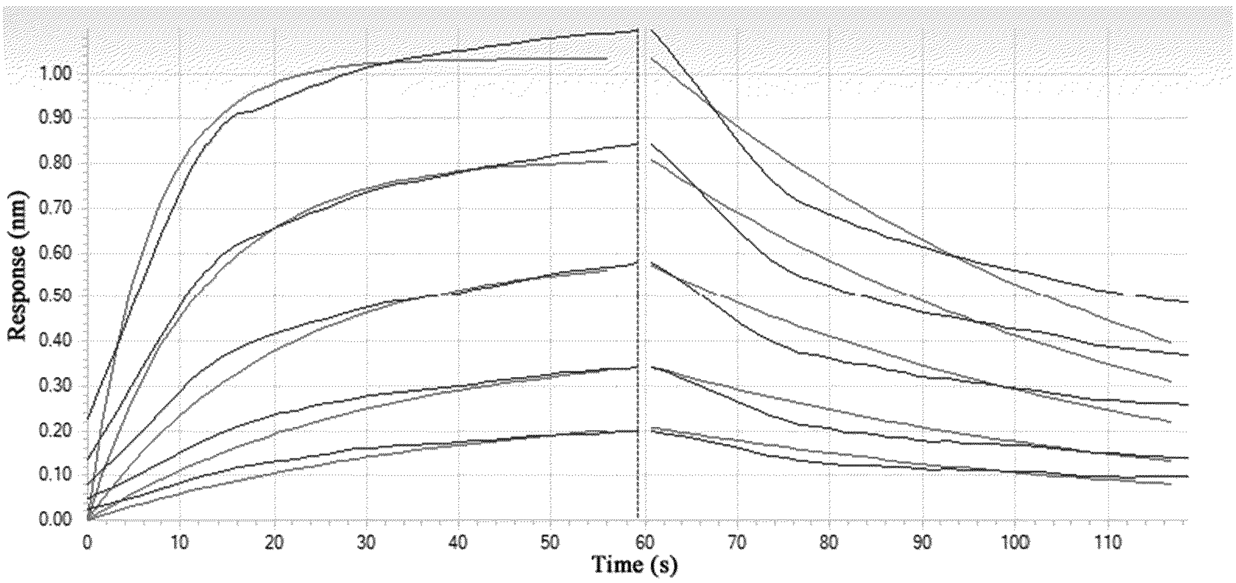

Response (nm)
1.00
0.90
0.80
0.70
0.60
0.50
0.40
0.30
0.20
0.10
0.00
0
10
20
30
40
50
60
70
80
90
100
110
Time (s)


FIG. 8

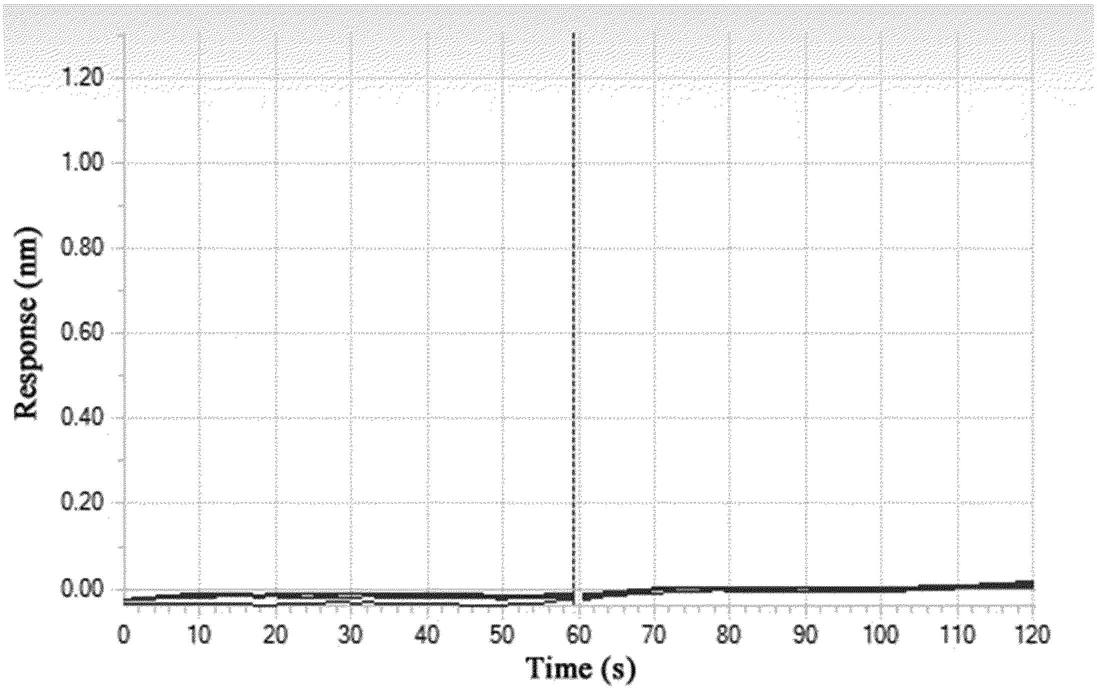
1.20
1.00
0.80
0.60
0.40
0.20
0.00
Response (nm)
0
10
20
30
40
50
60
70
80
90
100
110
120
Time (s)

FIG. 9

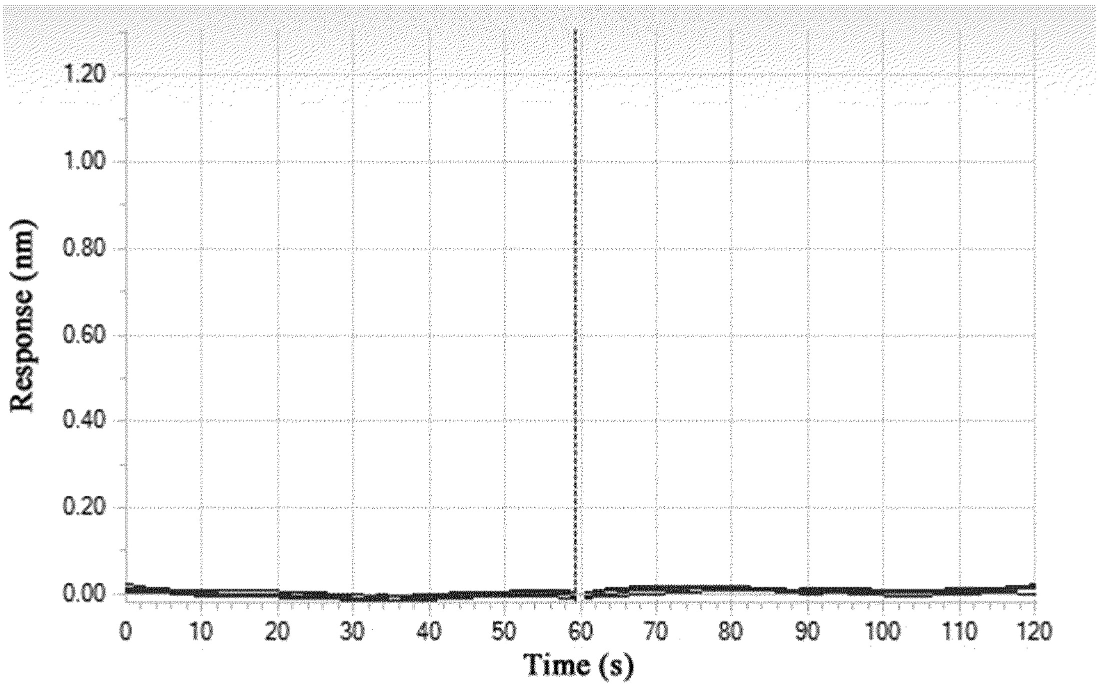
1.20
1.00
0.80
0.60
0.40
0.20
0.00
Response (nm)
0
10
20
30
40
50
60
70
80
90
100
110
120
Time (s)

FIG. 10

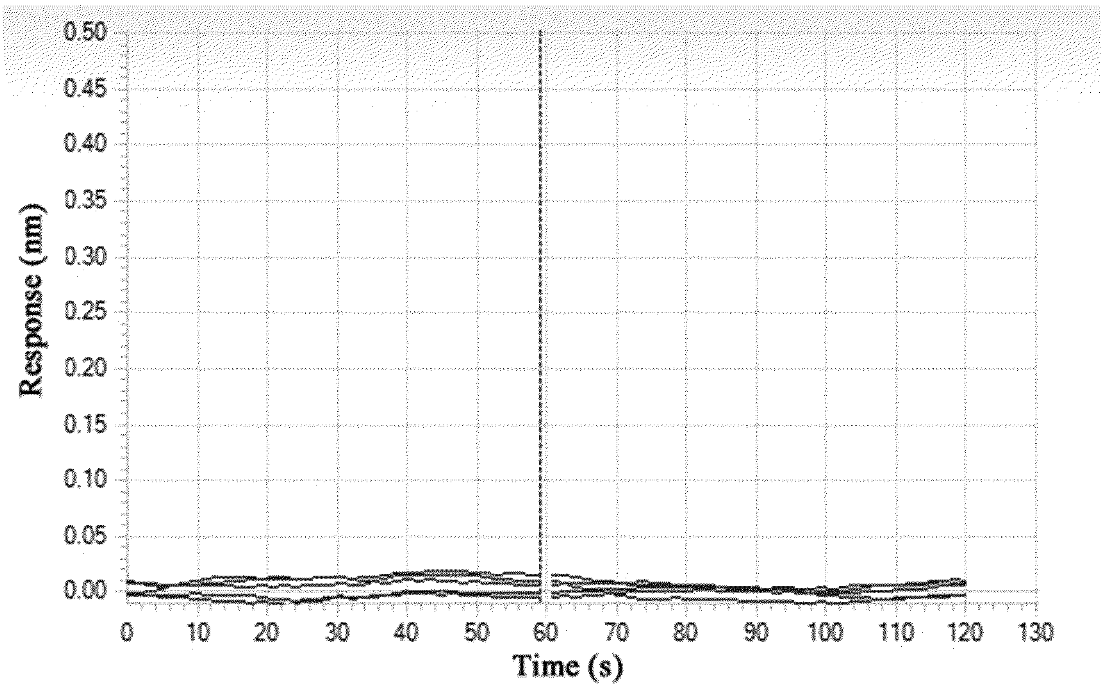
Response (nm)
0.50
0.45
0.40
0.35
0.30
0.25
0.20
0.15
0.10
0.05
0.00
0
10
20
30
40
50
60
70
80
90
100
110
120
130
Time (s)

FIG. 11

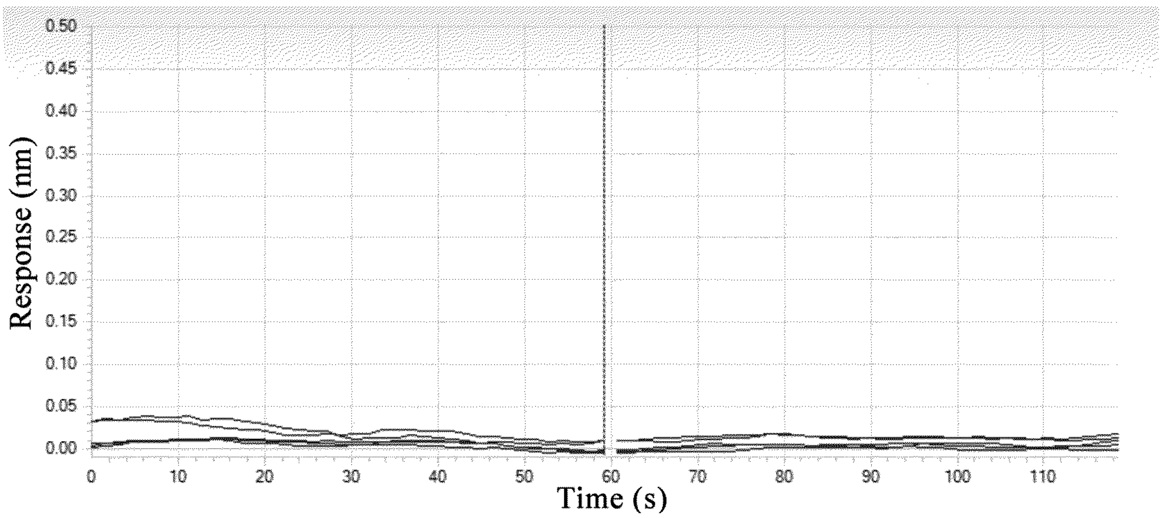
Response (nm)
0.50
0.45
0.40
0.35
0.30
0.25
0.20
0.15
0.10
0.05
0.00
0
10
20
30
40
50
60
70
80
90
100
110
Time (s)

FIG. 12

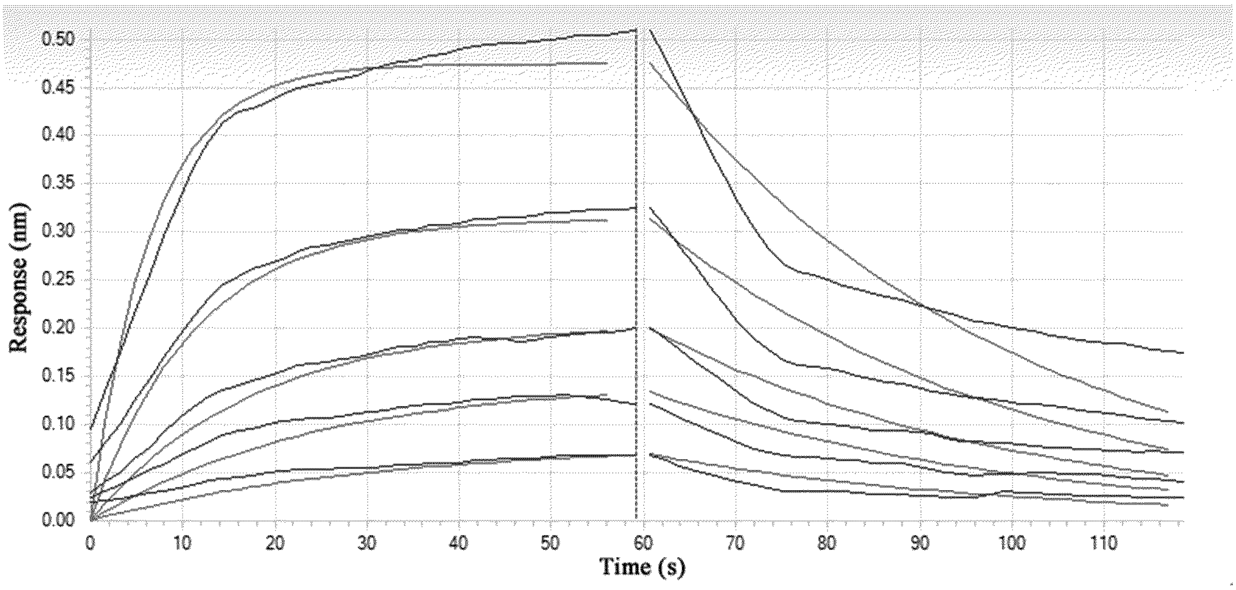


FIG. 13

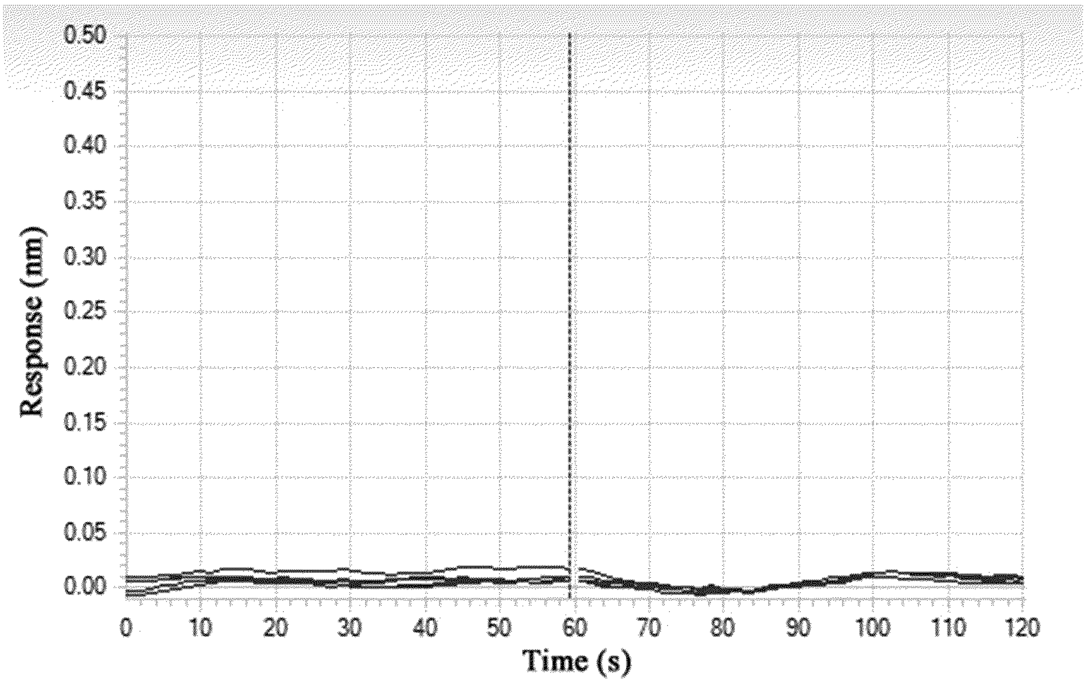


FIG. 14

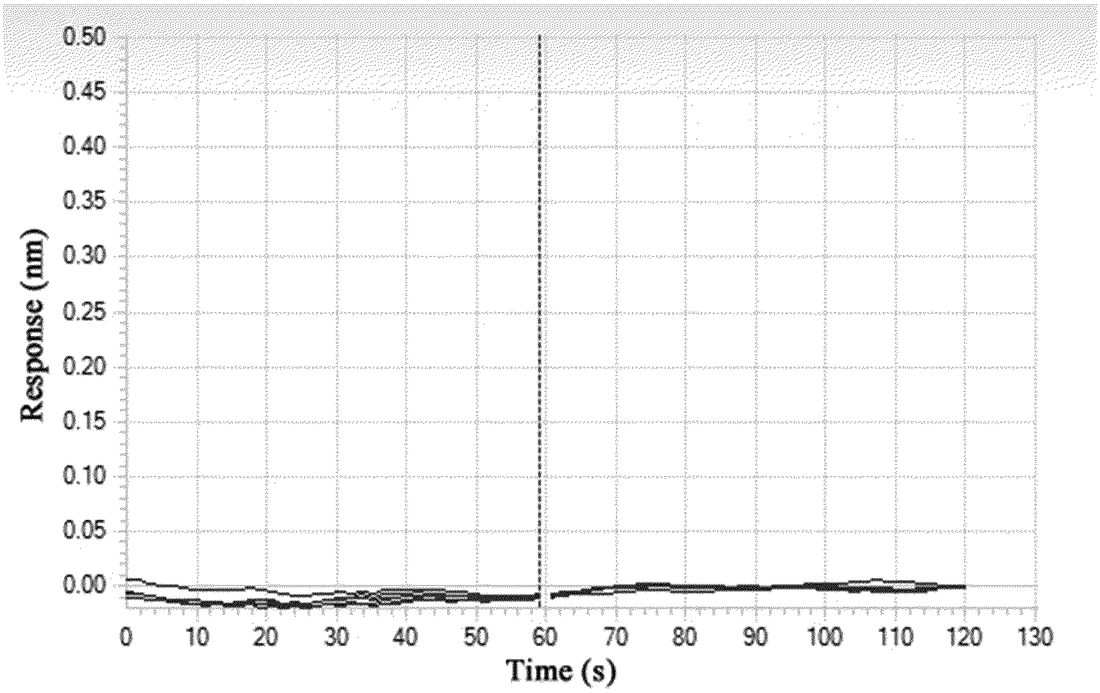
0.50
0.45
0.40
0.35
0.30
0.25
0.20
0.15
0.10
0.05
0.00
Response (nm)
0 10 20 30 40 50 60 70 80 90 100 110 120 130
Time (s)

FIG. 15

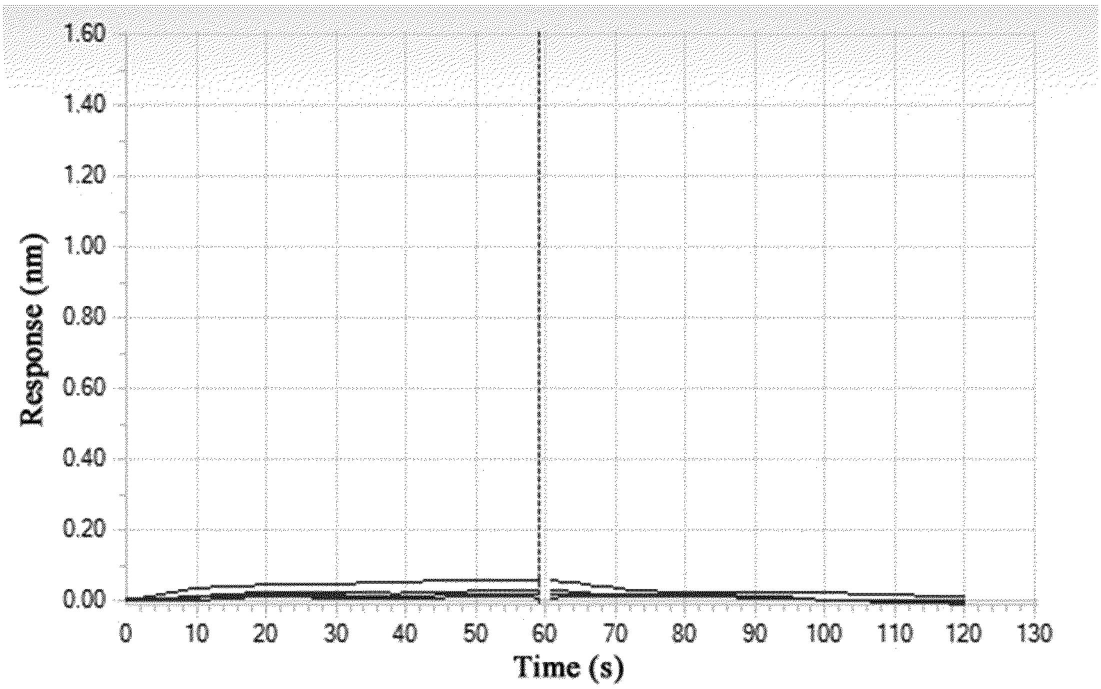
1.60
1.40
1.20
1.00
0.80
0.60
0.40
0.20
0.00
Response (nm)
0 10 20 30 40 50 60 70 80 90 100 110 120 130
Time (s)

FIG. 16

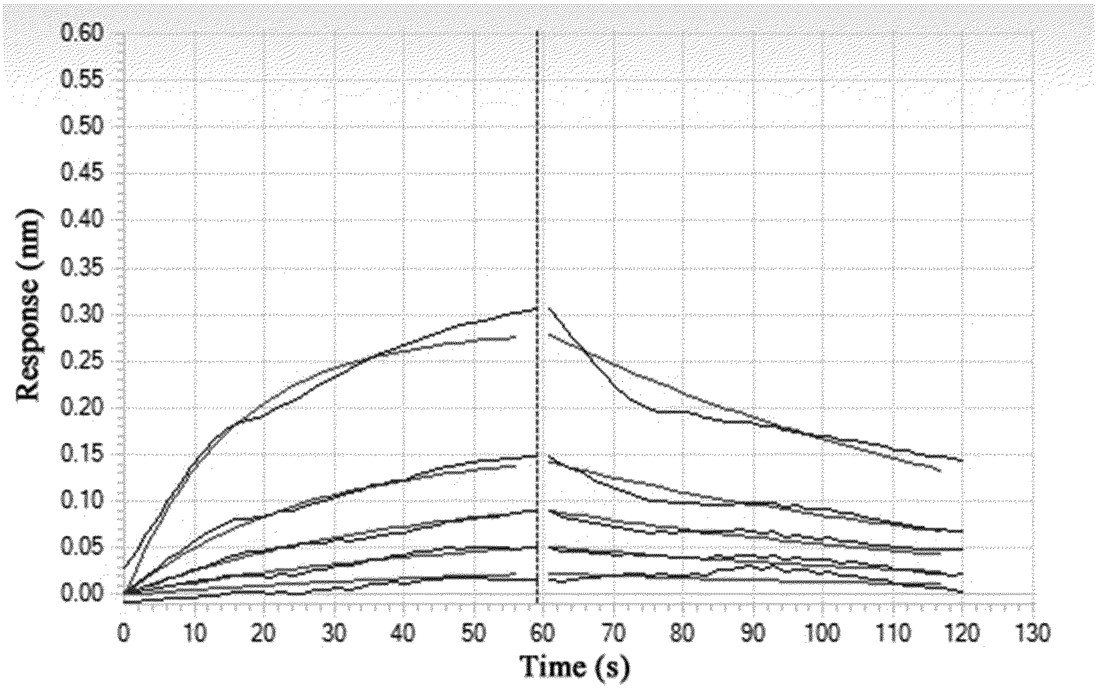
0.60
0.55
0.50
0.45
0.40
0.35
0.30
0.25
0.20
0.15
0.10
0.05
0.00
Response (nm)
0
10
20
30
40
50
60
70
80
90
100
110
120
130
Time (s)

FIG. 17

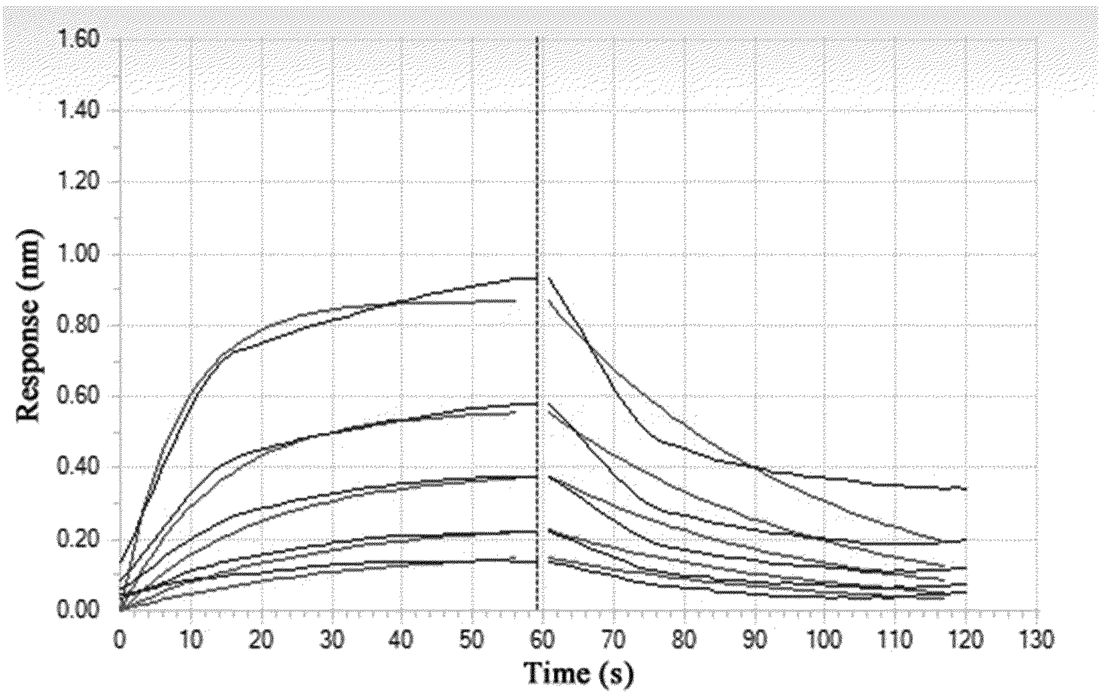
1.60
1.40
1.20
1.00
0.80
0.60
0.40
0.20
0.00
Response (nm)
0
10
20
30
40
50
60
70
80
90
100
110
120
130
Time (s)

FIG. 18

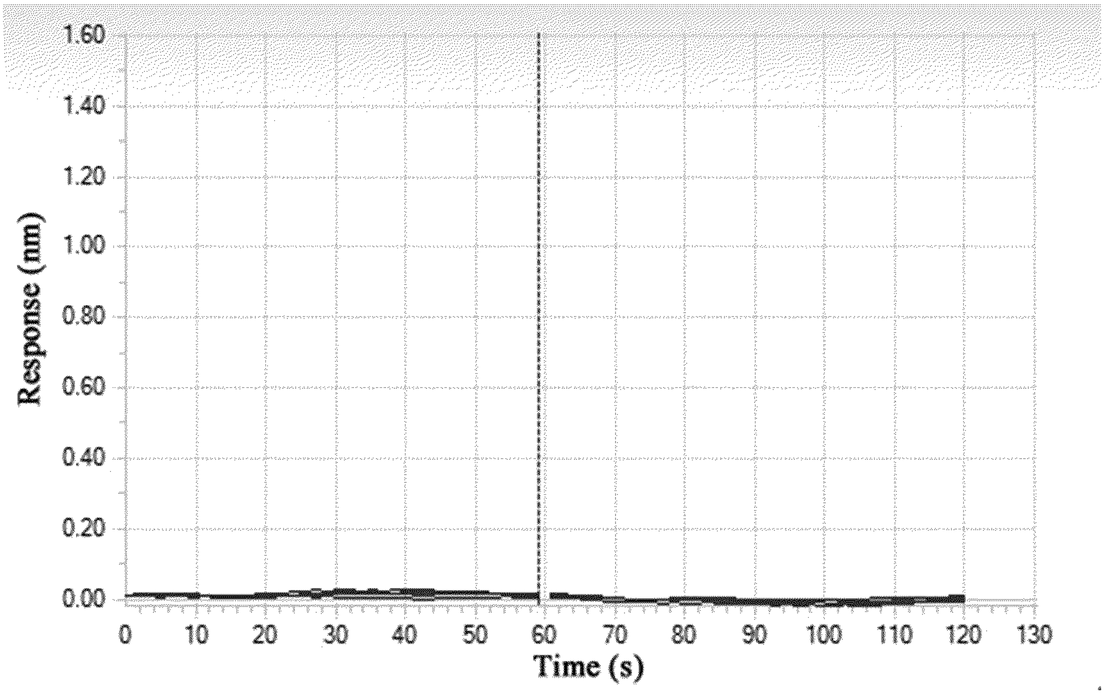
1.60
1.40
1.20
1.00
0.80
0.60
0.40
0.20
0.00
Response (nm)
0
10
20
30
40
50
60
70
80
90
100
110
120
130
Time (s)

FIG. 19

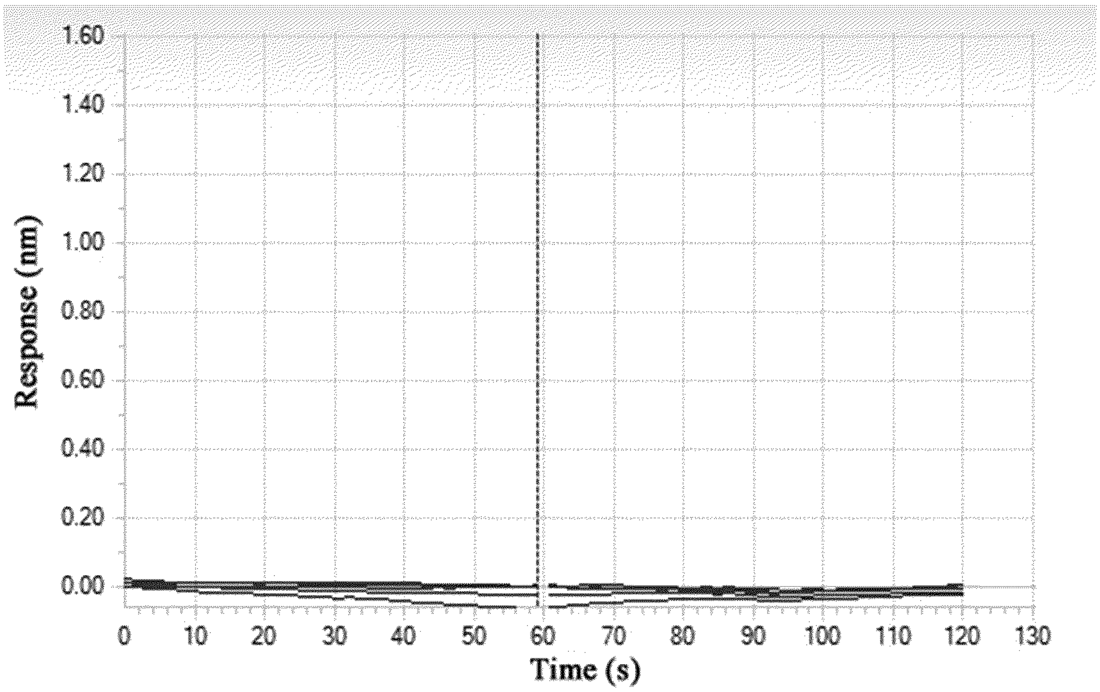
1.60
1.40
1.20
1.00
0.80
0.60
0.40
0.20
0.00
Response (nm)
0
10
20
30
40
50
60
70
80
90
100
110
120
130
Time (s)

FIG. 20

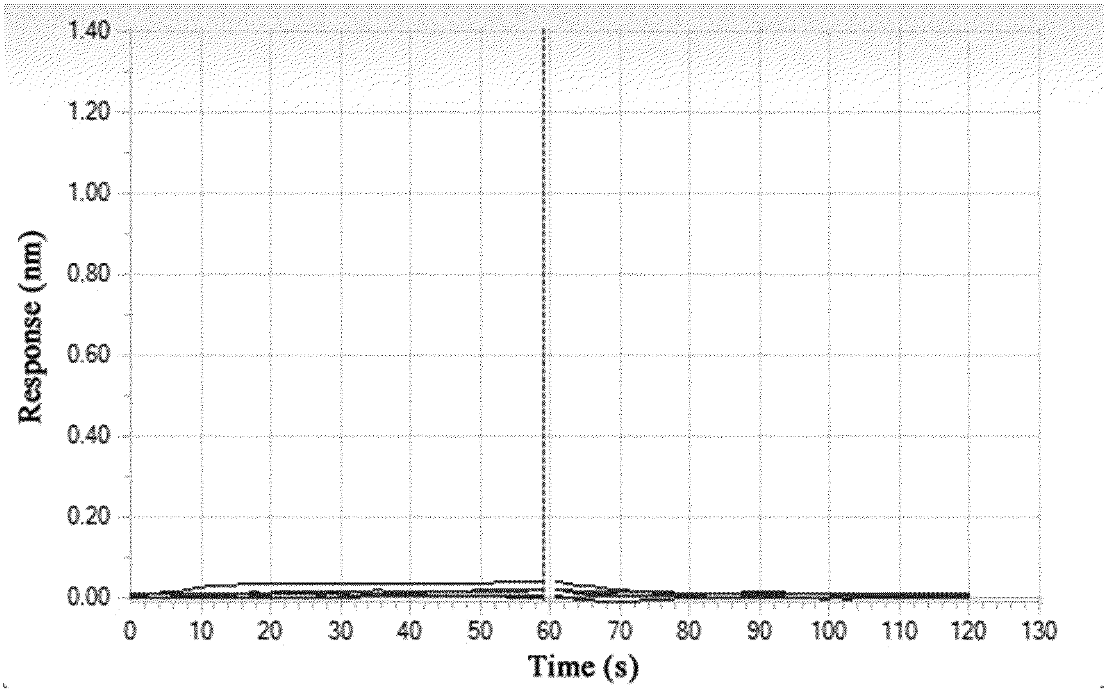
Response (nm)
1.40
1.20
1.00
0.80
0.60
0.40
0.20
0.00
0
10
20
30
40
50
60
70
80
90
100
110
120
130
Time (s)

FIG. 21

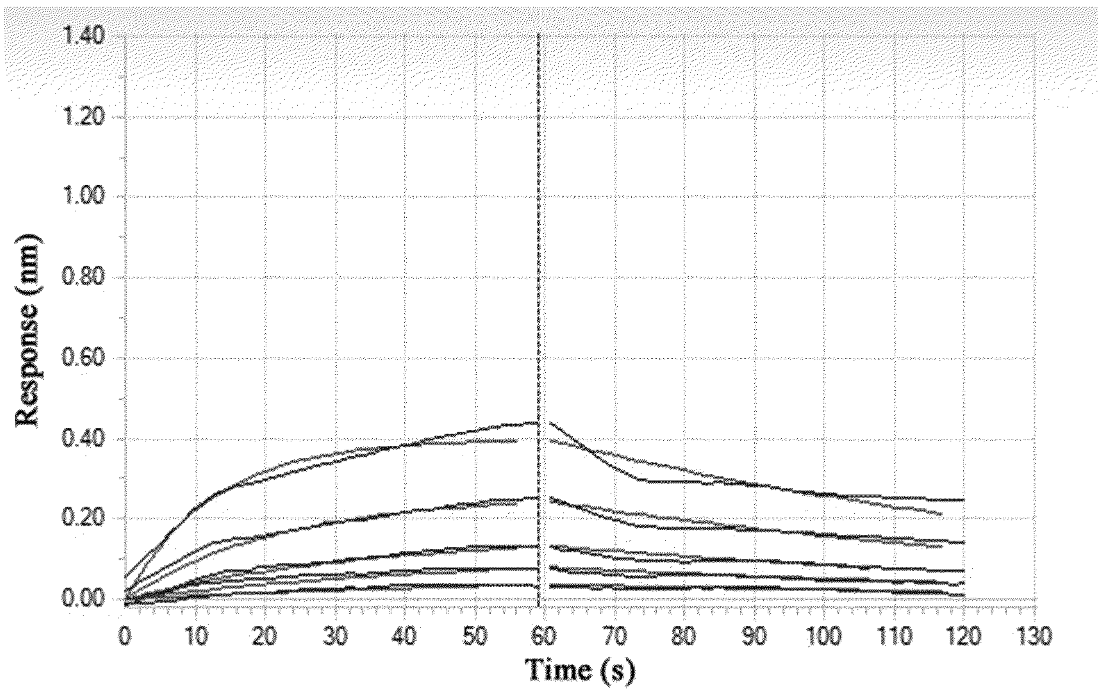
Response (nm)
1.40
1.20
1.00
0.80
0.60
0.40
0.20
0.00
0
10
20
30
40
50
60
70
80
90
100
110
120
130
Time (s)

FIG. 22

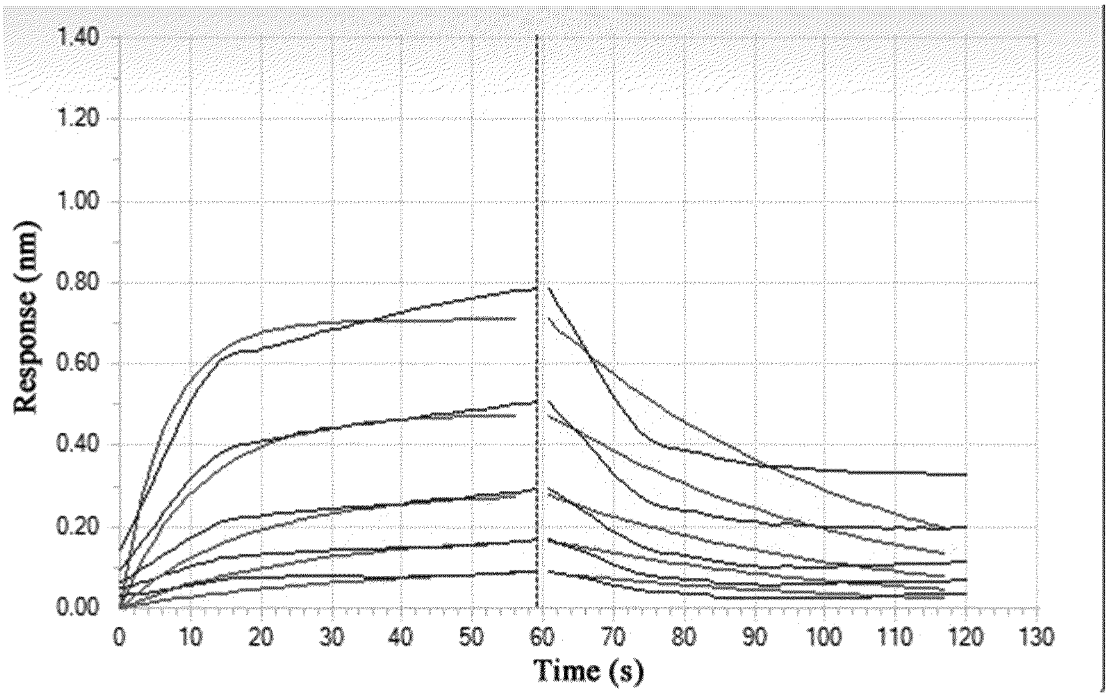
1.40
1.20
1.00
0.80
0.60
0.40
0.20
0.00
Response (nm)
0
10
20
30
40
50
60
70
80
90
100
110
120
130
Time (s)

FIG. 23

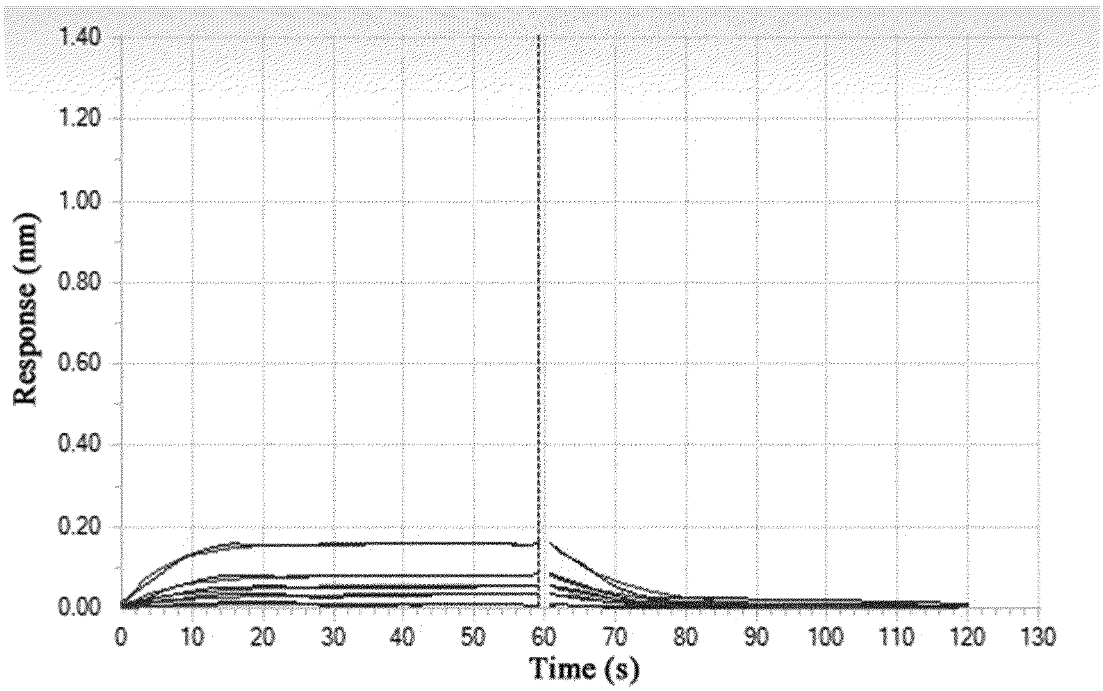
1.40
1.20
1.00
0.80
0.60
0.40
0.20
0.00
Response (nm)
0
10
20
30
40
50
60
70
80
90
100
110
120
130
Time (s)

FIG. 24

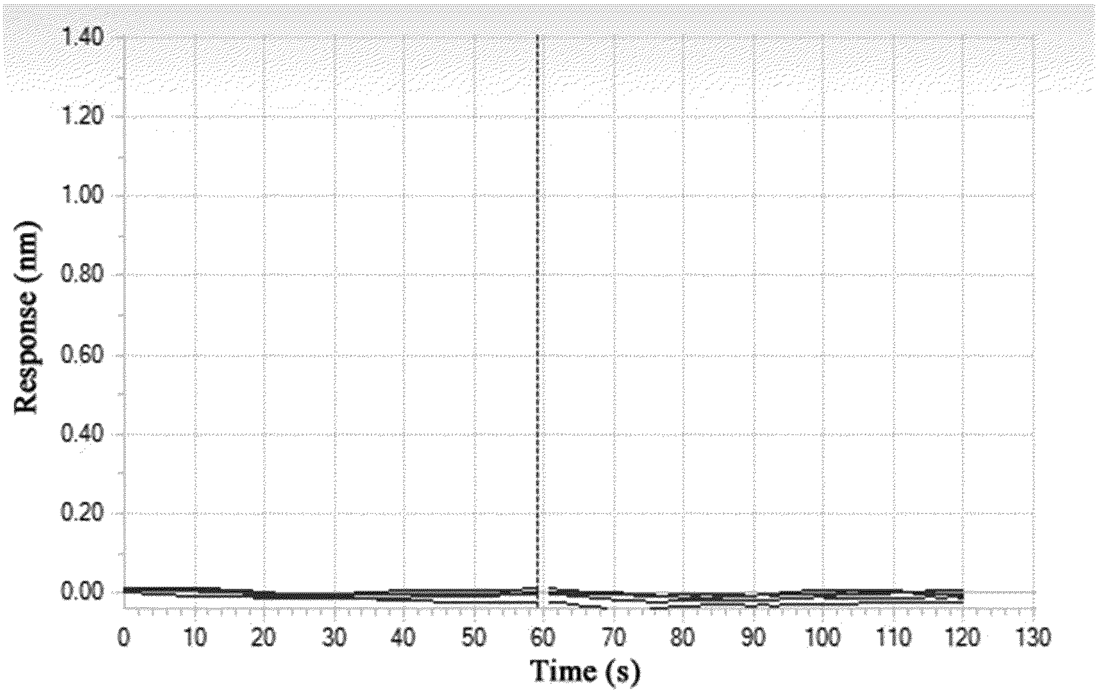
1.40
1.20
1.00
0.80
0.60
0.40
0.20
0.00
Response (nm)
0
10
20
30
40
50
60
70
80
90
100
110
120
130
Time (s)

FIG. 25

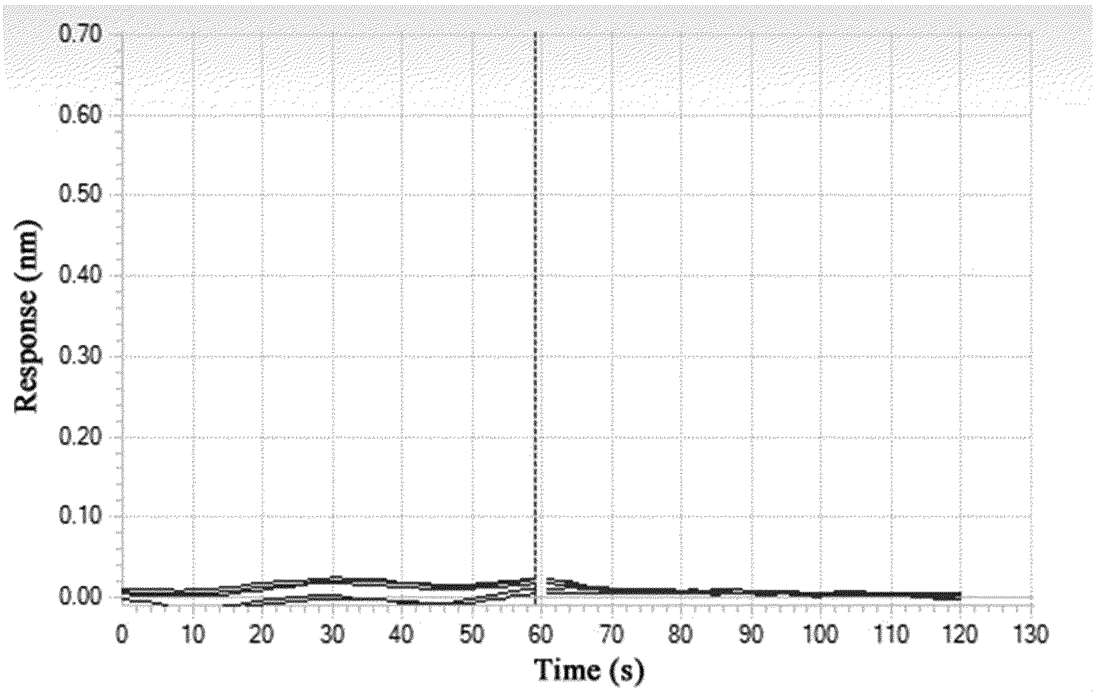
0.70
0.60
0.50
0.40
0.30
0.20
0.10
0.00
Response (nm)
0
10
20
30
40
50
60
70
80
90
100
110
120
130
Time (s)

FIG. 26

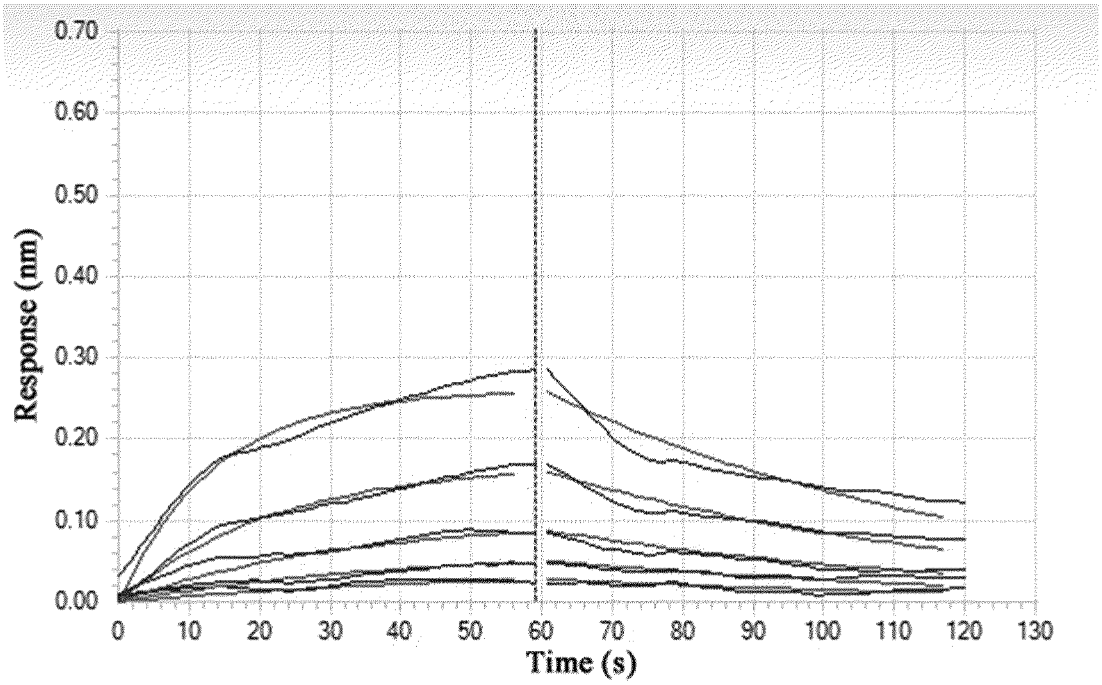
0.70
0.60
0.50
0.40
0.30
0.20
0.10
0.00
Response (nm)
0 10 20 30 40 50 60 70 80 90 100 110 120 130
Time (s)

FIG. 27

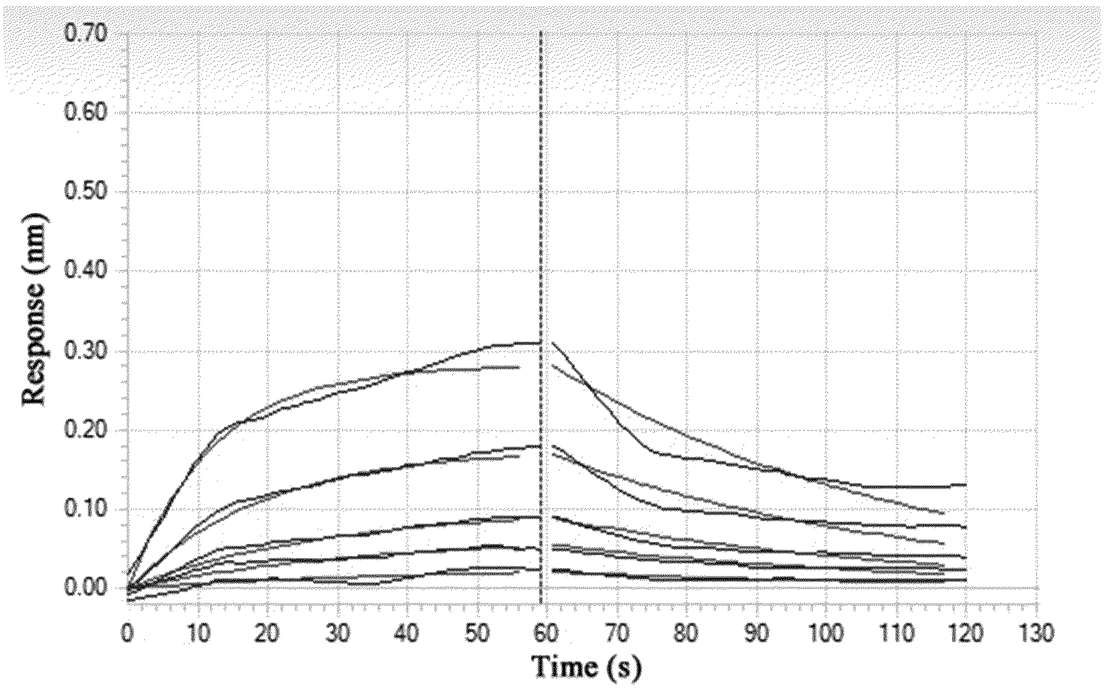
0.70
0.60
0.50
0.40
0.30
0.20
0.10
0.00
Response (nm)
0 10 20 30 40 50 60 70 80 90 100 110 120 130
Time (s)

FIG. 28

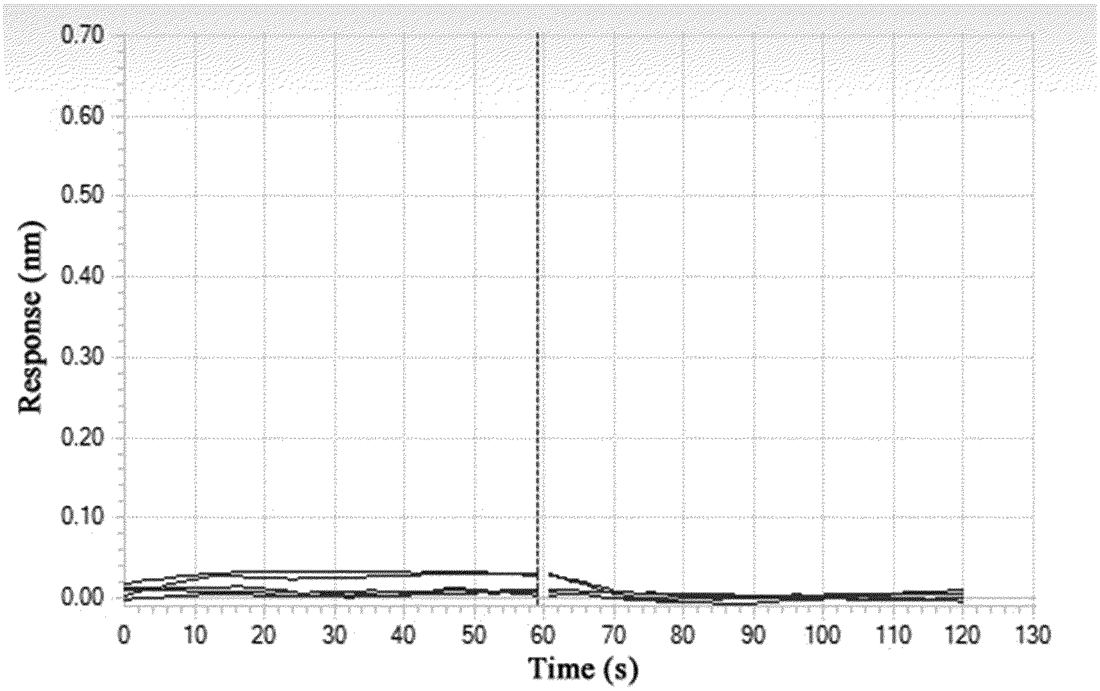
0.70
0.60
0.50
0.40
0.30
0.20
0.10
0.00
Response (nm)
0 10 20 30 40 50 60 70 80 90 100 110 120 130
Time (s)

FIG. 29

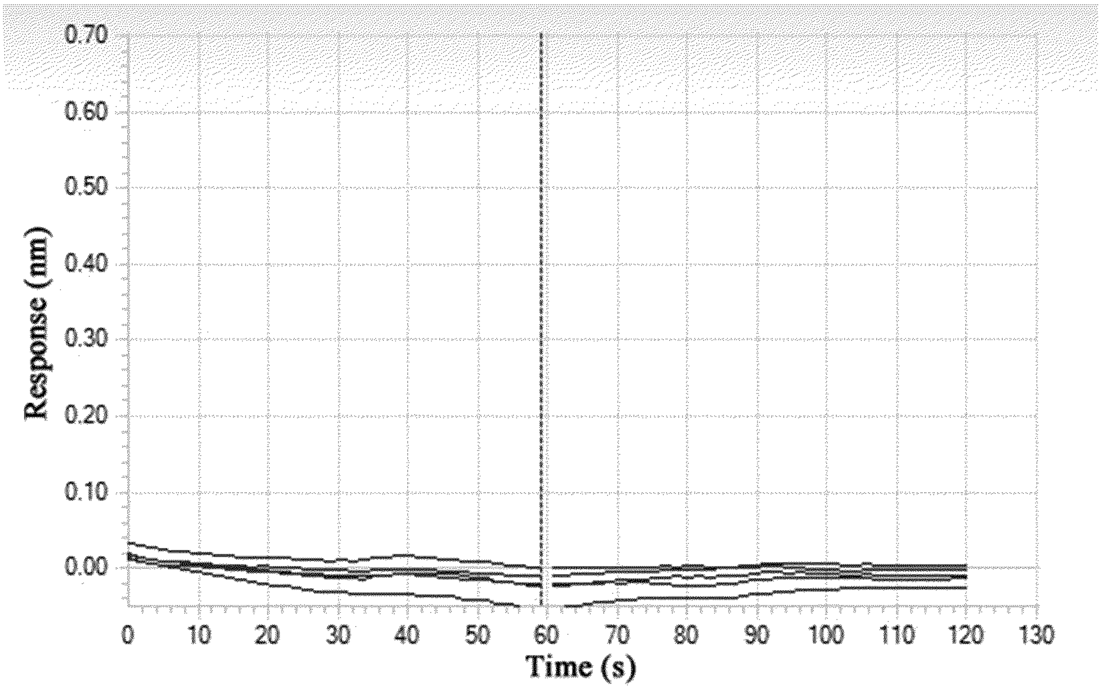
0.70
0.60
0.50
0.40
0.30
0.20
0.10
0.00
Response (nm)
0 10 20 30 40 50 60 70 80 90 100 110 120 130
Time (s)

FIG. 30

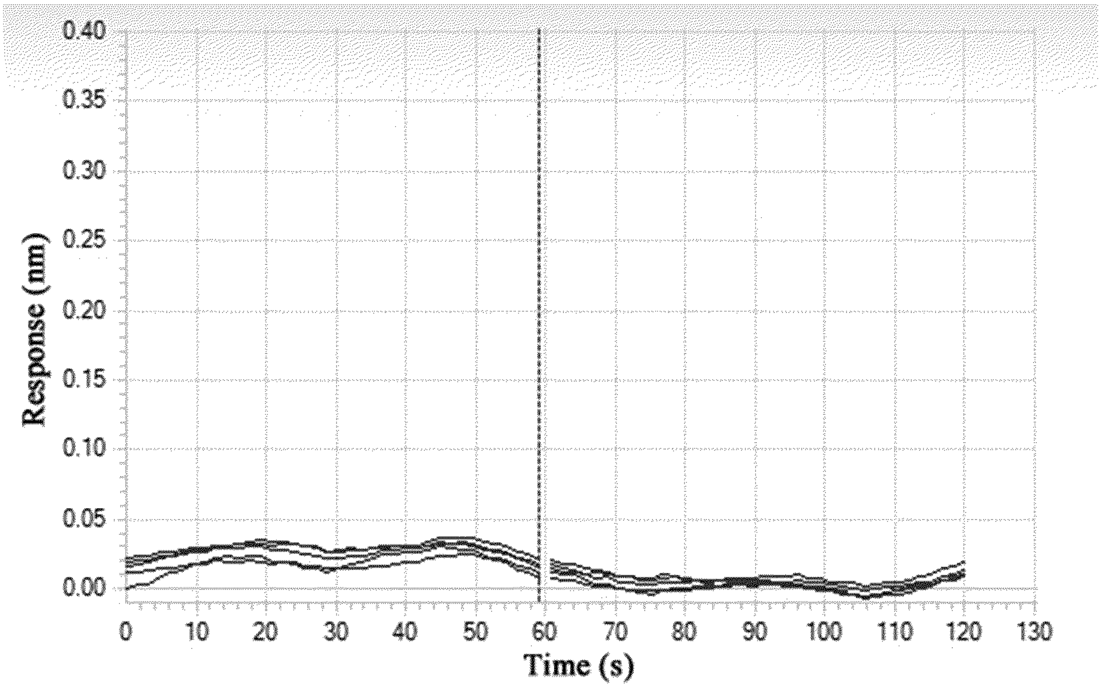
Response (nm)
0.40
0.35
0.30
0.25
0.20
0.15
0.10
0.05
0.00
0
10
20
30
40
50
60
70
80
90
100
110
120
130
Time (s)

FIG. 31

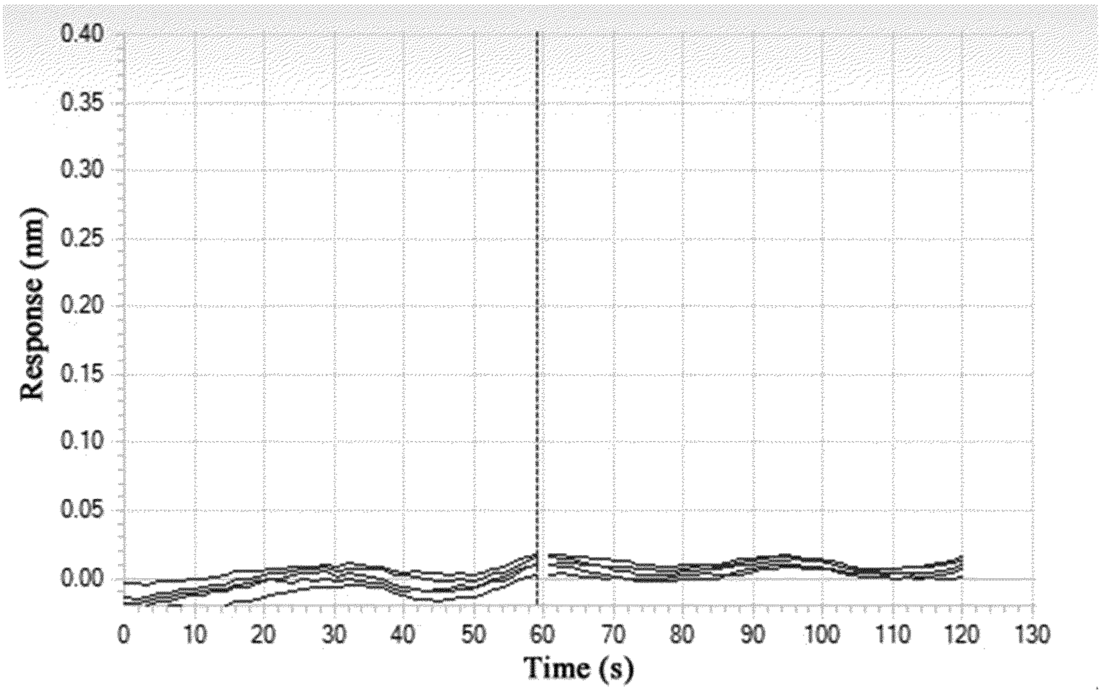
Response (nm)
0.40
0.35
0.30
0.25
0.20
0.15
0.10
0.05
0.00
0
10
20
30
40
50
60
70
80
90
100
110
120
130
Time (s)

FIG. 32

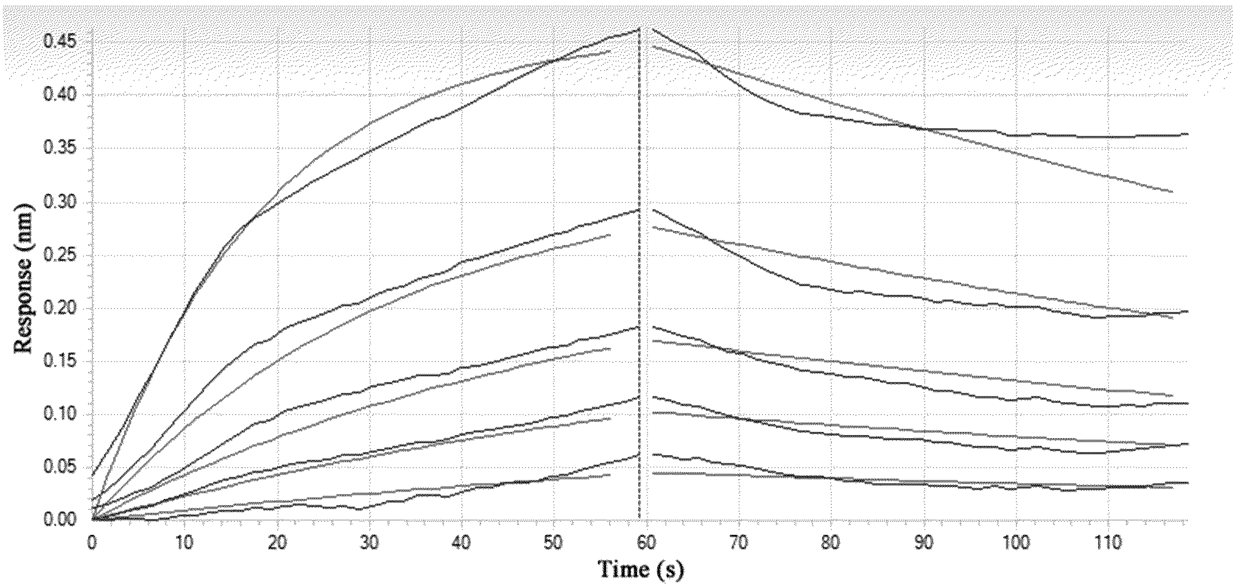


1

FIG. 33

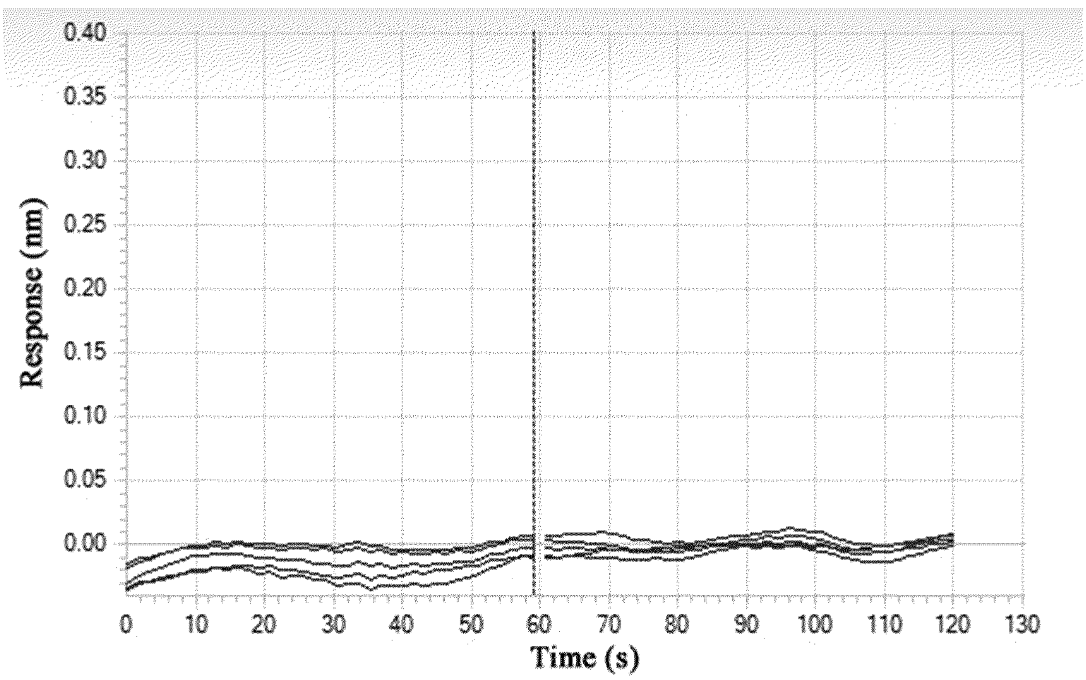


FIG. 34

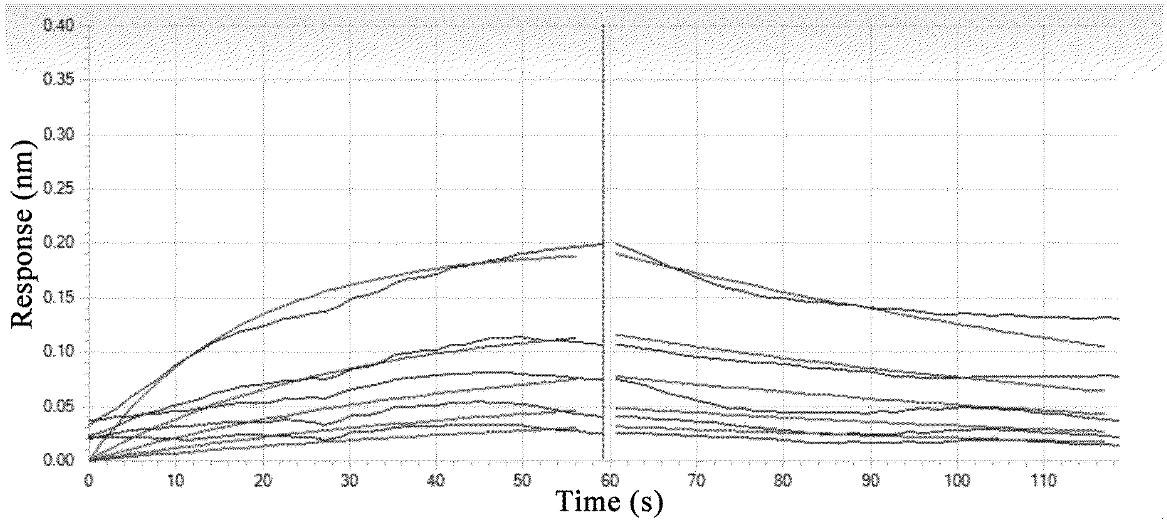
Response (nm)
0.40
0.35
0.30
0.25
0.20
0.15
0.10
0.05
0.00
0
10
20
30
40
50
60
70
80
90
100
110
Time (s)

FIG. 35

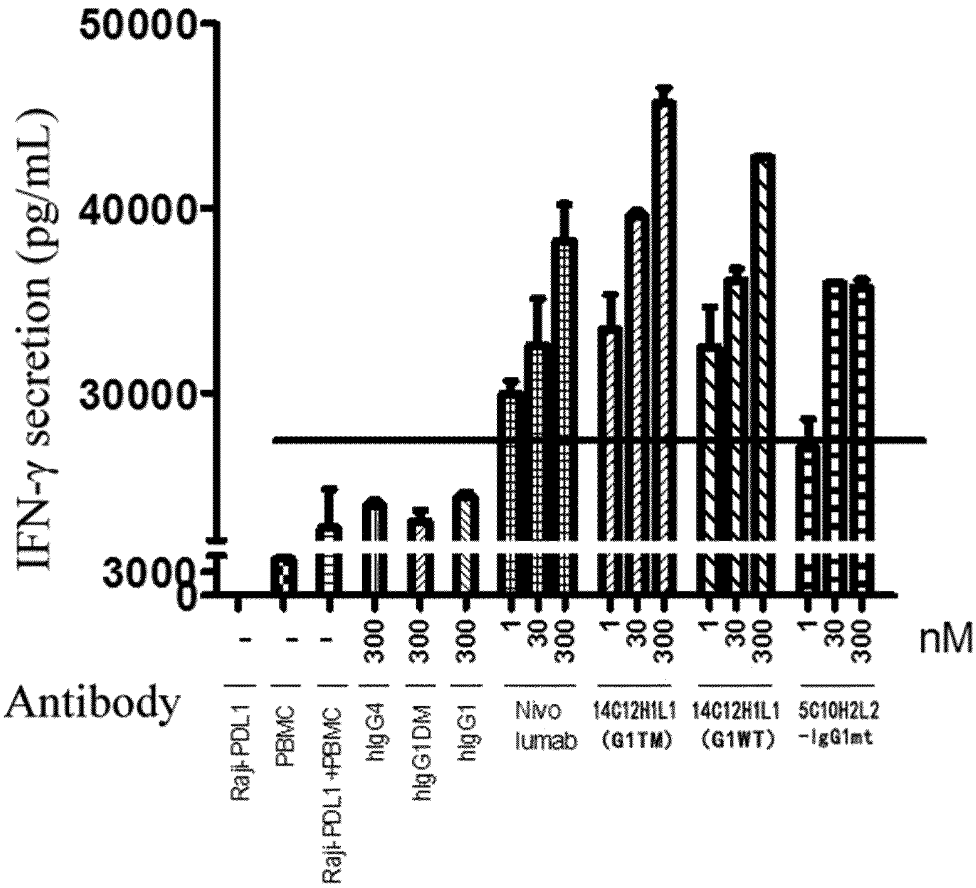
IFN-γ secretion (pg/mL)
50000
40000
30000
3000
0
- - - 300 300 300 1 30 300 1 30 300 1 30 300 1 30 300 nM
Antibody
Raji-PDL1
PBMC
Raji-PDL1 +PBMC
hIgG4
hIgG1DM
hIgG1
Nivo lumab
14C12H1L1 (G1TM)
14C12H1L1 (G1WT)
5C10H2L2 -IgG1mt

FIG. 36

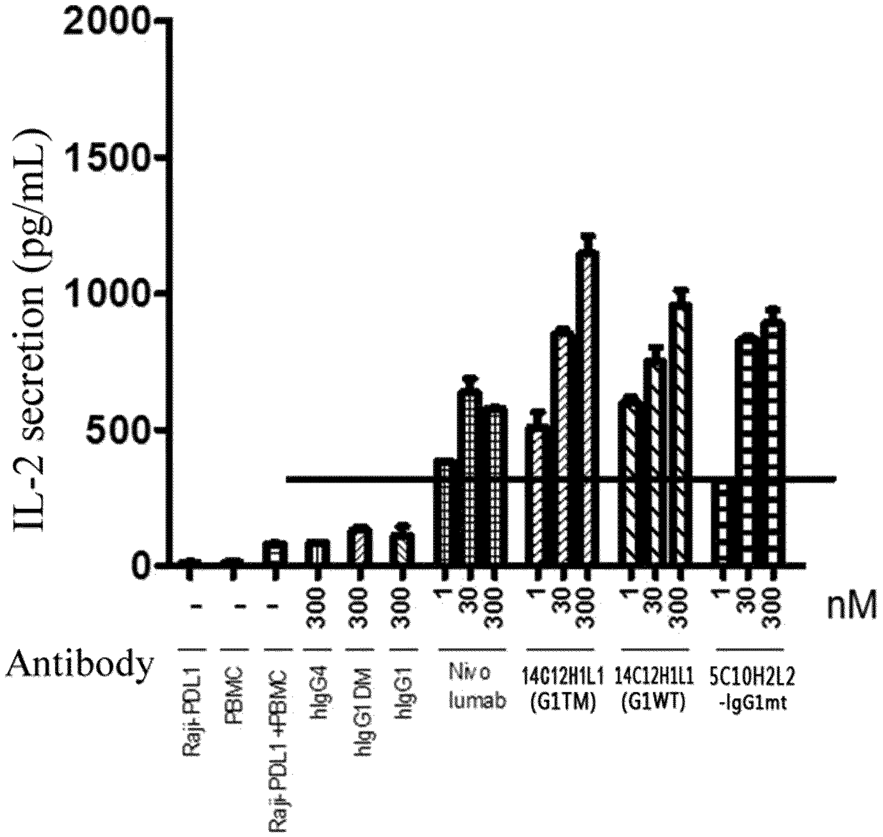
IL-2 secretion (pg/mL)
2000
1500
1000
500
0
nM
Antibody
Raji-PDL1
PBMC
Raji-PDL1 +PBMC
hIgG4
hIgG1 DM
hIgG1
Nivo lumab
14C12H1L1 (G1TM)
14C12H1L1 (G1WT)
5C10H2L2 -IgG1mt
-
300
1
30

FIG. 37

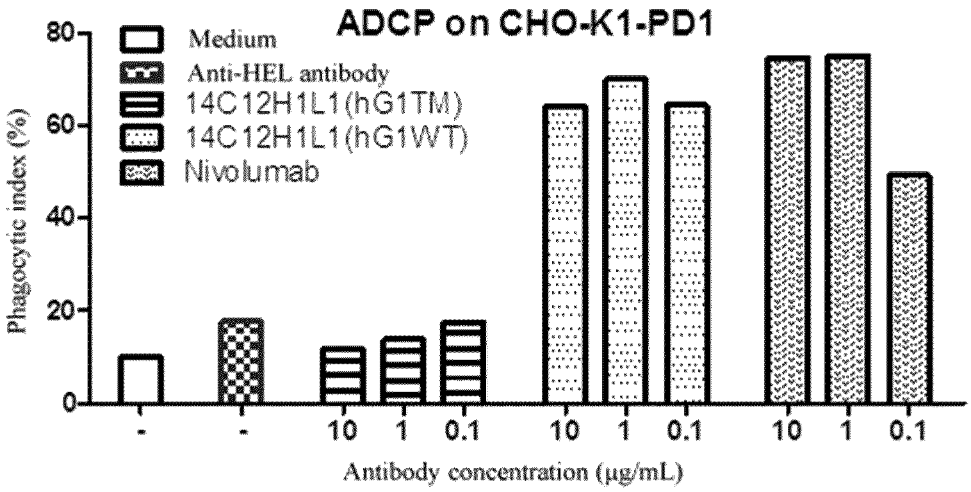
ADCP on CHO-K1-PD1
Medium
Anti-HEL antibody
14C12H1L1(hG1TM)
14C12H1L1(hG1WT)
Nivolumab
Phagocytic index (%)
80
60
40
20
0
-
10
1
0.1
Antibody concentration (μg/mL)

FIG. 38

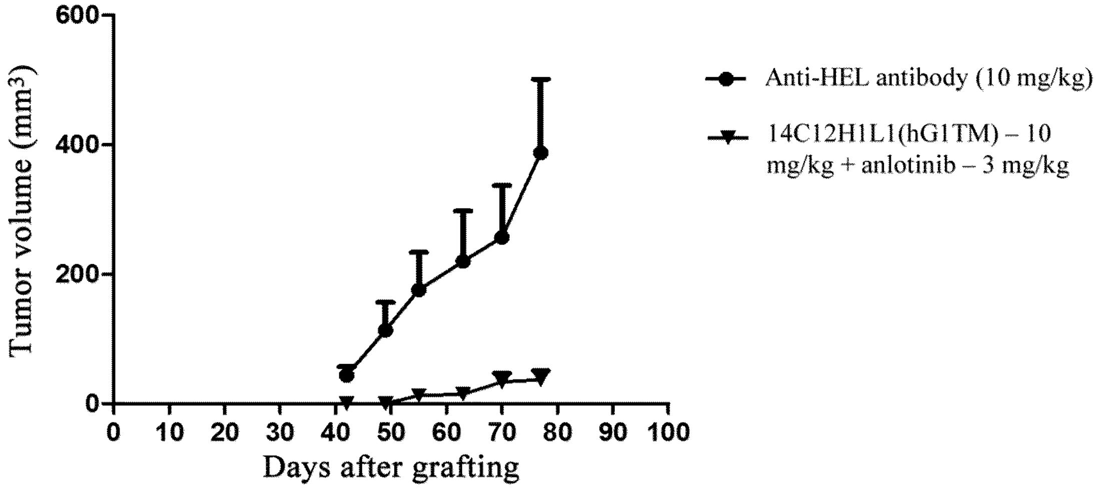
600
400
200
0
Tumor volume (mm3)
0 10 20 30 40 50 60 70 80 90 100
Days after grafting
Anti-HEL antibody (10 mg/kg)
14C12H1L1(hG1TM) – 10 mg/kg + anlotinib – 3 mg/kg

FIG. 39

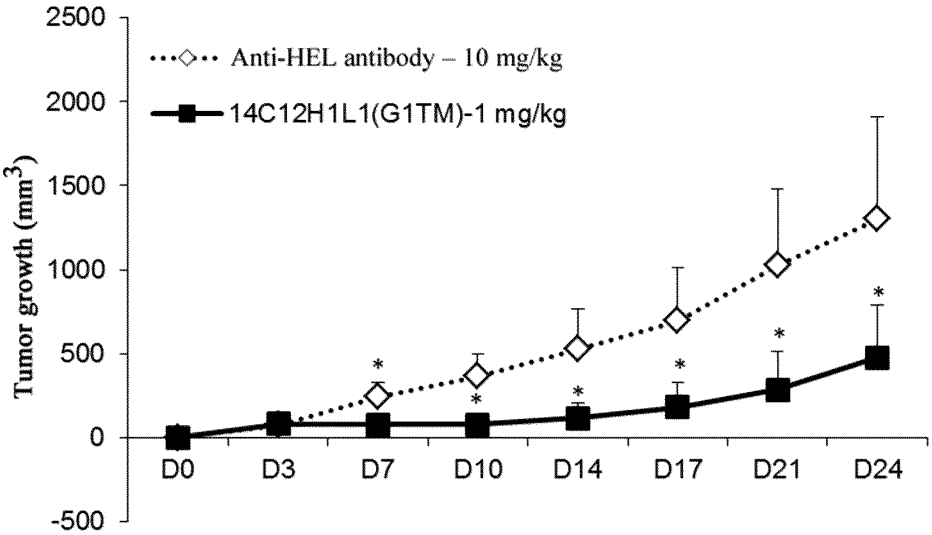
2500
2000
1500
1000
500
0
-500
Tumor growth (mm3)
D0 D3 D7 D10 D14 D17 D21 D24
Anti-HEL antibody – 10 mg/kg
14C12H1L1(G1TM)-1 mg/kg

FIG. 40

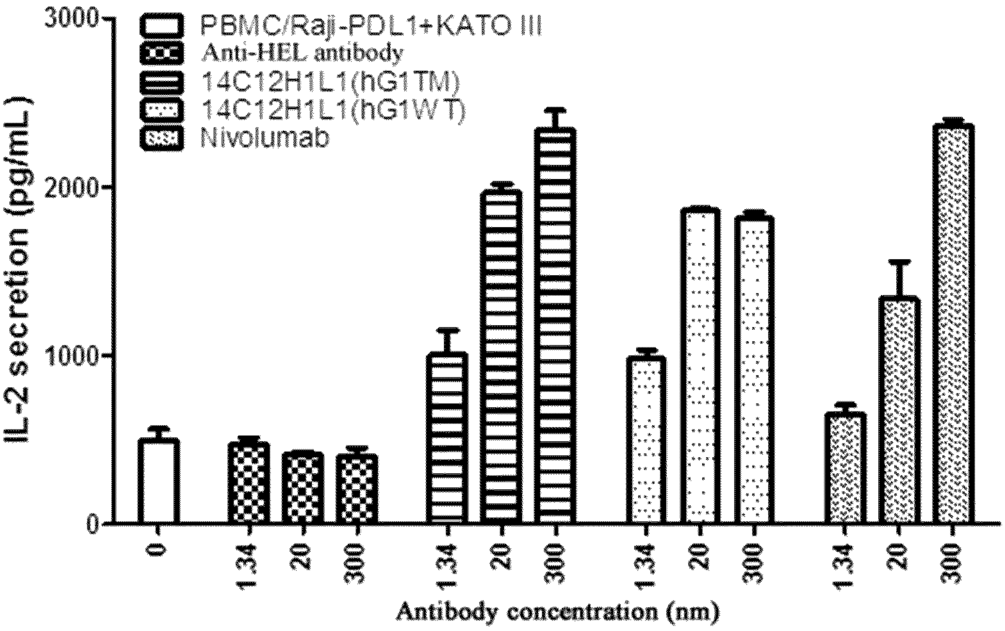

3000
2000
1000
0
IL-2 secretion (pg/mL)
PBMC/Raji-PDL1+KATO III
Anti-HEL antibody
14C12H1L1(hG1TM)
14C12H1L1(hG1WT)
Nivolumab
0
1.34
20
300
1.34
20
300
1.34
20
300
1.34
20
300
Antibody concentration (nm)


FIG. 41

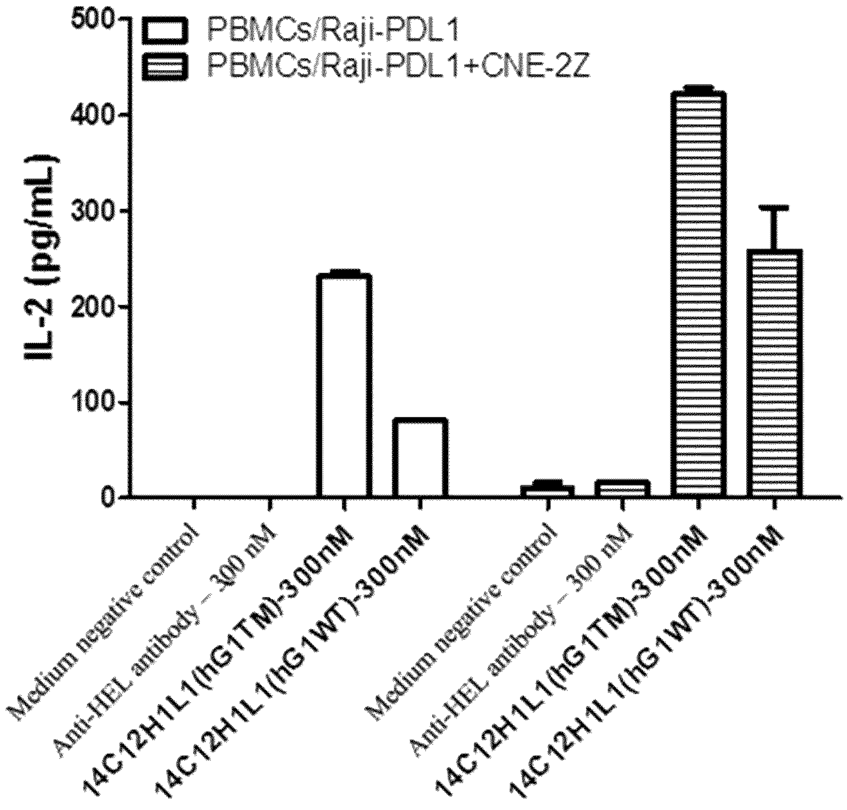

500
400
300
200
100
0
IL-2 (pg/mL)
PBMCs/Raji-PDL1
PBMCs/Raji-PDL1+CNE-2Z
Medium negative control
Anti-HEL antibody – 300 nM
14C12H1L1(hG1TM)-300nM
14C12H1L1(hG1WT)-300nM
Medium negative control
Anti-HEL antibody – 300 nM
14C12H1L1(hG1TM)-300nM
14C12H1L1(hG1WT)-300nM


FIG. 42

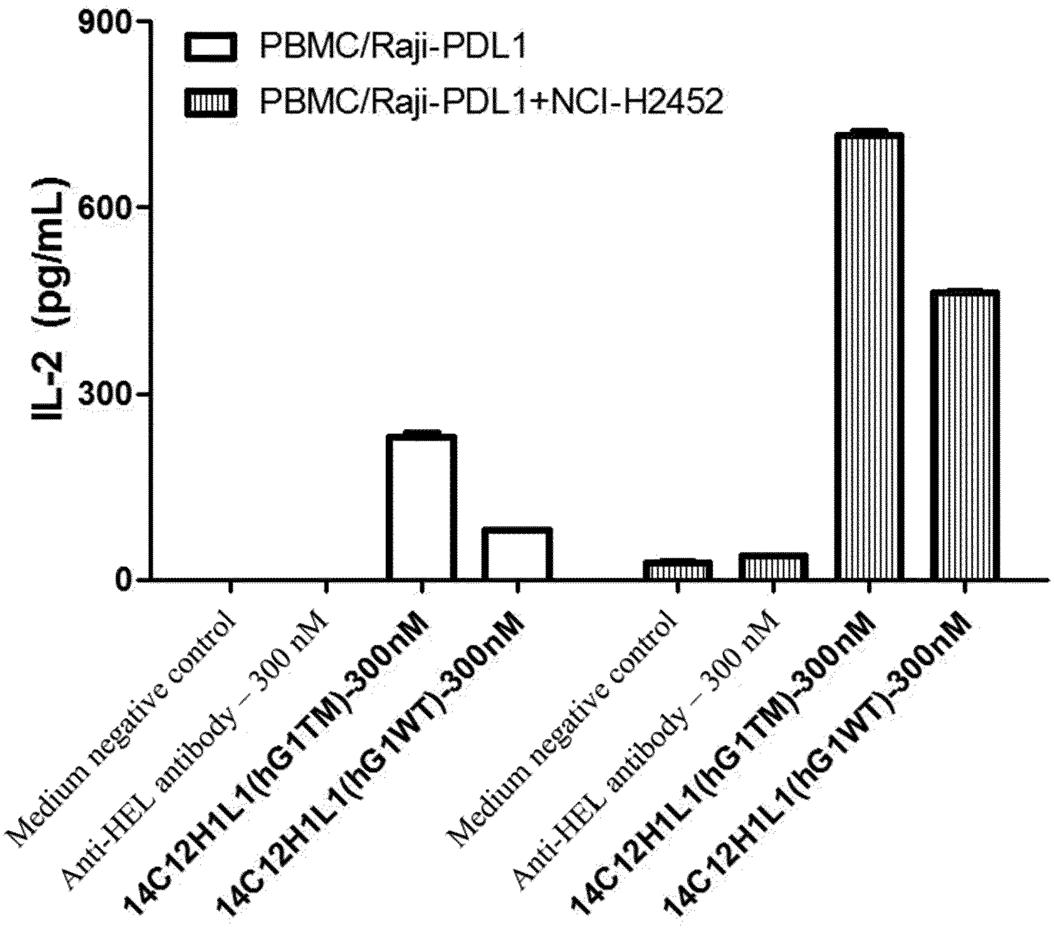

900
600
300
0
IL-2 (pg/mL)
PBMC/Raji-PDL1
PBMC/Raji-PDL1+NCI-H2452
Medium negative control
Anti-HEL antibody – 300 nM
14C12H1L1(hG1TM)-300nM
14C12H1L1(hG1WT)-300nM
Medium negative control
Anti-HEL antibody – 300 nM
14C12H1L1(hG1TM)-300nM
14C12H1L1(hG1WT)-300nM


FIG. 43

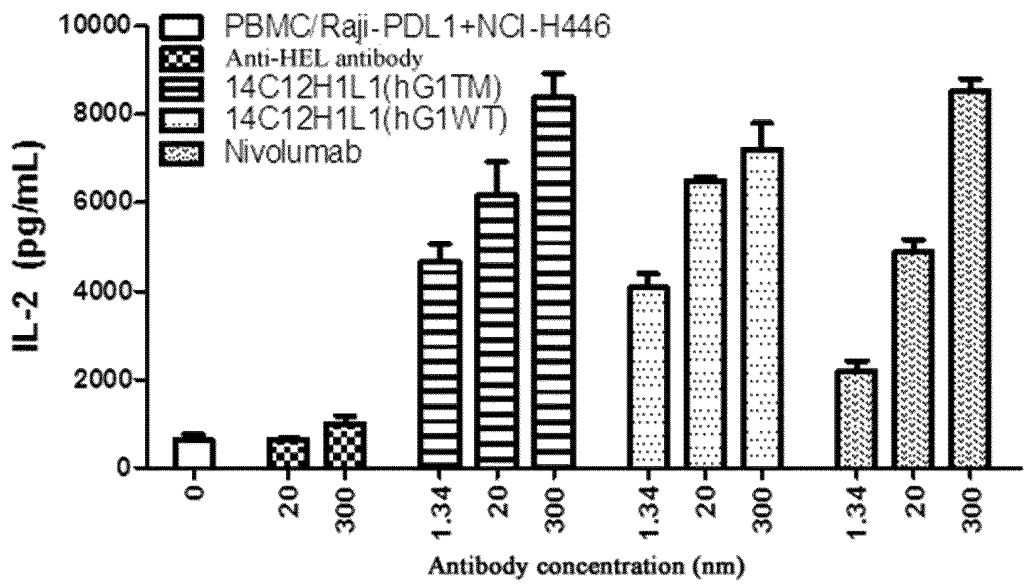

10000
8000
6000
4000
2000
0
IL-2 (pg/mL)
PBMC/Raji-PDL1+NCI-H446
Anti-HEL antibody
14C12H1L1(hG1TM)
14C12H1L1(hG1WT)
Nivolumab
0
20
300
1.34
20
300
1.34
20
300
1.34
20
300
Antibody concentration (nm)


FIG. 44

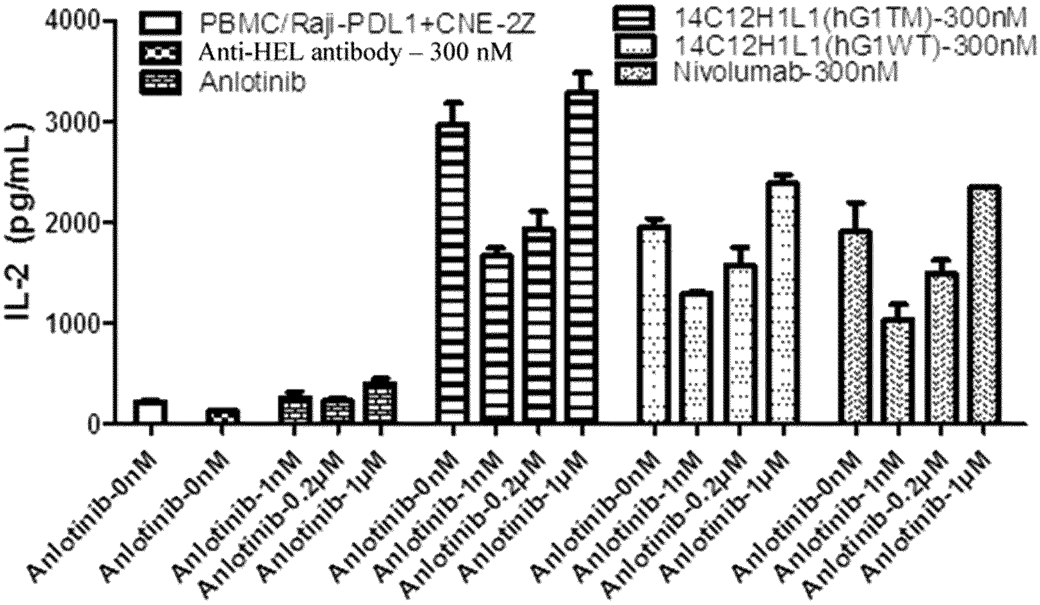
PBMC/Raji-PDL1+CNE-2Z
Anti-HEL antibody – 300 nM
Anlotinib
14C12H1L1(hG1TM)-300nM
14C12H1L1(hG1WT)-300nM
Nivolumab-300nM
IL-2 (pg/mL)
4000
3000
2000
1000
0
Anlotinib-0nM
Anlotinib-0nM
Anlotinib-1nM
Anlotinib-0.2μM
Anlotinib-1μM
Anlotinib-0nM
Anlotinib-1nM
Anlotinib-0.2μM
Anlotinib-1μM
Anlotinib-0nM
Anlotinib-1nM
Anlotinib-0.2μM
Anlotinib-1μM
Anlotinib-0nM
Anlotinib-1nM
Anlotinib-0.2μM
Anlotinib-1μM

FIG. 45

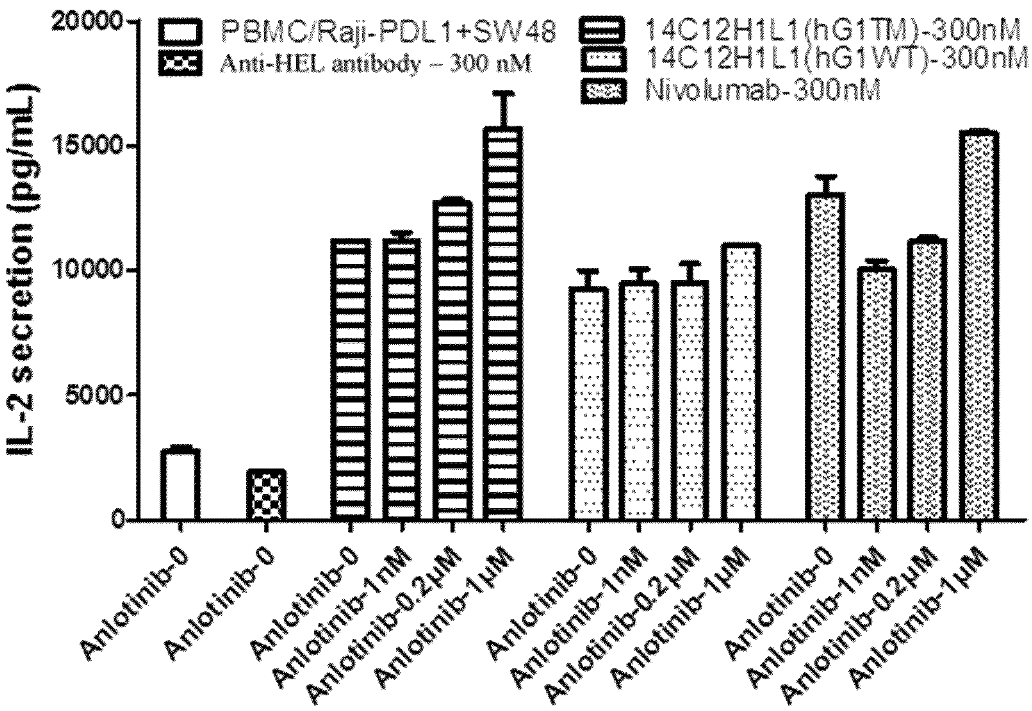
PBMC/Raji-PDL1+SW48
Anti-HEL antibody – 300 nM
14C12H1L1(hG1TM)-300nM
14C12H1L1(hG1WT)-300nM
Nivolumab-300nM
IL-2 secretion (pg/mL)
20000
15000
10000
5000
0
Anlotinib-0
Anlotinib-0
Anlotinib-0
Anlotinib-1nM
Anlotinib-0.2μM
Anlotinib-1μM
Anlotinib-0
Anlotinib-1nM
Anlotinib-0.2μM
Anlotinib-1μM
Anlotinib-0
Anlotinib-1nM
Anlotinib-0.2μM
Anlotinib-1μM

FIG. 46

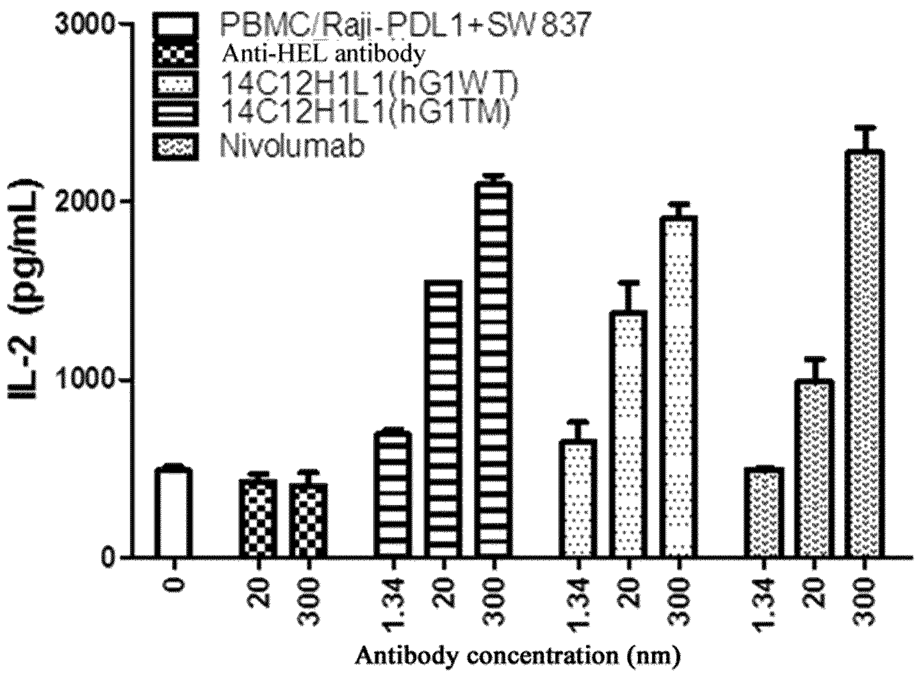
3000
2000
1000
0
IL-2 (pg/mL)
PBMC/Raji-PDL1+SW837
Anti-HEL antibody
14C12H1L1(hG1WT)
14C12H1L1(hG1TM)
Nivolumab
0
20
300
1.34
20
300
1.34
20
300
1.34
20
300
Antibody concentration (nm)

FIG. 47

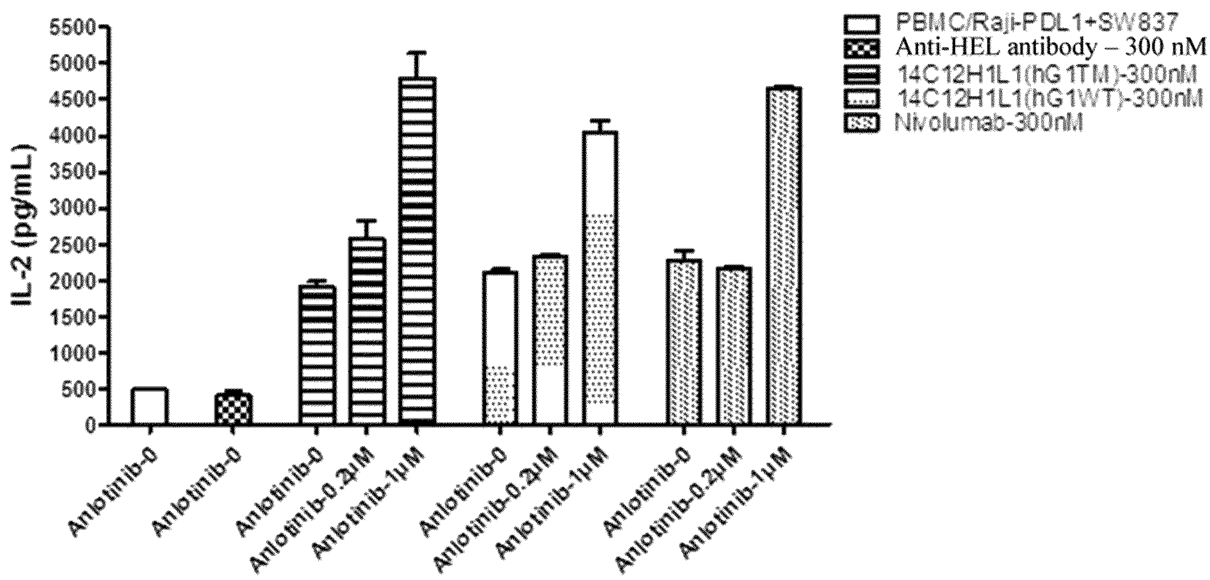
5500
5000
4500
4000
3500
3000
2500
2000
1500
1000
500
0
IL-2 (pg/mL)
PBMC/Raji-PDL1+SW837
Anti-HEL antibody – 300 nM
14C12H1L1(hG1TM)-300nM
14C12H1L1(hG1WT)-300nM
Nivolumab-300nM
Anlotinib-0
Anlotinib-0
Anlotinib-0
Anlotinib-0.2μM
Anlotinib-1μM
Anlotinib-0
Anlotinib-0.2μM
Anlotinib-1μM
Anlotinib-0
Anlotinib-0.2μM
Anlotinib-1μM

FIG. 48

**INTERNATIONAL SEARCH REPORT**

International application No. **PCT/CN2020/106219**

**A. CLASSIFICATION OF SUBJECT MATTER**

A61K 39/395(2006.01)i; C07K 16/18(2006.01)i; C12N 5/20(2006.01)i; C12N 15/13(2006.01)i; G01N 33/68(2006.01)i; G01N 33/577(2006.01)i; G01N 33/574(2006.01)i; A61P 35/00(2006.01)i; A61P 35/02(2006.01)i

According to International Patent Classification (IPC) or to both national classification and IPC

**B. FIELDS SEARCHED**

Minimum documentation searched (classification system followed by classification symbols)

A61K, A61P, C07K, C12N, G01N

Documentation searched other than minimum documentation to the extent that such documents are included in the fields searched

Electronic data base consulted during the international search (name of data base and, where practicable, search terms used)

CNABS, CNMED, MOABS, HKABS, TWABS, TWMED, DWPI, SIPOABS, CPEA, AUABS, SGABS, ILABS, FRABS, LEXIS, PLABS, JPABS, KRABS, DEABS, RUABS, WOTXT, EPTXT, USTXT, CNTXT, PUBMED, CNKI, GENBANK, 中国专利生物序列检索系统: 抗体, 亲和力, 突变, 取代, 肿瘤, antibody, affinity, mutation, substitution, tumor, ADCC, Fc, PD-1, L234A , leu234ala, L235A , leu235ala, G237A , gly237ala, sequence search for SEQ ID NOs: 2, 4, 6, 8, 12, 16, 18

**C. DOCUMENTS CONSIDERED TO BE RELEVANT**

| Category* | Citation of document, with indication, where appropriate, of the relevant passages | Relevant to claim No. |
|---|---|---|
| Y | CN 106977602 A (AKESO BIOPHARMA INC.; ZHONGSHAN AKESO INNOVATIVE DRUG RESEARCH INSTITUTE CO., LTD.) 25 July 2017 (2017-07-25) see claims 1-19, sequence table | 1-31 |
| Y | CN 108136010 A (MACROGENICS INC.) 08 June 2018 (2018-06-08) see description paragraphs 38, 130 | 1-31 |
| Y | CN 107151269 A (SICHUAN KELUN-BIOTECH BIOPHARMACEUTICA CO., LTD.) 12 September 2017 (2017-09-12) see description paragraphs 366-380, claim 8 | 1-3, 5-31 |
| Y | US 2017121409 A1 (JANSSEN BIOTECH, INC.) 04 May 2017 (2017-05-04) see description paragraph 1189, claim 41(e) | 4-31 |
| A | CN 107708666 A (MACROGENICS INC.) 16 February 2018 (2018-02-16) see entire document | 1-31 |
| A | CN 108976300 A (MACROGENICS INC.) 11 December 2018 (2018-12-11) see entire document | 1-31 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **30 October 2020** | **10 November 2020** |
| Name and mailing address of the ISA/CN<br>**China National Intellectual Property Administration (ISA/CN)**<br>**No. 6, Xitucheng Road, Jimenqiao Haidian District, Beijing 100088**<br>**China** | Authorized officer |
| Facsimile No. **(86-10)62019451** | Telephone No. |

Form PCT/ISA/210 (second sheet) (January 2015)

**INTERNATIONAL SEARCH REPORT**

International application No.
**PCT/CN2020/106219**

**C. DOCUMENTS CONSIDERED TO BE RELEVANT**

| Category* | Citation of document, with indication, where appropriate, of the relevant passages | Relevant to claim No. |
|---|---|---|
| A | WO 2018195506 A1 (AMGEN INC.) 25 October 2018 (2018-10-25)<br>see entire document | 1-31 |
| A | TW 201925223 A (ALPINE IMMUNE SCIENCES INC) 01 July 2019 (2019-07-01)<br>see entire document | 1-31 |
| A | Schlothauer T et al. "Novel Human Igg1 and Igg4 Fc-Engineered Antibodies with Completely Abolished Immune Effector Functions"<br>*Protein Engineering,* Vol. 29, No. 10, 29 August 2016 (2016-08-29),<br>pp. 457-466 | 1-31 |
| A | Vafa O et al. "An engineered Fc variant of an IgG eliminates all immune effector functions via structural perturbations"<br>*Methods,* Vol. 65, No. 1,, 17 July 2013 (2013-07-17),<br>pp. 114-126 | 1-31 |

Form PCT/ISA/210 (second sheet) (January 2015)

**INTERNATIONAL SEARCH REPORT**

International application No.
**PCT/CN2020/106219**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      - ☑ in the form of an Annex C/ST.25 text file.
      - ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      - ☐ in the form of an Annex C/ST.25 text file (Rule 13*ter*.1(a)).
      - ☐ on paper or in the form of an image file (Rule 13*ter*.1(b) and Administrative Instructions, Section 713).
2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.
3. Additional comments:

Form PCT/ISA/210 (continuation of first sheet) (January 2015)

INTERNATIONAL SEARCH REPORT

| International application No. |
|---|
| PCT/CN2020/106219 |

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **28-31**
because they relate to subject matter not required to be searched by this Authority, namely:

[1] The method for treating and/or preventing tumors or anemia in claims 28-31 is a therapeutic method for treating the human body, and thus belongs to subject matter for which the International Searching Authority is not to carry out a search as stipulated in PCT Rule 39.1(iv). A search has been carried out on the basis of a reasonable expectation that the method in claims 28-31 be subject matter of drug preparation use claims, that is, "a use of the antibody of any one of claims 1 to 9, the conjugate of claim 13, the pharmaceutical composition in any one of claims 16 to 18, or the combination of drugs in any one of claims 19 to 23 in preparing a drug for treating and/or preventing tumors or anemia".

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

[1] A first invention: claims 1-31 (in part), relating to an antibody and nucleic acid molecules encoding said antibody, a related carrier, a host cell, a conjugate, a test kit, a pharmaceutical composition, a combination of drugs, a drug kit product, a drug preparation use thereof, a method for using same for treating and/or preventing a disease and diagnosing a disease, HCDR1－HCDR3 of the antibody being as shown in SEQ ID NOs: 19-21, and LCDR1－LCDR3 being as shown in SEQ ID NOs: 22-24, the antibody being of the human IgG1 subclass, and according to the Eu numbering system, a mutation occurring in a heavy chain constant region of the antibody at position 234 and position 235.

[2] A second invention: claims 1-6 and 8-31 (in part), relating to an antibody and nucleic acid molecules encoding said antibody, a related carrier, a host cell, a conjugate, a test kit, a pharmaceutical composition, a combination of drugs, a drug kit product, a drug preparation use thereof, a method for using same for treating and/or preventing a disease and diagnosing a disease, HCDR1－HCDR3 of the antibody being as shown in SEQ ID NOs: 19-21, and LCDR1－LCDR3 being as shown in SEQ ID NOs: 22-24, the antibody being of the human IgG1 subclass, and according to the Eu numbering system, a mutation occurring in a heavy chain constant region of the antibody at position 234 and position 237.

[3] A third invention: claims 1-31 (in part), relating to an antibody and nucleic acid molecules encoding said antibody, a related carrier, a host cell, a conjugate, a test kit, a pharmaceutical composition, a combination of drugs, a drug kit product, a drug preparation use thereof, a method for using same for treating and/or preventing a disease and diagnosing a disease, HCDR1－HCDR3 of the antibody being as shown in SEQ ID NOs: 19-21, and LCDR1－LCDR3 being as shown in SEQ ID NOs: 22-24, the antibody being of the human IgG1 subclass, and according to the Eu numbering system, a mutation occurring in a heavy chain constant region of the antibody at position 235 and position 237.

[4] A fourth invention: claims 1-31 (in part), relating to an antibody and nucleic acid molecules encoding said antibody, a related carrier, a host cell, a conjugate, a test kit, a pharmaceutical composition, a combination of drugs, a drug kit product, a drug preparation use thereof, a method for using same for treating and/or preventing a disease and diagnosing a disease, HCDR1－HCDR3 of the antibody being as shown in SEQ ID NOs: 19-21, and LCDR1－LCDR3 being as shown in SEQ ID NOs: 22-24, the antibody being of the human IgG1 subclass, and according to the Eu numbering system, a mutation occurring in a heavy chain constant region of the antibody at position 234, position 235, and position 237.

Form PCT/ISA/210 (continuation of first sheet) (January 2015)

**INTERNATIONAL SEARCH REPORT**

International application No.
**PCT/CN2020/106219**

**Box No. III Observations where unity of invention is lacking (Continuation of item 3 of first sheet)**

[5] The same or corresponding technical feature shared between the described inventions is a human IgG1 subclass antibody having HCDR1－HCDR3 as shown in SEQ ID NOs: 19-21 and LCDR1－LCDR3 as shown in SEQ ID NOs: 22-24. However, CN 106977602 A (publication date 25 July 2017, see claim 1, and description, paragraphs 25-33 and 86) discloses two anti-PD1 monoclonal antibodies 14C12 and 14C12H1L1, having HCDR1－HCDR3 as shown in SEQ ID NOs: 9-11, and LCDR1－LCDR3 as shown in SEQ ID NOs: 12-14 (respectively the same as SEQ ID NOs: 19-21 and 22-24 of the present application), the antibodies being able to be of the IgG1 subclass. Therefore, the described same or corresponding technical feature is already disclosed in the prior art, and thus does not constitute a special technical feature. The four inventions described thus do not share a same or corresponding special technical feature, and do not form a single general inventive concept. The present invention lacks unity, and does not comply with PCT Rules 13.1, 13.2, and 13.3.

1. ☐ As all required additional search fees were timely paid by the applicant, this international search report covers all searchable claims.

2. ☐ As all searchable claims could be searched without effort justifying additional fees, this Authority did not invite payment of additional fees.

3. ☑ As only some of the required additional search fees were timely paid by the applicant, this international search report covers only those claims for which fees were paid, specifically claims Nos.: **1-31 (in part): the technical solution relating to an antibody having a mutation at position 234 and position 235, and claims 1-31 (in part): the technical solution relating to an antibody having a mutation at position 234, position 235, and position 237 (i.e. the first and the fourth invention).**

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet) (January 2015)

**INTERNATIONAL SEARCH REPORT**
**Information on patent family members**

International application No.
**PCT/CN2020/106219**

| Patent document cited in search report | Publication date (day/month/year) | Patent family member(s) | Publication date (day/month/year) |
|---|---|---|---|
| CN 106977602 A | 25 July 2017 | WO 2018036472 A1 | 01 March 2018 |
| | | JP 2019534034 A | 28 November 2019 |
| | | MX 2019002268 A | 21 October 2019 |
| | | CN 106977602 B | 25 September 2018 |
| | | CA 3034849 A1 | 01 March 2018 |
| | | EA 201990570 A1 | 30 August 2019 |
| | | EP 3505535 A4 | 15 April 2020 |
| | | SG CN 11201901584 U A | 28 March 2019 |
| | | US 2019321466 A1 | 24 October 2019 |
| | | EP 3505535 A1 | 03 July 2019 |
| | | BR 112019003775 A2 | 25 June 2019 |
| | | AU 2017316255 A1 | 11 April 2019 |
| | | KR 20190050994 A | 14 May 2019 |
| CN 108136010 A | 08 June 2018 | AU 2016334041 A1 | 26 April 2018 |
| | | EP 3359192 A2 | 15 August 2018 |
| | | KR 20180084772 A | 25 July 2018 |
| | | RU 2018111529 A3 | 09 January 2020 |
| | | EP 3359192 A4 | 01 May 2019 |
| | | HK 1251475 A1 | 01 February 2019 |
| | | BR 112018006817 A2 | 23 October 2018 |
| | | WO 2017062619 A2 | 13 April 2017 |
| | | WO 2017062619 A3 | 18 May 2017 |
| | | MX 2018004177 A | 11 September 2018 |
| | | TW 201718013 A | 01 June 2017 |
| | | ZA 201801797 B | 30 January 2019 |
| | | IL 258521 D0 | 31 May 2018 |
| | | PH 12018500692 A1 | 15 October 2018 |
| | | US 2019389952 A1 | 26 December 2019 |
| | | RU 2018111529 A | 08 November 2019 |
| | | JP 2018536632 A | 13 December 2018 |
| CN 107151269 A | 12 September 2017 | RU 2017145150 A | 21 June 2019 |
| | | EP 3309177 A1 | 18 April 2018 |
| | | KR 102068600 B1 | 21 January 2020 |
| | | US 10465014 B2 | 05 November 2019 |
| | | RU 2693661 C2 | 03 July 2019 |
| | | RU 2017145150 A3 | 21 June 2019 |
| | | WO 2017148424 A1 | 08 September 2017 |
| | | AU 2017226510 B2 | 17 January 2019 |
| | | US 2018305464 A1 | 25 October 2018 |
| | | JP 2018526974 A | 20 September 2018 |
| | | CA 2987118 C | 24 March 2020 |
| | | BR 112017027877 A2 | 11 September 2018 |
| | | CN 108752476 A | 06 November 2018 |
| | | EP 3309177 A4 | 10 October 2018 |
| | | CA 2987118 A1 | 08 September 2017 |
| | | CN 108752476 B | 20 September 2019 |
| | | CN 107922503 A | 17 April 2018 |
| | | US 2020010570 A1 | 09 January 2020 |
| | | CN 107922503 B | 28 August 2018 |

Form PCT/ISA/210 (patent family annex) (January 2015)

**INTERNATIONAL SEARCH REPORT**
**Information on patent family members**

International application No.
**PCT/CN2020/106219**

| Patent document cited in search report | Publication date (day/month/year) | Patent family member(s) | Publication date (day/month/year) |
|---|---|---|---|
| | | MX 2017016851 A | 30 April 2018 |
| | | EP 3309177 B1 | 13 May 2020 |
| | | HK 1247209 A1 | 21 September 2018 |
| | | HK 1247214 B | 19 July 2019 |
| | | AU 2017226510 C1 | 16 May 2019 |
| | | KR 20180004277 A | 10 January 2018 |
| | | AU 2017226510 A1 | 04 January 2018 |
| | | JP 6432121 B2 | 05 December 2018 |
| US 2017121409 A1 | 04 May 2017 | AU 2016350700 A1 | 17 May 2018 |
| | | BR 112018008891 A8 | 26 February 2019 |
| | | EP 3371226 A4 | 17 July 2019 |
| | | CN 108697791 A | 23 October 2018 |
| | | WO 2017079112 A1 | 11 May 2017 |
| | | JP 2019500892 A | 17 January 2019 |
| | | EP 3370768 A1 | 12 September 2018 |
| | | TW 201730213 A | 01 September 2017 |
| | | EA 201891093 A1 | 31 October 2018 |
| | | KR 20180069070 A | 22 June 2018 |
| | | CN 108473584 A | 31 August 2018 |
| | | MX 2018005550 A | 18 July 2019 |
| | | EP 3370769 A1 | 12 September 2018 |
| | | BR 112018008867 A2 | 06 November 2018 |
| | | KR 20180072821 A | 29 June 2018 |
| | | CA 3004134 A1 | 11 May 2017 |
| | | EC SP18041833 A | 30 June 2018 |
| | | EP 3370768 A4 | 24 July 2019 |
| | | GT 201800090 A | 13 November 2019 |
| | | SV 2018005684 A | 25 September 2018 |
| | | CA 3004117 A1 | 11 May 2017 |
| | | IL 258909 D0 | 28 June 2018 |
| | | PH 12018500906 A1 | 05 November 2018 |
| | | AU 2016348388 A1 | 17 May 2018 |
| | | AU 2016348391 A1 | 17 May 2018 |
| | | EP 3370769 A4 | 22 May 2019 |
| | | NI 201800055 A | 29 March 2019 |
| | | BR 112018008891 A2 | 06 November 2018 |
| | | JP 2019500891 A | 17 January 2019 |
| | | WO 2017079116 A3 | 20 July 2017 |
| | | KR 20180069071 A | 22 June 2018 |
| | | CL 2018001177 A1 | 12 October 2018 |
| | | CN 108430509 A | 21 August 2018 |
| | | BR 112018008904 A2 | 27 November 2018 |
| | | WO 2017079116 A2 | 11 May 2017 |
| | | MX 2018005551 A | 09 November 2018 |
| | | CA 3004138 A1 | 11 May 2017 |
| | | PE 20181326 A1 | 20 August 2018 |
| | | SG 11201803520P A | 30 May 2018 |
| | | JP 2019500893 A | 17 January 2019 |
| | | BR 112018008867 A8 | 26 February 2019 |
| | | WO 2017079115 A1 | 11 May 2017 |

Form PCT/ISA/210 (patent family annex) (January 2015)

**INTERNATIONAL SEARCH REPORT**
**Information on patent family members**

International application No.
**PCT/CN2020/106219**

| Patent document cited in search report | Publication date (day/month/year) | Patent family member(s) | Publication date (day/month/year) |
|---|---|---|---|
| | | MX 2018005546 A | 18 July 2019 |
| | | CR 20180234 A | 11 September 2018 |
| | | CO 2018005614 A2 | 31 May 2018 |
| | | EP 3371226 A2 | 12 September 2018 |
| | | AR 106583 A1 | 31 January 2018 |
| CN 107708666 A | 16 February 2018 | EP 3307240 A4 | 19 December 2018 |
| | | MX 2017016071 A | 21 February 2018 |
| | | PH 12017502270 A1 | 11 June 2018 |
| | | CA 2988602 A1 | 15 December 2016 |
| | | US 2018298100 A1 | 18 October 2018 |
| | | CL 2017003131 A1 | 25 May 2018 |
| | | EP 3307240 A1 | 18 April 2018 |
| | | IL 256224 D0 | 28 February 2018 |
| | | BR 112017026704 A2 | 28 August 2018 |
| | | AU 2016276706 A1 | 04 January 2018 |
| | | AU 2016276706 A2 | 14 June 2018 |
| | | RU 2018100424 A | 15 July 2019 |
| | | WO 2016201051 A1 | 15 December 2016 |
| | | KR 20180021774 A | 05 March 2018 |
| | | TW 201709929 A | 16 March 2017 |
| | | HK 1247129 A1 | 21 September 2018 |
| | | JP 2018516966 A | 28 June 2018 |
| | | RU 2018100424 A3 | 26 November 2019 |
| CN 108976300 A | 11 December 2018 | AU 2016298227 B9 | 31 October 2019 |
| | | SG 10201906059V A | 27 August 2019 |
| | | TW I691509 B | 21 April 2020 |
| | | JP 2019055948 A | 11 April 2019 |
| | | TW 201710292 A | 16 March 2017 |
| | | CN 107847574 A | 27 March 2018 |
| | | CO 2018000867 A2 | 19 April 2018 |
| | | US 2019127467 A1 | 02 May 2019 |
| | | AU 2018214151 B2 | 31 October 2019 |
| | | EC SP18006831 A | 30 April 2018 |
| | | AU 2016298227 A8 | 08 March 2018 |
| | | WO 2017019846 A8 | 25 January 2018 |
| | | AU 2020200054 A1 | 30 January 2020 |
| | | MX 2019001161 A | 04 July 2019 |
| | | MX 2018001227 A | 26 March 2018 |
| | | AU 2016298227 B2 | 03 October 2019 |
| | | IL 257216 D0 | 29 March 2018 |
| | | CA 2993948 A1 | 02 February 2017 |
| | | PE 20181151 A1 | 17 July 2018 |
| | | US 2020231675 A1 | 23 July 2020 |
| | | PH 12018500232 A1 | 29 August 2018 |
| | | EP 3328419 A1 | 06 June 2018 |
| | | KR 20180034588 A | 04 April 2018 |
| | | CL 2018000254 A1 | 20 July 2018 |
| | | KR 20180093127 A | 20 August 2018 |
| | | US 10577422 B2 | 03 March 2020 |
| | | JP 2018524394 A | 30 August 2018 |

Form PCT/ISA/210 (patent family annex) (January 2015)

**INTERNATIONAL SEARCH REPORT**
**Information on patent family members**

International application No.
**PCT/CN2020/106219**

| Patent document cited in search report | Publication date (day/month/year) | Patent family member(s) | Publication date (day/month/year) |
|---|---|---|---|
| | | CR 20180062 A | 25 May 2018 |
| | | AU 2016298227 A1 | 22 February 2018 |
| | | WO 2017019846 A1 | 02 February 2017 |
| | | AU 2016298227 A2 | 13 September 2018 |
| | | EP 3328419 A4 | 26 December 2018 |
| | | EA 201890296 A1 | 31 August 2018 |
| | | HK 1248106 A1 | 12 October 2018 |
| | | AU 2018214151 A1 | 30 August 2018 |
| | | EP 3456346 A1 | 20 March 2019 |
| WO 2018195506 A1 | 25 October 2018 | CA 3060409 A1 | 25 October 2018 |
| | | MX 2019012364 A | 28 November 2019 |
| | | TW 201906628 A | 16 February 2019 |
| | | UY 37698 A | 31 October 2018 |
| | | EP 3612218 A1 | 26 February 2020 |
| | | KR 20200018778 A | 20 February 2020 |
| | | CL 2019003000 A1 | 14 February 2020 |
| | | BR 112019021991 A2 | 16 June 2020 |
| | | SG 11201909646 T A | 28 November 2019 |
| | | PE 20200485 A1 | 03 March 2020 |
| | | AU 2018254600 A1 | 24 October 2019 |
| | | CR 20190525 A | 20 March 2020 |
| | | JP 2020517249 A | 18 June 2020 |
| | | CN 110709100 A | 17 January 2020 |
| | | CO 2019012998 A2 | 18 February 2020 |
| TW 201925223 A | 01 July 2019 | EP 3697810 A2 | 26 August 2020 |
| | | IL 273726 D0 | 31 May 2020 |
| | | CA 3078517 A1 | 25 April 2019 |
| | | AR 113455 A1 | 06 May 2020 |
| | | WO 2019079520 A3 | 23 May 2019 |
| | | WO 2019079520 A2 | 25 April 2019 |
| | | KR 20200085777 A | 15 July 2020 |
| | | AU 2018351000 A1 | 30 April 2020 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2008112407 A
- US 7612208 B
- US 7253286 B
- US 4816567 A
- WO 2017148424 A1
- CN 106967172 A
- CN 106977602 A


### Non-patent literature cited in the description


- **JULIE R et al.** *N Engl J Med.,* 2012, vol. 366, 2455-2465
- **FIFE et al.** *Nature Immunology,* 2011, vol. 10, 1185-1193
- **HOFMEYER et al.** *Journal of Biomedicine and Biotechnology,* 2011, vol. 2011, 1-9
- **ARMANATH et al.** *Science Trans. Med.,* 2011, vol. 3, 1-13
- **FRANCISCO et al.** *J. Exp. Med.,* 2009, vol. 206, 3015-3029
- **LATCHMAN et al.** *PNAS,* 2004, vol. 101, 10691-10696
- **IWAI et al.** *PNAS,* 2002, vol. 99, 12293-12297
- **HOGARTH PM ; PIETERSZ GA.** *NATURE REVIEWS DRUG DISCOVERY,* 2012, vol. 11 (4), 311-331
- **ZHANG T et al.** *Cancer Immunol Immunother.,* 2018, vol. 67 (7), 1079-1090
- **DAHAN R et al.** *Cancer cell,* 2015, vol. 28 (3), 285-95
- **PFISTER DG. et al.** *J. Clin. Oncol.,* 2003, vol. 22, 330
- **DE RUYSSCHER et al.** *Annals of Oncology,* 2006, vol. 17, 543-552
- **HE JIE et al.** Clinical Oncology. People's Medical Publishing House, vol. 2016, 230-237
- **HAN B et al.** *Br J Cancer,* 2018, vol. 118 (5), 654-661
- **HAN B et al.** *JAMA Oncol.,* November 2018, vol. 4 (11), 1569-1575
- **KABAT et al.** Sequences of Proteins of Immunological Interest. 1991, vol. 1-3, 91-3242
- **BARETTI M et al.** *Pharmacol Ther.,* 2018, vol. 189, 45-62
- **SCHOENBORN JR ; WILSON CB.** Regulation of Interferon-γ During Innate and Adaptive Immune Responses. *Advances in Immunology,* 2007, vol. 96, 41-101
- Pharmacologic administration of interleukin-2. *Ann. N.Y. Acad. Sci.,* 2009, vol. 1182, 14-27
- **KABAT.** Sequences of Proteins of Immunological Interest. National Institutes of Health, 1987
- **CHOTHIA ; LESK.** *J. Mol. Biol.,* 1987, vol. 196, 901-917
- **CHOTHIA et al.** *Nature,* 1989, vol. 342, 878-883
- **KOHLER et al.** *Nature,* 1975, vol. 256, 495
- **JONES et al.** *Nature,* 1986, vol. 321, 522-525
- **REICHMANN et al.** *Nature,* 1988, vol. 332, 323-329
- **PRESTA.** *Curr. Op. Struct. Biol.,* 1992, vol. 2, 593-596
- **CLARK.** *Immunol. Today,* 2000, vol. 21, 397-402
- Remington's Pharmaceutical Sciences. Mack Publishing Company, 1995
- **J. SAMBROOK et al.** Molecular Cloning: A Laboratory Manual. Science Press
- **ACIERNO et al.** *J Mol Biol,* 2007, vol. 374 (1), 130-146
- **EFREMOVA M et al.** *Nat Commun.,* 2018, vol. 9 (1), 32
- **BRANCH P et al.** *Cancer Res,* 1995, vol. 55 (11), 2304-2309
- **GUO J et al.** *Cancer Res.,* 2011, vol. 71 (8), 2978-2987